# EUROPEAN PATENT SPECIFICATION

(11) **EP 4 136 091 B1**
(45) Date of publication and mention of the grant of the patent: **28.05.2025**
(21) Application number: 21724430.0
(22) Date of filing: 16.04.2021
(51) Int. Cl.: C07D 519/00, A61P 9/00, A61K 31/437

(54) **FORMS AND COMPOSITIONS OF INHIBITORS OF PLASMA KALLIKREIN**
FORMEN UND ZUSAMMENSETZUNGEN VON PLASMAKALLIKREINHEMMERN
FORMES ET COMPOSITION D'INHIBITEURS DE LA KALLICRÉINE PLASMATIQUE

(30) Priority: 17.04.2020 US 202063011776 P
(43) Date of publication of application: 22.02.2023
(73) Proprietor: Takeda Pharmaceutical Company Limited, Osaka-shi, Osaka (JP)
(72) Inventor: TIPPARAJU, Suresh Kumar, Lexington, MA 02421 (US); DEPUE, Jeffrey Scott, Lexington, MA 02421 (US); REISCH, Helge Alfred, Lexington, MA 02421 (US); STRATFORD, Samuel Alexander, Cambridge CB4 0WE (GB); HARRIS, Joseph Stephen, Cambridge CB4 0WE (GB); FINK, Sarah Jocelyn, Lexington, MA 02421 (US); PAPAIOANNOU, Nikolaos, Lexington, MA 02421 (US)
(74) Representative: Hoffmann Eitle
(86) International application number: PCT/US2021/027620
(87) International publication number: WO 2021/211930

(56) References cited:
- WO-A1-2019/178129

## Description

### BACKGROUND OF THE INVENTION

Plasma kallikrein (pKal) is a serine protease zymogen in blood that is converted to its catalytically active form by coagulation factor XIIa, and contributes to the innate inflammatory response and intrinsic cascade of blood coagulation. The mechanisms that lead to the activation of this pathway *in vivo* include interactions with polyphosphates released from activated platelets and deficiency of C1 inhibitor (C1-INH), the primary physiological inhibitor of pKal. pKal-mediated cleavage of high-molecular weight kininogen generates the potent vasodilator and pro-inflammatory nonapeptide bradykinin (BK), which activates the bradykinin 2 receptor. Subsequent cleavage of BK by carboxypeptidases generates des-Arg9-BK, which activates the B1 receptor. Both B1 and B2 receptors are expressed by vascular, glial, and neuronal cell types, with the highest levels of retinal expression detected in the ganglion cell layer and inner and outer nuclear layers. Activation of B1 and B2 receptors causes vasodilation and increases vascular permeability.

pKal is also associated with a number of disorders, such as hereditary angioedema (HAE), an autosomal dominant disease characterized by painful, unpredictable, recurrent attacks of inflammation affecting the hands, feet, face, abdomen, urogenital tract, and the larynx. Prevalence for HAE is uncertain but is estimated to be approximately 1 case per 50,000 persons without known differences among ethnic groups. HAE is caused by deficient (Type I) or dysfunctional (Type II) levels of C1-INH, which inhibits pKal, bradykinin, and other serine proteases in the blood. Individuals with hereditary angioedema (HAE) are deficient in C1-INH and consequently undergo excessive bradykinin generation, which in turn cause painful, debilitating, and potentially fatal swelling attacks. If left untreated, HAE can result in a mortality rate as high as 40% primarily due to upper airway obstruction. Consequently, there is a great need in the art for effective inhibitors of pKal.

Chemical compounds can form one or more different pharmaceutically acceptable salts and/or solid forms, including amorphous and polymorphic crystal forms. Individual salts and solid forms of bioactive chemical compounds can have different properties. There is a need for the identification and selection of appropriate salts and/or solid forms of bioactive chemical compounds (including appropriate crystalline forms, where applicable) for the development of pharmaceutically acceptable dosage forms for the treatment of various diseases or conditions associated with pKal.

### SUMMARY OF THE INVENTION

The present disclosure provides novel salts and solid forms useful as inhibitors to plasma kallikrein (pKal). In general, salt forms or free base forms, and pharmaceutically acceptable compositions thereof, are useful for treating or lessening the severity of a variety of diseases or disorders as described in detail herein.

### BRIEF DESCRIPTION OF THE DRAWINGS

**Figure 1** provides X-ray powder diffraction (XRPD) pattern of Compound 1 Form 1 (free base).
**Figure 2** provides TGA/DSC curves for Compound **1** Form 1 (free base).
**Figure 3** provides X-ray powder diffraction (XRPD) pattern of Compound **1** Form 2 (free base).
**Figure 4** provides TGA/DSC curves for Compound **1** Form 2 (free base).
**Figure 5** provides X-ray powder diffraction (XRPD) pattern of Compound **1** Form 3 (free base).
**Figure 6** provides TGA/DSC curves for Compound **1** Form 3 (free base).
**Figure 7** provides X-ray powder diffraction (XRPD) pattern of Compound 3 Pattern 1 (L-malate).
**Figure 8** provides TGA/DSC curves for Compound **3** Pattern 1 (L-malate).
**Figure 9** provides X-ray powder diffraction (XRPD) pattern of Compound **4** Pattern 1 (succinate).
**Figure 10** provides TGA/DSC curves for Compound **4** Pattern 1 (succinate).
**Figure 11** provides X-ray powder diffraction (XRPD) pattern of Compound **5** Pattern 1 (phosphate).
**Figure 12** provides TGA/DSC curves for Compound **5** Pattern 1 (phosphate).
**Figure** 13 provides X-ray powder diffraction (XRPD) pattern of Compound **6** Pattern 1 (oxalate).
**Figure 14** provides TGA/DSC curves for Compound 6 Pattern 1 (oxalate).
**Figure 15** provides X-ray powder diffraction (XRPD) pattern of Compound **6** Pattern 4 (oxalate).
**Figure 16** provides TGA/DSC curves for Compound 6 Pattern 4 (oxalate).
**Figure 17** provides overlay of Compound **1** Form 1 (free base) and Compound **7** Pattern 1 (L-tartrate).
**Figure 18** provides TGA/DSC curves for Compound 7 Pattern 1 (L-tartrate).
**Figure 19** provides X-ray powder diffraction (XRPD) pattern of Compound **9** Pattern 1 (fumarate).
**Figure 20** provides TGA/DSC curves for Compound **9** Pattern 1 (fumarate).
**Figure 21** provides thermodynamic stability diagram of different forms of Compound 1 (free base).
**Figure 22** provides the overlay of XRPD patterns of Compound **1** obtained from the synthetic procedure described in Example 1 (Compound **1** as synthesized), Compound **1** Form 1 (free base), and Compound **1** Form 2 (free base).

### DETAILED DESCRIPTION OF THE INVENTION

### General Description of Certain Aspects of the Invention:

PCT patent application publication number WO2019/178129, filed March 12, 2019 and published September 19, 2019 ("the '129 publication") describes certain plasma kallikerin (pKal) inhibitor compounds. Such compounds include N-((7-chloro-8-fluoroimidazo[1,5-a]pyridin-1-yl)methyl)-1-((6-cyclopropylimidazo[1,2-a]pyridin-2-yl)methyl)-1H-1,2,3-triazole-4-carboxamide:

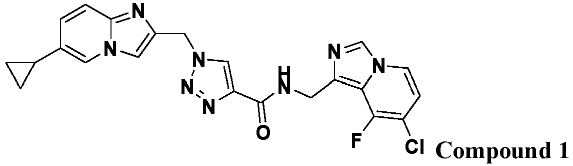

Compound **1,** which is a free base, is one of many compounds identified as a small molecule inhibitor of pKal in the '129 publication. In the '129 publication, Compound **1** is identified as compound I-148 and its synthesis is described in detail at Example 148, which is reproduced herein for ease of reference.

Compound **1** has shown potency against plasma kallikrein in an *in vitro* assay (See, e.g., Table 1 of the '129 publication). For example, the '129 publication reports that Compound **1** has an EC₅₀ < 1 nM as measured in an *in vitro* kallikrein kinase assay. Accordingly, Compound **1** is useful for treating one or more disorders associated with activity of pKal.

The present disclosure provides various free base solid forms of Compound **1,** salt forms of Compound **1** and solid forms thereof, pharmaceutical compositions thereof, and methods of preparing solid forms of Compound **1** and salts and solid forms thereof. Salt forms and solid forms (e.g., crystalline solid forms) impart or may impart characteristics such as improved aqueous solubility, stability, absorption, bioavailability, and ease of formulation. As used herein, unless otherwise indicated the term "salt" refers to a salt or co-crystal of two or more (e.g., two) component molecules (e.g., Compound **1** and a co-former). In the combination of an acid and a base compound for the preparation of a solid form, a Δ pKₐ (pKₐ(base)- pKₐ₋ (acid)) ≥ 1 generally will permit the formation of a salt compound where the two compounds are ionized. Where this threshold is not met, non-ionic interactions (e.g., hydrogen bonds) can still occur between neutral acid and the base compounds to form, e.g., a co-crystal. In some embodiments, a provided solid form is a salt. In other embodiments, a provided solid form is a co-crystal.

### I. Free base Forms of Compound 1

### 1. Compound 1

It is contemplated that Compound **1** can exist in a variety of physical forms. For example, Compound **1** can be in solution, suspension, or in solid form. Compound **1** may be in solid form. When Compound **1** is in solid form, said compound may be amorphous, crystalline, or a mixture thereof. Exemplary solid forms are described in more detail below.

A form of Compound **1** may be substantially free of impurities. As used herein, the term "substantially free of impurities" means that the compound contains no significant amount of extraneous matter. Such extraneous matter may include different forms of Compound **1,** residual solvents, or any other impurities that may result from the preparation of, and/or isolation of, Compound **1.** In certain aspects, at least about 95% by weight of a form of Compound **1** is present. In still other aspects, at least about 99% by weight of a form of Compound **1** is present.

A form of Compound **1** may be present in an amount of at least about 97.0, 97.5, 98.0, 98.5, 99.0, 99.5, or 99.8 weight percent where the percentages are based on the total weight of the composition. form of compound **1** may contain no more than about 3.0 area percent HPLC of total organic impurities and, in certain aspects, no more than about 1.5 area percent HPLC total organic impurities relative to the total area of the HPLC chromatogram. A form of Compound **1** may contain no more than about 1.0 area percent HPLC of any single impurity; no more than about 0.6 area percent HPLC of any single impurity, and, in certain aspects, no more than about 0.5 area percent HPLC of any single impurity, relative to the total area of the HPLC chromatogram.

The structure depicted for a form of Compound **1** is also meant to include all tautomeric forms of Compound **1.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are included.

It has been found that Compound **1** can exist in a variety of solid forms. Exemplary such forms include polymorphs such as those described herein.

Compound **1** may be amorphous. Compound **1** may be amorphous, and substantially free of crystalline compound **1.**

As used herein, the term "polymorph" refers to the different crystal structures into which a compound, or a salt or co-crystal or solvate thereof, can crystallize.

Compound **1** may be a crystalline solid. Compound **1** may be a crystalline solid substantially free of amorphous Compound **1.** As used herein, the term "substantially free of amorphous Compound **1**" means that the compound contains no significant amount of amorphous Compound **1.** At least about 95% by weight of crystalline Compound **1** may be present. At least about 99% by weight of crystalline Compound **1** may be present.

It has been found that Compound **1** can exist in at least three distinct polymorphic forms. Described herein are a polymorphic form of Compound **1** referred to herein as Form 1, a polymorphic form of Compound **1** referred to herein as Form 2, and a polymorphic form of Compound **1** referred to herein as Form 3.

Compound **1** may be in a polymorphic form substantially free of other polymorphic forms. Compound **1** may be in Form 1, substantially free from other free base forms of Compound **1.** Compound **1** may be in Form 2, substantially free from other free base forms of Compound **1.** Compound **1** may be in Form 3, substantially free from other free base forms of Compound **1.**

Compound **1** may be an anhydrate. Compound **1** may be a hydrate.

### Compound 1 Form 1

Compound **1** Form 1 may have at least 1, 2, 3, 4 or 5 X-ray Powder Diffraction (XRPD) peaks selected from the angles (2 theta ± 0.2) listed in **Table 1** below.

**Table 1. XRPD Peak Positions for Compound 1 Form 1**

| **Angle / ° 2θ** | **Angle / ° 2θ** | **Angle /** °**2θ** |
|---|---|---|
| 6.1 | 17.0 | 24.5 |
| 6.7 | 18.0 | 25.3 |
| 8.5 | 19.4 | 26.6 |
| 10.9 | 20.4 | 27.4 |
| 11.8 | 21.6 | 28.0 |
| 12.1 | 21.9 | 28.7 |
| 13.6 | 22.6 | 29.6 |
| 15.0 | 23.5 | |
| 16.4 | 23.8 | |

Compound **1** Form 1 may be characterized by an X-ray powder diffraction (XRPD) pattern having diffractions at angles (2 theta ± 0.2) and corresponding d-spacing (angstroms ± 0.2) of:

**Table 2. XRPD Peak Positions and d-Spacing for Compound 1 Form 1**

| **Angle / ° 2θ** | **d- spacing / Angstrom** | **Angle / ° 2θ** | **d- spacing / Angstrom** |
|---|---|---|---|
| 6.1 | 14.41 | 21.6 | 4.11 |
| 6.7 | 13.26 | 21.9 | 4.06 |
| 8.5 | 10.38 | 22.6 | 3.94 |
| 10.9 | 8.10 | 23.5 | 3.79 |
| 11.8 | 7.52 | 23.8 | 3.73 |
| 12.1 | 7.28 | 24.5 | 3.63 |
| 13.6 | 6.52 | 25.3 | 3.51 |
| 15.0 | 5.92 | 26.6 | 3.35 |
| 16.4 | 5.39 | 27.4 | 3.25 |
| 17.0 | 5.20 | 28.0 | 3.19 |
| 18.0 | 4.93 | 28.7 | 3.11 |
| 19.4 | 4.58 | 29.6 | 3.02 |
| 20.4 | 4.35 | | |

Compound **1** Form 1 may be characterized in that it has one or more peaks in its X-ray powder diffraction pattern selected from those at about 11.8, 15.0, 17.0, 18.0, 19.4, and 23.5. Compound **1** Form 1 may be characterized in that it has two or more peaks in its X-ray powder diffraction pattern selected from those at about 11.8, 15.0, 17.0, 18.0, 19.4, and 23.5. Compound **1** Form 1 may be characterized in that it has three or more peaks in its X-ray powder diffraction pattern selected from those at about 11.8, 15.0, 17.0, 18.0, 19.4, and 23.5. Compound **1** Form 1 may be characterized in that it has four or more peaks in its X-ray powder diffraction pattern selected from those at about 11.8, 15.0, 17.0, 18.0, 19.4, and 23.5. Compound **1** Form 1 may be characterized in that it has five or more peaks in its X-ray powder diffraction pattern selected from those at about 11.8, 15.0, 17.0, 18.0, 19.4, and 23.5. Compound **1** Form 1 may be characterized in that it has all six peaks in its X-ray powder diffraction pattern selected from those at about 11.8, 15.0, 17.0, 18.0, 19.4, and 23.5. Compound **1** Form 1 may be characterized in that it has all six peaks in its X-ray powder diffraction pattern selected from those at about 11.8, 15.0, 17.0, 18.0, 19.4, and 23.5, corresponding to d-spacing (angstroms ± 0.2) of 7.52, 5.92, 5.20, 4.93, 4.58, and 3.79 (respectively). As used herein, the term "about", when used in reference to a degree 2-theta value refers to the stated value ± 0.2 degree 2-theta.

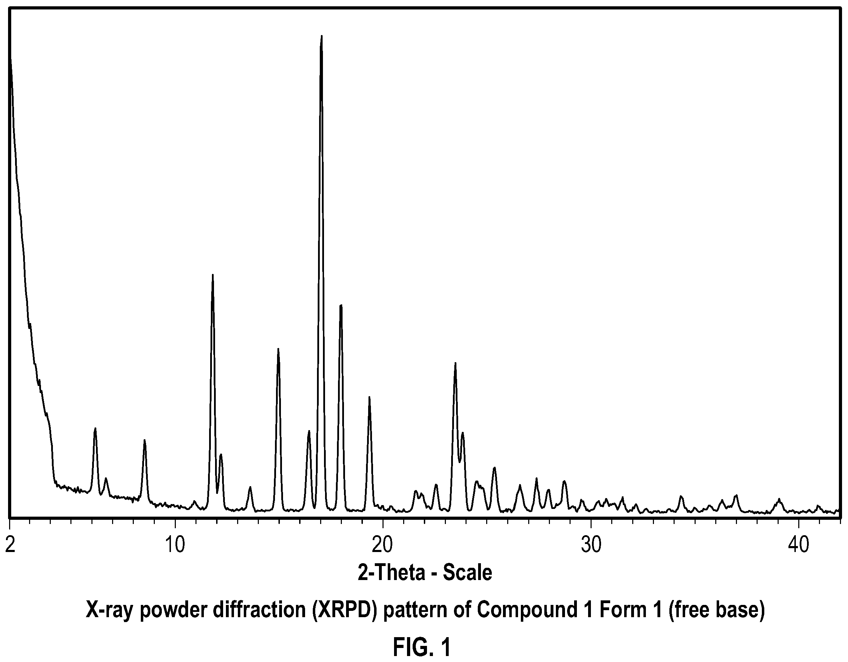

The X-ray powder diffraction pattern of Compound **1** Form 1 may be substantially similar to the XRPD provided in **Figure 1****.**

Methods for preparing Compound **1** Form 1 are described *infra.*

### Compound 1 Form 2

Compound **1** Form 2 may have at least 1, 2, 3, 4 or 5 X-ray Powder Diffraction (XRPD) peaks selected from the angles (2 theta ± 0.2) listed in **Table 3** below.

**Table 3. XRPD Peak Positions for Compound 1 Form 2**

| **Angle / ° 2θ** | **Angle / ° 2θ** |
|---|---|
| 6.1 | 19.8 |
| 8.0 | 20.8 |
| 8.9 | 22.6 |
| 11.8 | 23.6 |
| 13.0 | 24.3 |
| 14.9 | 24.8 |
| 16.3 | 25.6 |
| 17.0 | 28.6 |
| 17.8 | 29.6 |
| 19.3 | 30.7 |

Compound **1** Form 2 may be characterized by an X-ray powder diffraction (XRPD) pattern having diffractions at angles (2 theta ± 0.2) and corresponding d-spacing (angstroms ± 0.2) of:

**Table 4. XRPD Peak Positions and d-Spacing for Compound 1 Form 2**

| **Angle / ° 2θ** | **d- spacing / Angstrom** | **Angle / ° 2θ** | **d- spacing / Angstrom** |
|---|---|---|---|
| 6.1 | 14.52 | 19.8 | 4.47 |
| 8.0 | 11.04 | 20.8 | 4.28 |
| 8.9 | 9.96 | 22.6 | 3.94 |
| 11.8 | 7.51 | 23.6 | 3.77 |
| 13.0 | 6.81 | 24.3 | 3.66 |
| 14.9 | 5.92 | 24.8 | 3.59 |
| 16.3 | 5.42 | 25.6 | 3.48 |
| 17.0 | 5.20 | 28.6 | 3.12 |
| 17.8 | 4.98 | 29.6 | 3.02 |
| 19.3 | 4.60 | 30.7 | 2.91 |

Compound **1** Form 2 may be characterized in that it has one or more peaks in its X-ray powder diffraction pattern selected from those at about 6.1, 8.0, 13.0, 17.0, 17.8, and 25.6. Compound **1** Form 2 may be characterized in that it has two or more peaks in its X-ray powder diffraction pattern selected from those at about 6.1, 8.0, 13.0, 17.0, 17.8, and 25.6. Compound **1** Form 2 may be characterized in that it has three or more peaks in its X-ray powder diffraction pattern selected from those at about 6.1, 8.0, 13.0, 17.0, 17.8, and 25.6. Compound **1** Form 2 may be characterized in that it has four or more peaks in its X-ray powder diffraction pattern selected from those at about 6.1, 8.0, 13.0, 17.0, 17.8, and 25.6. Compound **1** Form 2 may be characterized in that it has five or more peaks in its X-ray powder diffraction pattern selected from those at about 6.1, 8.0, 13.0, 17.0, 17.8, and 25.6. Compound **1** Form 2 may be characterized in that it has all six peaks in its X-ray powder diffraction pattern selected from those at about 6.1, 8.0, 13.0, 17.0, 17.8, and 25.6. Compound **1** Form 2 may be characterized in that it has all six peaks in its X-ray powder diffraction pattern selected from those at about 6.1, 8.0, 13.0, 17.0, 17.8, and 25.6, corresponding to d-spacing (angstroms ± 0.2) of 14.52, 11.04, 6.81, 5.20, 4.98, and 3.48 (respectively).

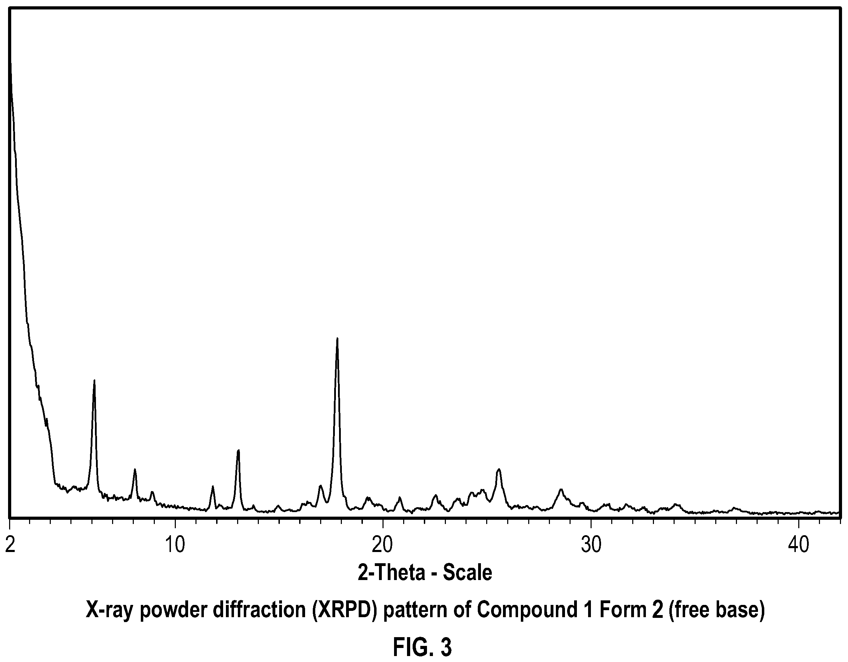

The X-ray powder diffraction pattern of Compound **1** Form 2 may be substantially similar to the XRPD provided in **Figure 3****.**

Methods for preparing Compound **1** Form 2 are described *infra.*

### Compound 1 Form 3

Compound **1** Form 3 may have at least 1, 2, 3, 4 or 5 X-ray Powder Diffraction (XRPD) peaks selected from the angles (2 theta ± 0.2) listed in **Table 5** below.

**Table 5. XRPD Peak Positions for Compound 1 Form 3**

| **Angle / ° 2θ** | **Angle / ° 2θ** |
|---|---|
| 5.8 | 20.8 |
| 8.3 | 21.8 |
| 12.7 | 22.4 |
| 13.8 | 23.5 |
| 14.3 | 24.1 |
| 16.9 | 25.3 |
| 17.5 | 27.3 |
| 17.9 | 28.0 |
| 18.4 | 29.1 |
| 19.3 | 23.9 |

Compound **1** Form 3 may be characterized by an X-ray powder diffraction (XRPD) pattern having diffractions at angles (2 theta ± 0.2) and corresponding d-spacing (angstroms ± 0.2) of:

**Table 6. XRPD Peak Positions and d-Spacing for Compound 1 Form 3**

| **Angle / ° 2θ** | **d- spacing / Angstrom** | **Angle / ° 2θ** | **d- spacing / Angstrom** |
|---|---|---|---|
| 5.8 | 15.21 | 20.8 | 4.27 |
| 8.3 | 10.58 | 21.8 | 4.07 |
| 12.7 | 6.98 | 22.4 | 3.97 |
| 13.8 | 6.40 | 23.5 | 3.78 |
| 14.3 | 6.19 | 24.1 | 3.68 |
| 16.9 | 5.25 | 25.3 | 3.52 |
| 17.5 | 5.07 | 27.3 | 3.26 |
| 17.9 | 4.95 | 28.0 | 3.19 |
| 18.4 | 4.81 | 29.1 | 3.07 |
| 19.3 | 4.59 | 23.9 | 3.72 |

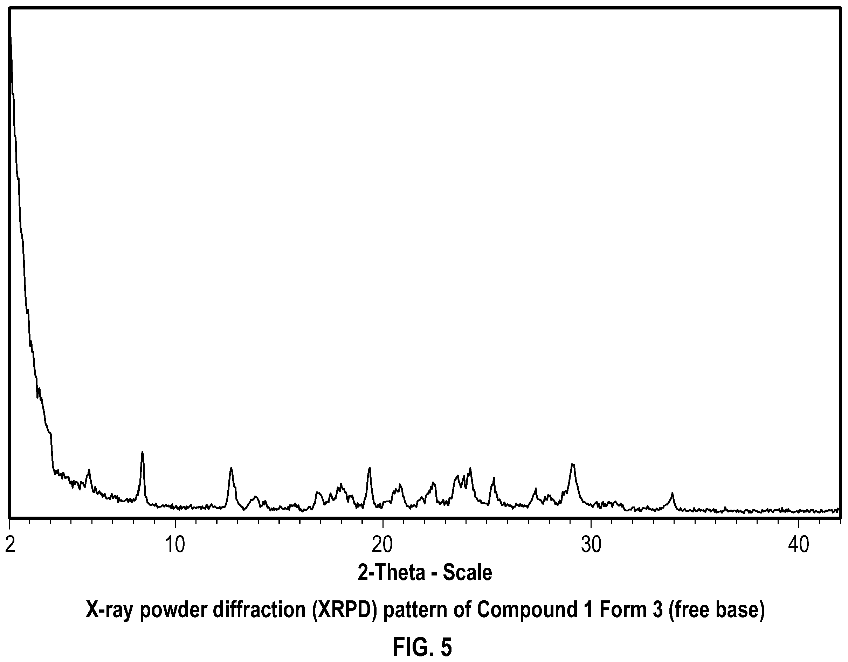

The X-ray powder diffraction pattern of Compound **1** Form 3 may be substantially similar to the XRPD provided in **Figure 5****.**

Methods for preparing Compound **1** Form 3 are described *infra.*

### II. Combinations of Co-Formers with Compound 1

Compound **1** and a co-former (e.g., an acid) may be combined to provide a species where Compound **1** and the co-former are, e.g., ionically bonded or are hydrogen bonded to form one of Compounds **2** through **11,** described below. It is contemplated that Compounds **2** through **11** can exist in a variety of physical forms. For example, Compounds **2** through **11** can be in solution, suspension, or in solid form. Compounds **2** through **11** may be in solid form. When Compounds **2** through **11** are in solid form, said compounds may be amorphous, crystalline, or a mixture thereof. Exemplary solid forms of Compounds **2** through **11** are described in more detail below.

### 2. Compound 2 (Hydrochloric Acid × Compound 1)

Described herein is a chemical species Compound **2** comprising Compound **1** and hydrochloric acid: 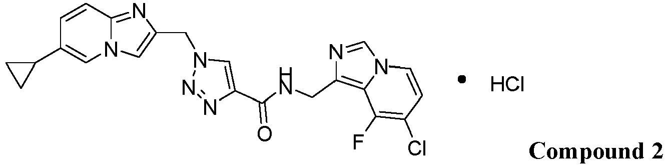 The solid form of Compound 2 may have a stoichiometry of (Compound **1**):(hydrochloric acid) that is about 1:1. The solid form of Compound **2** may have a stoichiometry of (Compound **1**):(hydrochloric acid) that is about 1:2. When Compound **1** is in contact with one or two equivalents of HCl in various solvents, the resulting Compound **2** can exist in at least thirteen distinct polymorphic forms. Described herein are a polymorphic form of Compound **2** referred to herein as Pattern 1, a polymorphic form of Compound **2** referred to herein as Pattern 2, a polymorphic form of Compound **2** referred to herein as Pattern 3, a polymorphic form of Compound **2** referred to herein as Pattern 4, a polymorphic form of Compound **2** referred to herein as Pattern 5, a polymorphic form of Compound **2** referred to herein as Pattern 6, a polymorphic form of Compound **2** referred to herein as Pattern 7, a polymorphic form of Compound **2** referred to herein as Pattern 8, a polymorphic form of Compound **2** referred to herein as Pattern 9, a polymorphic form of Compound **2** referred to herein as Pattern 10, a polymorphic form of Compound **2** referred to herein as Pattern 11, a polymorphic form of Compound **2** referred to herein as Pattern 12, and a polymorphic form of Compound **2** referred to herein as Pattern 13.

It was found that all provided HCl patterns exhibited complex thermal behavior with the onset of endotherms at low temperatures. In three instances on drying, the isolated Compound **2** Pattern 4 material was observed to change to Compound **2** Pattern 9.

### 2. Compound 3 (L-malic Acid × Compound 1)

According to one embodiment, the present invention provides a chemical species Compound **3** comprising Compound **1** and L-malic acid:

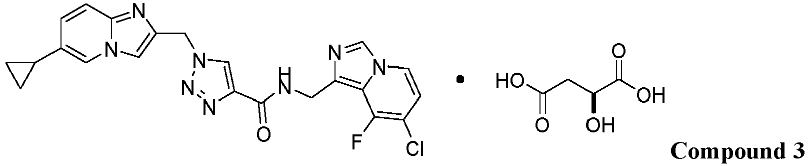

It is contemplated that Compound **3** can exist in a variety of physical forms. For example, Compound **3** can be in solution, suspension, or in solid form. In certain embodiments, Compound **3** is in solid form. When Compound **3** is in solid form, said compound may be amorphous, crystalline, or a mixture thereof. Exemplary solid forms are described in more detail below.

In one embodiment, the solid form of Compound **3** has a stoichiometry of (Compound **1**):(L-malic acid) that is about 1:1.

In some embodiments, the present invention provides Compound **3** substantially free of impurities. As used herein, the term "substantially free of impurities" means that the compound contains no significant amount of extraneous matter. Such extraneous matter may include excess L-malic acid, excess Compound **1,** residual solvents, or any other impurities that may result from the preparation of, and/or isolation of, Compound **3.** In certain embodiments, at least about 95% by weight of Compound **3** is present. In still other embodiments of the invention, at least about 99% by weight of Compound **3** is present.

According to one embodiment, Compound **3** is present in an amount of at least about 97.0, 97.5, 98.0, 98.5, 99.0, 99.5, or 99.8 weight percent where the percentages are based on the total weight of the composition. According to another embodiment, Compound **3** contains no more than about 3.0 area percent HPLC of total organic impurities and, in certain embodiments, no more than about 1.5 area percent HPLC total organic impurities relative to the total area of the HPLC chromatogram. In other embodiments, Compound **3** contains no more than about 1.0 area percent HPLC of any single impurity; no more than about 0.6 area percent HPLC of any single impurity, and, in certain embodiments, no more than about 0.5 area percent HPLC of any single impurity, relative to the total area of the HPLC chromatogram.

The structure depicted for Compound **3** is also meant to include all tautomeric forms of Compound **3.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are within the scope of this invention.

It has been found that Compound **3** can exist in a variety of solid forms. Exemplary such forms include polymorphs such as those described herein.

In some embodiments, Compound **3** is amorphous. In some embodiments, Compound **3** is amorphous, and is substantially free of crystalline Compound **3.**

In certain embodiments, Compound **3** is a crystalline solid. In other embodiments, Compound **3** is a crystalline solid substantially free of amorphous Compound **3.** As used herein, the term "substantially free of amorphous Compound **3**" means that the compound contains no significant amount of amorphous Compound **3.** In certain embodiments, at least about 95% by weight of crystalline Compound **3** is present. In still other embodiments of the invention, at least about 99% by weight of crystalline Compound **3** is present.

It has been found that Compound **3** can exist in at least one polymorphic form. In some embodiments, the present invention provides a polymorphic form of Compound **3** referred to herein as Pattern 1.

### Compound 3 Pattern 1

In some embodiments, Compound **3** Pattern 1 has at least 1, 2, 3, 4 or 5 X-ray Powder Diffraction (XRPD) peaks selected from the angles (2 theta ± 0.2) listed in **Table 7** below.

**Table 7. XRPD Peak Positions for Compound 3 Pattern 1**

| **Angle / ° 2θ** | **Angle / ° 2θ** | **Angle / ° 2θ** |
|---|---|---|
| 6.2 | 18.0 | 25.3 |
| 10.1 | 18.5 | 26.1 |
| 10.9 | 19.3 | 26.4 |
| 11.3 | 20.0 | 26.7 |
| 12.3 | 20.4 | 27.4 |
| 12.7 | 20.8 | 27.9 |
| 13.9 | 21.2 | 28.6 |
| 15.0 | 22.4 | 29.7 |
| 16.5 | 23.5 | 30.2 |
| 17.3 | 23.7 | 30.9 |

In some embodiments, Compound **3** Pattern 1 is characterized by an X-ray powder diffraction (XRPD) pattern having diffractions at angles (2 theta ± 0.2) and corresponding d-spacing (angstroms ± 0.2) of:

**Table 8. XRPD Peak Positions and d-Spacing for Compound 3 Pattern 1**

| **Angle / ° 2θ** | **d- spacing / Angstrom** | **Angle / ° 2θ** | **d- spacing / Angstrom** |
|---|---|---|---|
| 6.2 | 14.32 | 20.8 | 4.26 |
| 10.1 | 8.74 | 21.2 | 4.19 |
| 10.9 | 8.09 | 22.4 | 3.96 |
| 11.3 | 7.82 | 23.5 | 3.79 |
| 12.3 | 7.22 | 23.7 | 3.76 |
| 12.7 | 6.97 | 25.3 | 3.52 |
| 13.9 | 6.37 | 26.1 | 3.41 |
| 15.0 | 5.90 | 26.4 | 3.37 |
| 16.5 | 5.37 | 26.7 | 3.33 |
| 17.3 | 5.13 | 27.4 | 3.25 |
| 18.0 | 4.91 | 27.9 | 3.19 |
| 18.5 | 4.80 | 28.6 | 3.12 |
| 19.3 | 4.60 | 29.7 | 3.00 |
| 20.0 | 4.44 | 30.2 | 2.96 |
| 20.4 | 4.34 | 30.9 | 2.89 |

In some embodiments, Compound **3** Pattern 1 is characterized in that it has one or more peaks in its X-ray powder diffraction pattern selected from those at about 6.2, 12.7, 17.3, 20.4, 25.3, and 26.4. In some embodiments, Compound **3** Pattern 1 is characterized in that it has two or more peaks in its X-ray powder diffraction pattern selected from those at about 6.2, 12.7, 17.3, 20.4, 25.3, and 26.4. In some embodiments, Compound **3** Pattern 1 is characterized in that it has three or more peaks in its X-ray powder diffraction pattern selected from those at about 6.2, 12.7, 17.3, 20.4, 25.3, and 26.4. In some embodiments, Compound **3** Pattern 1 is characterized in that it has four or more peaks in its X-ray powder diffraction pattern selected from those at about 6.2, 12.7, 17.3, 20.4, 25.3, and 26.4. In some embodiments, Compound **3** Pattern 1 is characterized in that it has five or more peaks in its X-ray powder diffraction pattern selected from those at about 6.2, 12.7, 17.3, 20.4, 25.3, and 26.4. In some embodiments, Compound **3** Pattern 1 is characterized in that it has all six peaks in its X-ray powder diffraction pattern selected from those at about 6.2, 12.7, 17.3, 20.4, 25.3, and 26.4. In some embodiments, Compound **3** Pattern 1 is characterized in that it has all six peaks in its X-ray powder diffraction pattern selected from those at about 6.2, 12.7, 17.3, 20.4, 25.3, and 26.4, corresponding to d-spacing (angstroms ± 0.2) of 14.32, 6.97, 5.13, 4.34, 3.52, and 3.37 (respectively).

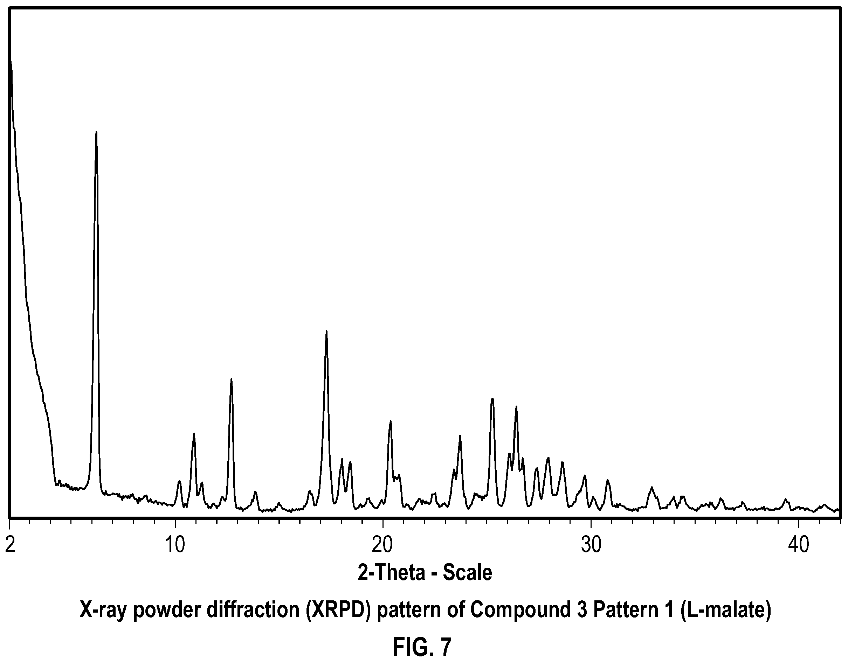

In certain embodiments, the X-ray powder diffraction pattern of Compound **3** Pattern 1 is substantially similar to the XRPD provided in **Figure 7****.**

Methods for preparing Compound **3** Pattern 1 are described *infra.*

### 4. Compound 4 (Succinic acid × Compound 1)

According to one embodiment, the present invention provides a chemical species Compound **4** comprising Compound **1** and succinic acid:

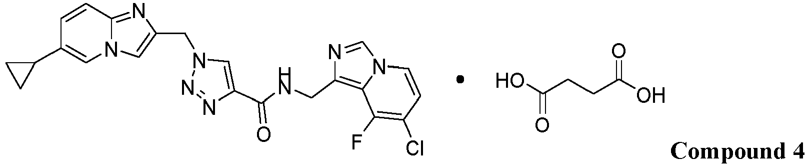

In one embodiment, the solid form of Compound **4** has a stoichiometry of (Compound **1**):(succinic acid) that is about 1:1. As used herein, the term "about", when used in reference to a stoichiometric ratio refers to 1:(1±0.2) ratio of (Compound **1**):(co-former, e.g., an acid), e.g., a 1:(1±0.2) ratio, a 1:(1±0.1) ratio, or a 1:(1±0.05) ratio.

In some embodiments, the present invention provides Compound **4** substantially free of impurities. As used herein, the term "substantially free of impurities" means that the compound contains no significant amount of extraneous matter. Such extraneous matter may include excess succinic acid, excess Compound **1,** residual solvents, or any other impurities that may result from the preparation of, and/or isolation of, Compound **4.** In certain embodiments, at least about 95% by weight of Compound **4** is present. In still other embodiments of the invention, at least about 99% by weight of Compound **4** is present.

According to one embodiment, Compound **4** is present in an amount of at least about 97.0, 97.5, 98.0, 98.5, 99.0, 99.5, or 99.8 weight percent where the percentages are based on the total weight of the composition. According to another embodiment, Compound **4** contains no more than about 3.0 area percent HPLC of total organic impurities and, in certain embodiments, no more than about 1.5 area percent HPLC total organic impurities relative to the total area of the HPLC chromatogram. In other embodiments, Compound **4** contains no more than about 1.0 area percent HPLC of any single impurity; no more than about 0.6 area percent HPLC of any single impurity, and, in certain embodiments, no more than about 0.5 area percent HPLC of any single impurity, relative to the total area of the HPLC chromatogram.

The structure depicted for Compound **4** is also meant to include all tautomeric forms of Compound **4.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are within the scope of this invention.

It has been found that Compound **4** can exist in a variety of solid forms. Exemplary such forms include polymorphs such as those described herein.

In some embodiments, Compound **4** is amorphous. In some embodiments, Compound **4** is amorphous, and is substantially free of crystalline Compound **4.**

In certain embodiments, Compound **4** is a crystalline solid. In other embodiments, Compound **4** is a crystalline solid substantially free of amorphous Compound **4.** As used herein, the term "substantially free of amorphous Compound **4**" means that the compound contains no significant amount of amorphous Compound **4.** In certain embodiments, at least about 95% by weight of crystalline Compound **4** is present. In still other embodiments of the invention, at least about 99% by weight of crystalline Compound **4** is present.

It has been found that Compound **4** can exist in at least one polymorphic form. In some embodiments, the present invention provides a polymorphic form of Compound **4** referred to herein as Pattern 1.

### Compound 4 Pattern 1

In some embodiments, Compound **4** Pattern 1 has at least 1, 2, 3, 4 or 5 X-ray Powder Diffraction (XRPD) peaks selected from the angles (2 theta ± 0.2) listed in **Table 9** below.

**Table 9. XRPD Peak Positions for Compound 4 Pattern 1**

| **Angle / ° 2θ** | **Angle / ° 2θ** | **Angle / ° 2θ** |
|---|---|---|
| 6.2 | 18.5 | 26.5 |
| 7.9 | 19.3 | 26.8 |
| 10.2 | 20.4 | 27.4 |
| 10.9 | 20.9 | 27.9 |
| 11.2 | 21.8 | 28.4 |
| 11.5 | 23.0 | 28.7 |
| 12.7 | 23.8 | 29.8 |
| 16.5 | 24.6 | 31.0 |
| 17.3 | 25.4 | |
| 18.1 | 26.2 | |

In some embodiments, Compound **4** Pattern 1 is characterized by an X-ray powder diffraction (XRPD) pattern having diffractions at angles (2 theta ± 0.2) and corresponding d-spacing (angstroms ± 0.2) of:

**Table 10. XRPD Peak Positions and d-Spacing for Compound 4 Pattern 1**

| **Angle / ° 2θ** | **d- spacing / Angstrom** | **Angle / ° 2θ** | **d- spacing / Angstrom** |
|---|---|---|---|
| 6.2 | 14.26 | 21.8 | 4.08 |
| 7.9 | 11.19 | 23.0 | 3.86 |
| 10.2 | 8.64 | 23.8 | 3.73 |
| 10.9 | 8.11 | 24.6 | 3.62 |
| 11.2 | 7.86 | 25.4 | 3.51 |
| 11.5 | 7.69 | 26.2 | 3.40 |
| 12.7 | 6.95 | 26.5 | 3.36 |
| 16.5 | 5.37 | 26.8 | 3.32 |
| 17.3 | 5.12 | 27.4 | 3.25 |
| 18.1 | 4.89 | 27.9 | 3.19 |
| 18.5 | 4.80 | 28.4 | 3.14 |
| 19.3 | 4.59 | 28.7 | 3.10 |
| 20.4 | 4.35 | 29.8 | 3.00 |
| 20.9 | 4.24 | 31.0 | 2.89 |

In some embodiments, Compound **4** Pattern 1 is characterized in that it has one or more peaks in its X-ray powder diffraction pattern selected from those at about 6.2, 10.2, 12.7, 17.3, 20.4, and 23.8. In some embodiments, Compound **4** Pattern 1 is characterized in that it has two or more peaks in its X-ray powder diffraction pattern selected from those at about 6.2, 10.2, 12.7, 17.3, 20.4, and 23.8. In some embodiments, Compound **4** Pattern 1 is characterized in that it has three or more peaks in its X-ray powder diffraction pattern selected from those at about 6.2, 10.2, 12.7, 17.3, 20.4, and 23.8. In some embodiments, Compound **4** Pattern 1 is characterized in that it has four or more peaks in its X-ray powder diffraction pattern selected from those at about 6.2, 10.2, 12.7, 17.3, 20.4, and 23.8. In some embodiments, Compound **4** Pattern 1 is characterized in that it has five or more peaks in its X-ray powder diffraction pattern selected from those at about 6.2, 10.2, 12.7, 17.3, 20.4, and 23.8. In some embodiments, Compound **4** Pattern 1 is characterized in that it has all six peaks in its X-ray powder diffraction pattern selected from those at about 6.2, 10.2, 12.7, 17.3, 20.4, and 23.8. In some embodiments, Compound **4** Pattern 1 is characterized in that it has all six peaks in its X-ray powder diffraction pattern selected from those at about 6.2, 10.2, 12.7, 17.3, 20.4, and 23.8, corresponding to d-spacing (angstroms ± 0.2) of 14.26, 8.64, 6.95, 5.12, 4.35, and 3.73 (respectively).

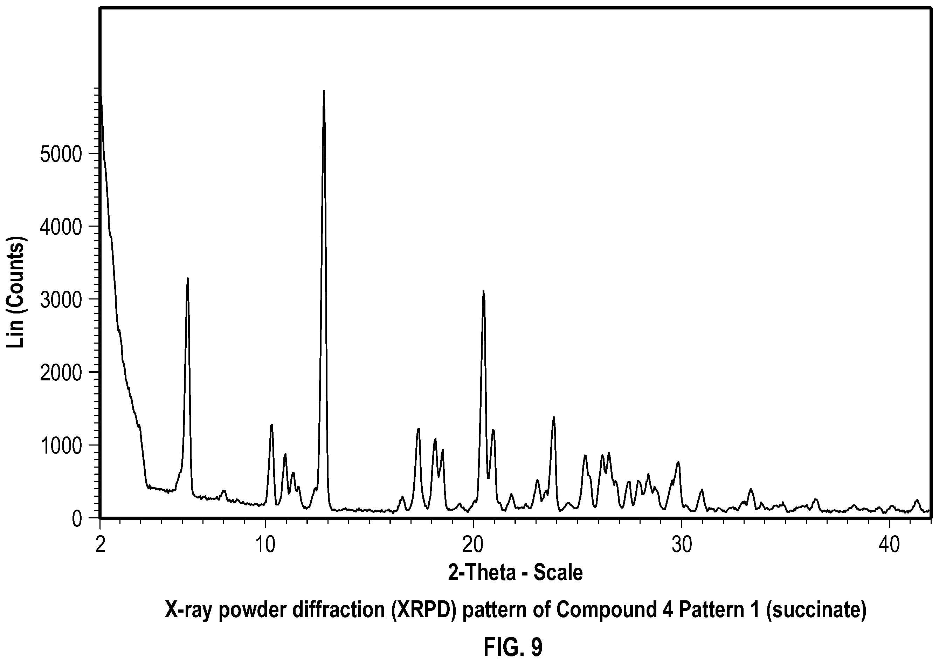

In certain embodiments, the X-ray powder diffraction pattern of Compound **4** Pattern is substantially similar to the XRPD provided in **Figure 9****.**

Methods for preparing Compound **4** Pattern 1 are described *infra.*

### 5. Compound 5 (Phosphoric acid × Compound 1)

According to one embodiment, the present invention provides a chemical species Compound 5 comprising Compound 1 and phosphoric acid:

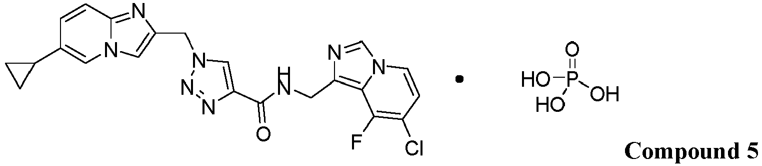

In one embodiment, the solid form of Compound **5** has a stoichiometry of (Compound **1**):(phosphoric acid) that is about 1:1.

In some embodiments, the present invention provides Compound **5** substantially free of impurities. As used herein, the term "substantially free of impurities" means that the compound contains no significant amount of extraneous matter. Such extraneous matter may include excess L-malic acid, excess Compound **1,** residual solvents, or any other impurities that may result from the preparation of, and/or isolation of, Compound **5.** In certain embodiments, at least about 95% by weight of Compound **5** is present. In still other embodiments of the invention, at least about 99% by weight of Compound **5** is present.

According to one embodiment, Compound **5** is present in an amount of at least about 97.0, 97.5, 98.0, 98.5, 99.0, 99.5, or 99.8 weight percent where the percentages are based on the total weight of the composition. According to another embodiment, Compound **5** contains no more than about 3.0 area percent HPLC of total organic impurities and, in certain embodiments, no more than about 1.5 area percent HPLC total organic impurities relative to the total area of the HPLC chromatogram. In other embodiments, Compound 5 contains no more than about 1.0% area percent HPLC of any single impurity; no more than about 0.6 area percent HPLC of any single impurity, and, in certain embodiments, no more than about 0.5 area percent HPLC of any single impurity, relative to the total area of the HPLC chromatogram.

The structure depicted for Compound 5 is also meant to include all tautomeric forms of Compound **5.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are within the scope of this invention.

It has been found that Compound **5** can exist in a variety of solid forms. Exemplary such forms include polymorphs such as those described herein.

In some embodiments, Compound **5** is amorphous. In some embodiments, Compound **5** is amorphous, and is substantially free of crystalline Compound **5.**

In certain embodiments, Compound **5** is a crystalline solid. In other embodiments, Compound **5** is a crystalline solid substantially free of amorphous Compound **5.** As used herein, the term "substantially free of amorphous Compound **5**" means that the compound contains no significant amount of amorphous Compound **5.** In certain embodiments, at least about 95% by weight of crystalline Compound **5** is present. In still other embodiments of the invention, at least about 99% by weight of crystalline Compound **5** is present.

It has been found that Compound **5** can exist in at least one polymorphic form. In some embodiments, the present invention provides a polymorphic form of Compound **5** referred to herein as Pattern 1.

### Compound 5 Pattern 1

In some embodiments, Compound **5** Pattern 1 has at least 1, 2, 3, 4 or 5 X-ray Powder Diffraction (XRPD) peaks selected from the angles (2 theta ± 0.2) listed in **Table 11** below.

**Table 11. XRPD Peak Positions for Compound 5 Pattern 1**

| **Angle** / °**2θ** | **Angle** / °**2θ** |
|---|---|
| 4.7 | 19.4 |
| 6.1 | 21.4 |
| 12.1 | 22.8 |
| 13.6 | 23.6 |
| 14.2 | 24.6 |
| 15.0 | 25.3 |
| 16.4 | 27.4 |
| 17.0 | 28.7 |
| 17.4 | 30.9 |
| 18.1 | 31.6 |

In some embodiments, Compound **5** Pattern 1 is characterized by an X-ray powder diffraction (XRPD) pattern having diffractions at angles (2 theta ± 0.2) and corresponding d-spacing (angstroms ± 0.2) of:

**Table 12. XRPD Peak Positions and d-Spacing for Compound 5 Pattern 1**

| **Angle / ° 2θ** | **d- spacing / Angstrom** | **Angle / ° 2θ** | **d- spacing / Angstrom** |
|---|---|---|---|
| 4.7 | 18.68 | 19.4 | 4.58 |
| 6.1 | 14.45 | 21.4 | 4.15 |
| 12.1 | 7.33 | 22.8 | 3.89 |
| 13.6 | 6.51 | 23.6 | 3.77 |
| 14.2 | 6.24 | 24.6 | 3.62 |
| 15.0 | 5.91 | 25.3 | 3.51 |
| 16.4 | 5.40 | 27.4 | 3.25 |
| 17.0 | 5.20 | 28.7 | 3.11 |
| 17.4 | 5.10 | 30.9 | 2.89 |
| 18.1 | 4.91 | 31.6 | 2.83 |

In some embodiments, Compound **5** Pattern 1 is characterized in that it has one or more peaks in its X-ray powder diffraction pattern selected from those at about 4.7, 6.1, 17.0, 17.4, 18.1, and 24.6. In some embodiments, Compound **5** Pattern 1 is characterized in that it has two or more peaks in its X-ray powder diffraction pattern selected from those at about 4.7, 6.1, 17.0, 17.4, 18.1, and 24.6. In some embodiments, Compound **5** Pattern 1 is characterized in that it has three or more peaks in its X-ray powder diffraction pattern selected from those at about 4.7, 6.1, 17.0, 17.4, 18.1, and 24.6. In some embodiments, Compound **5** Pattern 1 is characterized in that it has four or more peaks in its X-ray powder diffraction pattern selected from those at about 4.7, 6.1, 17.0, 17.4, 18.1, and 24.6. In some embodiments, Compound **5** Pattern 1 is characterized in that it has five or more peaks in its X-ray powder diffraction pattern selected from those at about 4.7, 6.1, 17.0, 17.4, 18.1, and 24.6. In some embodiments, Compound **5** Pattern 1 is characterized in that it has all six peaks in its X-ray powder diffraction pattern selected from those at about 4.7, 6.1, 17.0, 17.4, 18.1, and 24.6. In some embodiments, Compound **5** Pattern 1 is characterized in that it has all six peaks in its X-ray powder diffraction pattern selected from those at about 4.7, 6.1, 17.0, 17.4, 18.1, and 24.6, corresponding to d-spacing (angstroms ± 0.2) of 18.68, 14.45, 5.20, 5.10, 4.91, and 3.62 (respectively). .

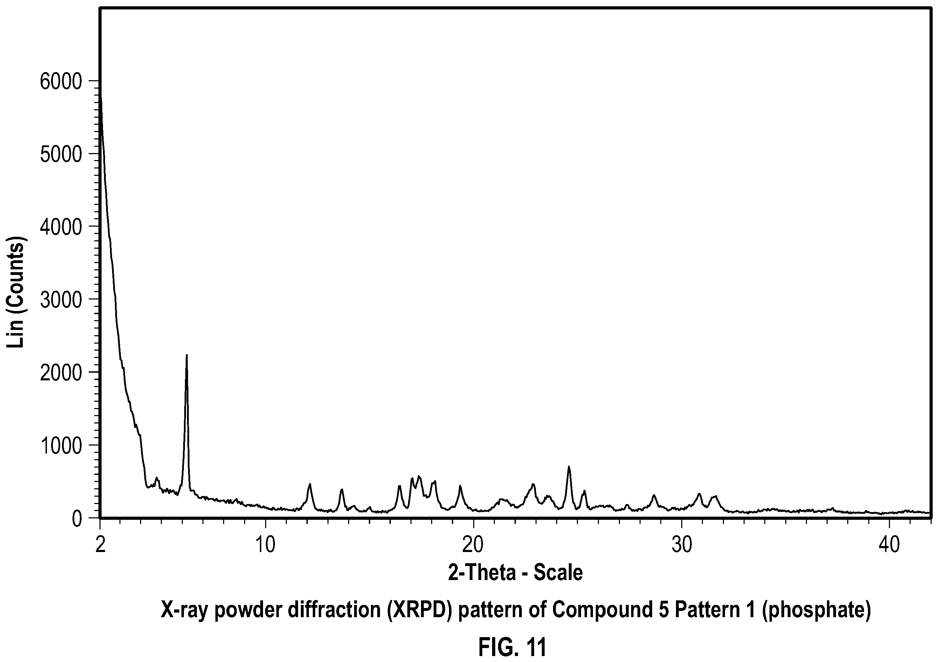

In certain embodiments, the X-ray powder diffraction pattern of Compound 5 Pattern 1 is substantially similar to the XRPD provided in **Figure 11****.**

Methods for preparing Compound **5** Pattern 1 are described *infra.*

### 6. Compound 6 (Oxalic acid × Compound 1)

According to one embodiment, the present invention provides a chemical species Compound **6** comprising Compound **1** and oxalic acid:

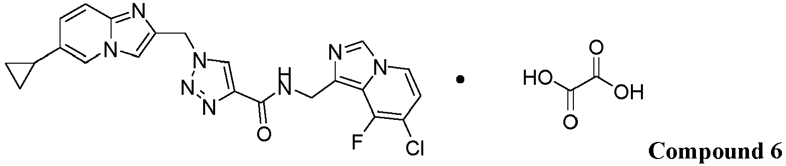

In one embodiment, the solid form of Compound 6 has a stoichiometry of (Compound **1**):(oxalic acid) that is about 1:1.

In some embodiments, the present invention provides Compound **6** substantially free of impurities. As used herein, the term "substantially free of impurities" means that the compound contains no significant amount of extraneous matter. Such extraneous matter may include excess oxalic acid, excess Compound **1,** residual solvents, or any other impurities that may result from the preparation of, and/or isolation of, Compound **6.** In certain embodiments, at least about 95% by weight of Compound **6** is present. In still other embodiments of the invention, at least about 99% by weight of Compound **6** is present.

According to one embodiment, Compound **6** is present in an amount of at least about 97.0, 97.5, 98.0, 98.5, 99.0, 99.5, or 99.8 weight percent where the percentages are based on the total weight of the composition. According to another embodiment, Compound **6** contains no more than about 3.0 area percent HPLC of total organic impurities and, in certain embodiments, no more than about 1.5 area percent HPLC total organic impurities relative to the total area of the HPLC chromatogram. In other embodiments, Compound **6** contains no more than about 1.0 area percent HPLC of any single impurity; no more than about 0.6 area percent HPLC of any single impurity, and, in certain embodiments, no more than about 0.5 area percent HPLC of any single impurity, relative to the total area of the HPLC chromatogram.

The structure depicted for Compound **6** is also meant to include all tautomeric forms of Compound **6.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are within the scope of this invention.

It has been found that Compound **6** can exist in a variety of solid forms. Exemplary such forms include polymorphs such as those described herein.

In some embodiments, Compound **6** is amorphous. In some embodiments, Compound **4** is amorphous, and is substantially free of crystalline Compound **6.**

In certain embodiments, Compound **6** is a crystalline solid. In other embodiments, Compound **6** is a crystalline solid substantially free of amorphous Compound **6.** As used herein, the term "substantially free of amorphous Compound **6**" means that the compound contains no significant amount of amorphous Compound **6.** In certain embodiments, at least about 95% by weight of crystalline Compound **6** is present. In still other embodiments of the invention, at least about 99% by weight of crystalline Compound **6** is present.

It has been found that Compound **6** can exist in at least two polymorphic forms. In some embodiments, the present invention provides a polymorphic form of Compound **6** referred to herein as Pattern 1. In some embodiments, the present invention provides a polymorphic form of Compound **6** referred to herein as Pattern 4.

### Compound 6 Pattern 1

In some embodiments, Compound 6 Pattern 1 has at least 1, 2, 3, 4 or 5 X-ray Powder Diffraction (XRPD) peaks selected from the angles (2 theta ± 0.2) listed in **Table 13** below.

**Table 13. XRPD Peak Positions for Compound 6 Pattern 1**

| **Angle / ° 2θ** | **Angle / ° 2θ** | **Angle / ° 2θ** |
|---|---|---|
| 4.7 | 15.9 | 24.0 |
| 6.1 | 16.4 | 24.2 |
| 7.1 | 16.7 | 24.6 |
| 8.4 | 17.0 | 25.3 |
| 9.3 | 17.3 | 26.1 |
| 11.9 | 18.7 | 27.3 |
| 13.6 | 19.7 | 27.9 |
| 14.0 | 20.3 | 28.7 |
| 14.9 | 21.7 | 29.8 |
| 15.4 | 22.0 | 30.9 |

In some embodiments, Compound **6** Pattern 1 is characterized by an X-ray powder diffraction (XRPD) pattern having diffractions at angles (2 theta ± 0.2) and corresponding d-spacing (angstroms ± 0.2) of:

**Table 14. XRPD Peak Positions and d-Spacing for Compound 6 Pattern 1**

| **Angle / ° 2θ** | **d- spacing / Angstrom** | **Angle / ° 20** | **d- spacing / Angstrom** |
|---|---|---|---|
| 4.7 | 18.88 | 18.7 | 4.73 |
| 6.1 | 14.36 | 19.7 | 4.50 |
| 7.1 | 12.43 | 20.3 | 4.37 |
| 8.4 | 10.58 | 21.7 | 4.09 |
| 9.3 | 9.51 | 22.0 | 4.03 |
| 11.9 | 7.44 | 24.0 | 3.71 |
| 13.6 | 6.51 | 24.2 | 3.67 |
| 14.0 | 6.32 | 24.6 | 3.62 |
| 14.9 | 5.96 | 25.3 | 3.52 |
| 15.4 | 5.74 | 26.1 | 3.42 |
| 15.9 | 5.56 | 27.3 | 3.26 |
| 16.4 | 5.40 | 27.9 | 3.19 |
| 16.7 | 5.30 | 28.7 | 3.11 |
| 17.0 | 5.20 | 29.8 | 2.99 |
| 17.3 | 5.12 | 30.9 | 2.90 |

In some embodiments, Compound **6** Pattern 1 is characterized in that it has one or more peaks in its X-ray powder diffraction pattern selected from those at about 4.7, 6.1, 18.7, 24.0, 24.2, and 24.6. In some embodiments, Compound **6** Pattern 1 is characterized in that it has two or more peaks in its X-ray powder diffraction pattern selected from those at about 4.7, 6.1, 18.7, 24.0, 24.2, and 24.6. In some embodiments, Compound **6** Pattern 1 is characterized in that it has three or more peaks in its X-ray powder diffraction pattern selected from those at about 4.7, 6.1, 18.7, 24.0, 24.2, and 24.6. In some embodiments, Compound **6** Pattern 1 is characterized in that it has four or more peaks in its X-ray powder diffraction pattern selected from those at about 4.7, 6.1, 18.7, 24.0, 24.2, and 24.6. In some embodiments, Compound **6** Pattern 1 is characterized in that it has five or more peaks in its X-ray powder diffraction pattern selected from those at about 4.7, 6.1, 18.7, 24.0, 24.2, and 24.6. In some embodiments, Compound **6** Pattern 1 is characterized in that it has all six peaks in its X-ray powder diffraction pattern selected from those at about 4.7, 6.1, 18.7, 24.0, 24.2, and 24.6. In some embodiments, Compound **6** Pattern 1 is characterized in that it has all six peaks in its X-ray powder diffraction pattern selected from those at about 4.7, 6.1, 18.7, 24.0, 24.2, and 24.6, corresponding to d-spacing (angstroms ± 0.2) of 18.88, 14.36, 4.73, 3.71, 3.67, and 3.62 (respectively).

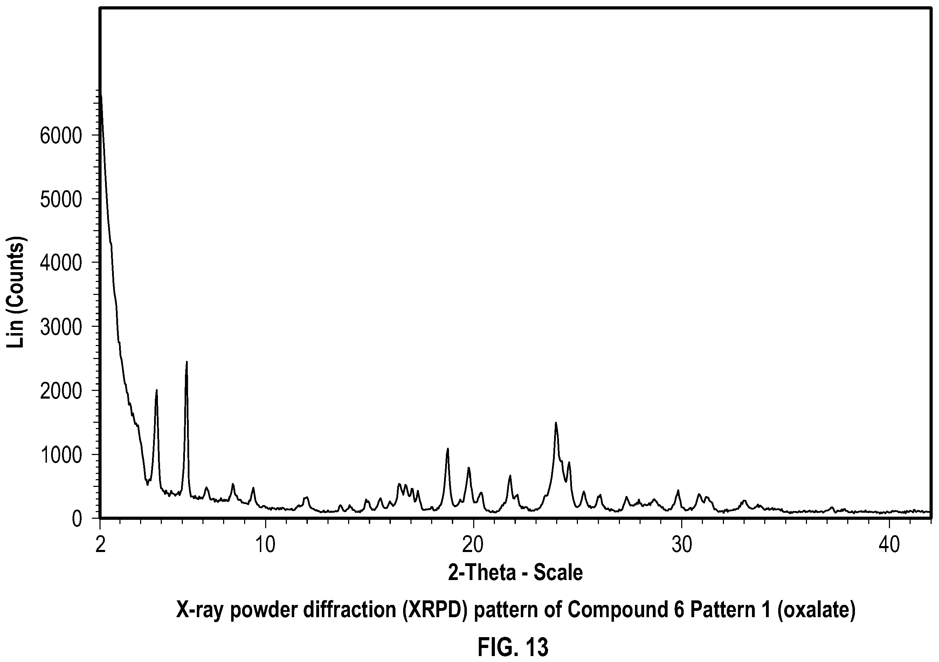

In certain embodiments, the X-ray powder diffraction pattern of Compound **6** Pattern 1 is substantially similar to the XRPD provided in **Figure 13****.**

Methods for preparing Compound **6** Pattern 1 are described *infra.*

### Compound 6 Pattern 4

In some embodiments, Compound 6 Pattern 4 has at least 1, 2, 3, 4 or 5 X-ray Powder Diffraction (XRPD) peaks selected from the angles (2 theta ± 0.2) listed in **Table 15** below.

**Table 15. XRPD Peak Positions for Compound 6 Pattern 4**

| **Angle / ° 2θ** | **Angle / ° 2θ** | **Angle / ° 2θ** |
|---|---|---|
| 6.4 | 18.2 | 24.0 |
| 9.7 | 19.2 | 24.5 |
| 11.6 | 19.5 | 25.5 |
| 11.9 | 20.0 | 26.1 |
| 12.7 | 20.9 | 26.6 |
| 14.0 | 21.6 | 27.2 |
| 15.6 | 22.6 | 28.1 |
| 16.7 | 23.3 | 29.1 |

In some embodiments, Compound **6** Pattern 4 is characterized by an X-ray powder diffraction (XRPD) pattern having diffractions at angles (2 theta ± 0.2) and corresponding d-spacing (angstroms ± 0.2) of:

**Table 16. XRPD Peak Positions and d-Spacing for Compound 6 Pattern 4**

| **Angle / ° 2θ** | **d- spacing / Angstrom** | **Angle / ° 2θ** | **d- spacing / Angstrom** |
|---|---|---|---|
| 6.4 | 13.89 | 20.9 | 4.25 |
| 9.7 | 9.08 | 21.6 | 4.10 |
| 11.6 | 7.65 | 22.6 | 3.93 |
| 11.9 | 7.43 | 23.3 | 3.81 |
| 12.7 | 6.97 | 24.0 | 3.71 |
| 14.0 | 6.31 | 24.5 | 3.62 |
| 15.6 | 5.67 | 25.5 | 3.49 |
| 16.7 | 5.31 | 26.1 | 3.42 |
| 18.1 | 4.87 | 26.6 | 3.35 |
| 19.2 | 4.63 | 27.2 | 3.27 |
| 19.5 | 4.55 | 28.1 | 3.18 |
| 20.0 | 4.45 | 29.2 | 3.06 |

In some embodiments, Compound **6** Pattern 4 is characterized in that it has one or more peaks in its X-ray powder diffraction pattern selected from those at about 6.4, 11.9, 19.2, 26.1, 26.6, and 27.2. In some embodiments, Compound **6** Pattern 4 is characterized in that it has two or more peaks in its X-ray powder diffraction pattern selected from those at about 6.4, 11.9, 19.2, 26.1, 26.6, and 27.2. In some embodiments, Compound **6** Pattern 4 is characterized in that it has three or more peaks in its X-ray powder diffraction pattern selected from those at about 6.4, 11.9, 19.2, 26.1, 26.6, and 27.2. In some embodiments, Compound **6** Pattern 4 is characterized in that it has four or more peaks in its X-ray powder diffraction pattern selected from those at about 6.4, 11.9, 19.2, 26.1, 26.6, and 27.2. In some embodiments, Compound **6** Pattern 4 is characterized in that it has five or more peaks in its X-ray powder diffraction pattern selected from those at about 6.4, 11.9, 19.2, 26.1, 26.6, and 27.2. In some embodiments, Compound **6** Pattern 4 is characterized in that it has all six peaks in its X-ray powder diffraction pattern selected from those at about 6.4, 11.9, 19.2, 26.1, 26.6, and 27.2. In some embodiments, Compound 6 Pattern 4 is characterized in that it has all six peaks in its X-ray powder diffraction pattern selected from those at about 6.4, 11.9, 19.2, 26.1, 26.6, and 27.2, corresponding to d-spacing (angstroms ± 0.2) of 13.89, 7.43, 4.63, 3.42, 3.35, and 3.27 (respectively).

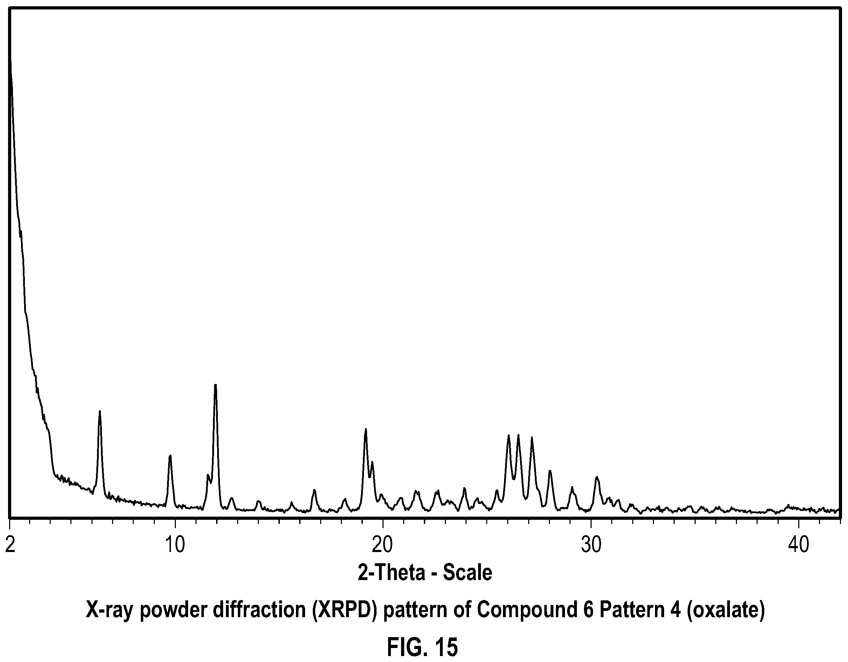

In certain embodiments, the X-ray powder diffraction pattern is substantially similar to the XRPD provided in **Figure 15****.**

Methods for preparing Compound **6** Pattern 4 are described *infra.*

### 7. Compound 7 (L-tartaric acid × Compound 1)

According to one embodiment, the present invention provides a chemical species Compound 7 comprising Compound **1** and L-tartaric acid:

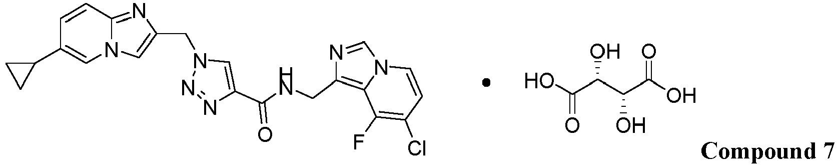

In one embodiment, the solid form of Compound 7 has a stoichiometry of (Compound **1**):(L-tartaric acid) that is about 1:1.

In some embodiments, the present invention provides Compound 7 substantially free of impurities. As used herein, the term "substantially free of impurities" means that the compound contains no significant amount of extraneous matter. Such extraneous matter may include excess L-tartaric acid, excess Compound **1,** residual solvents, or any other impurities that may result from the preparation of, and/or isolation of, Compound 7. In certain embodiments, at least about 95% by weight of Compound 7 is present. In still other embodiments of the invention, at least about 99% by weight of Compound 7 is present.

According to one embodiment, Compound 7 is present in an amount of at least about 97.0, 97.5, 98.0, 98.5, 99.0, 99.5, or 99.8 weight percent where the percentages are based on the total weight of the composition. According to another embodiment, Compound 7 contains no more than about 3.0 area percent HPLC of total organic impurities and, in certain embodiments, no more than about 1.5 area percent HPLC total organic impurities relative to the total area of the HPLC chromatogram. In other embodiments, Compound **7** contains no more than about 1.0 area percent HPLC of any single impurity; no more than about 0.6 area percent HPLC of any single impurity, and, in certain embodiments, no more than about 0.5 area percent HPLC of any single impurity, relative to the total area of the HPLC chromatogram.

The structure depicted for Compound **7** is also meant to include all tautomeric forms of Compound **7.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are within the scope of this invention.

It has been found that Compound **7** can exist in a variety of solid forms. Exemplary such forms include polymorphs such as those described herein.

In some embodiments, Compound **7** is amorphous. In some embodiments, Compound **7** is amorphous, and is substantially free of crystalline Compound **7.**

In certain embodiments, Compound **7** is a crystalline solid. In other embodiments, Compound **7** is a crystalline solid substantially free of amorphous Compound **7.** As used herein, the term "substantially free of amorphous Compound **7**" means that the compound contains no significant amount of amorphous Compound **7.** In certain embodiments, at least about 95% by weight of crystalline Compound **7** is present. In still other embodiments of the invention, at least about 99% by weight of crystalline Compound **7** is present.

It has been found that Compound 7 can exist in at least one polymorphic form. In some embodiments, the present invention provides a polymorphic form of Compound **7** referred to herein as Pattern 1.

### 8. Compound 8 (methanesulfonic acid × Compound 1)

Described herein is a chemical species Compound **8** comprising Compound **1** and methanesulfonic acid:

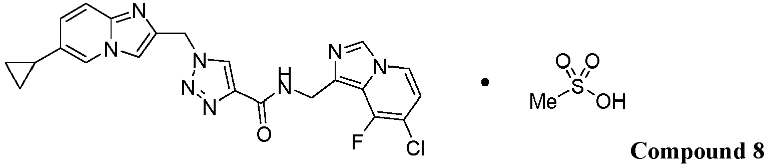

The solid form of Compound **8** may have a stoichiometry of (Compound **1**):(methanesulfonic acid) that is about 1:1.

Compound **8** may be substantially free of impurities. As used herein, the term "substantially free of impurities" means that the compound contains no significant amount of extraneous matter. Such extraneous matter may include excess methanesulfonic acid, excess Compound **1,** residual solvents, or any other impurities that may result from the preparation of, and/or isolation of, Compound **8.** At least about 95% by weight of Compound **8** may be present. At least about 99% by weight of Compound **8** may be present.

Compound **8** may be present in an amount of at least about 97.0, 97.5, 98.0, 98.5, 99.0, 99.5, or 99.8 weight percent where the percentages are based on the total weight of the composition. Compound **8** may contain no more than about 3.0 area percent HPLC of total organic impurities and, in certain aspects, no more than about 1.5 area percent HPLC total organic impurities relative to the total area of the HPLC chromatogram. Compound **8** may contain no more than about 1.0 area percent HPLC of any single impurity; no more than about 0.6 area percent HPLC of any single impurity, and, in certain aspects, no more than about 0.5 area percent HPLC of any single impurity, relative to the total area of the HPLC chromatogram.

The structure depicted for Compound **8** is also meant to include all tautomeric forms of Compound **8.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are included.

It has been found that Compound **8** can exist in a variety of solid forms. Exemplary such forms include polymorphs such as those described herein.

Compound **8** may be amorphous. Compound **8** may be amorphous, and substantially free of crystalline Compound **8.**

Compound **8** may be a crystalline solid. Compound **8** may be a crystalline solid substantially free of amorphous Compound **8.** As used herein, the term "substantially free of amorphous Compound **8**" means that the compound contains no significant amount of amorphous Compound **8.** At least about 95% by weight of crystalline Compound **8** may be present. At least about 99% by weight of crystalline Compound **8** may be present.

It has been found that Compound **8** can exist in at least one polymorphic form. Described herein are a polymorphic form of Compound **8** referred to herein as Pattern 1, a polymorphic form of Compound **8** referred to herein as Pattern 2, and a polymorphic form of Compound **8** referred to herein as Pattern 3.

### 9. Compound 9 (Fumaric acid × Compound 1)

Described herein is a chemical species Compound **9** comprising Compound 1 and fumaric acid:

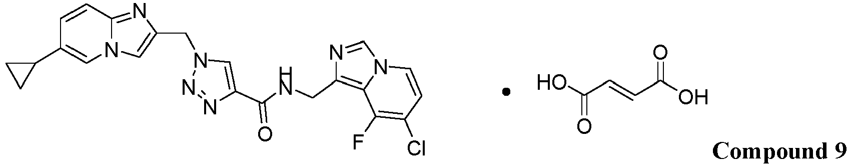

The solid form of Compound **9** may havea stoichiometry of (Compound **1**):(fumaric acid) that is about 1:1.

Compound **9** may be substantially free of impurities. As used herein, the term "substantially free of impurities" means that the compound contains no significant amount of extraneous matter. Such extraneous matter may include excess fumaric acid, excess Compound **1,** residual solvents, or any other impurities that may result from the preparation of, and/or isolation of, Compound **9.** At least about 95% by weight of Compound **9** may be present. At least about 99% by weight of Compound **9** may be present.

Compound **9** may be present in an amount of at least about 97.0, 97.5, 98.0, 98.5, 99.0, 99.5, or 99.8 weight percent where the percentages are based on the total weight of the composition. Compound **9** may contain no more than about 3.0 area percent HPLC of total organic impurities and, in certain aspects, no more than about 1.5 area percent HPLC total organic impurities relative to the total area of the HPLC chromatogram. Compound **9** may contain no more than about 1.0 area percent HPLC of any single impurity; no more than about 0.6 area percent HPLC of any single impurity, and, in certain aspects, no more than about 0.5 area percent HPLC of any single impurity, relative to the total area of the HPLC chromatogram.

The structure depicted for Compound **9** is also meant to include all tautomeric forms of Compound **9.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are included

It has been found that Compound **9** can exist in a variety of solid forms. Exemplary such forms include polymorphs such as those described herein.

Compound **9** may be amorphous. Compound **9** may be amorphous, and substantially free of crystalline Compound **9.**

Compound **9** may be a crystalline solid. Compound **9** may be a crystalline solid substantially free of amorphous Compound **9.** As used herein, the term "substantially free of amorphous Compound 9" means that the compound contains no significant amount of amorphous Compound **9.** At least about 95% by weight of crystalline Compound **9** may be present. At least about 99% by weight of crystalline Compound **9** may be present.

It has been found that Compound **9** can exist in at least one polymorphic form. Described herein is a polymorphic form of Compound **9** referred to herein as Pattern 1.

### Compound 9 Pattern 1

Compound **9** Pattern 1 may have at least 1, 2, 3, 4 or 5 X-ray Powder Diffraction (XRPD) peaks selected from the angles (2 theta ± 0.2) listed in **Table 17** below.

**Table 17. XRPD Peak Positions for Compound 9 Pattern 1**

| **Angle / ° 2θ** | **Angle / ° 2θ** | **Angle / ° 2θ** |
|---|---|---|
| 6.1 | 18.1 | 25.3 |
| 8.0 | 18.4 | 26.1 |
| 10.2 | 19.3 | 26.5 |
| 10.8 | 20.4 | 26.9 |
| 11.2 | 20.9 | 27.4 |
| 11.5 | 21.2 | 27.9 |
| 12.2 | 21.7 | 28.8 |
| 12.7 | 22.5 | 29.6 |
| 16.5 | 23.0 | 30.9 |
| 17.3 | 23.8 | 28.4 |
| 17.5 | 24.5 | |

Compound **9** Pattern 1 may be characterized by an X-ray powder diffraction (XRPD) pattern having diffractions at angles (2 theta ± 0.2) and corresponding d-spacing (angstroms ± 0.2) of:

**Table 18. XRPD Peak Positions and d-Spacing for Compound 9 Pattern 1**

| **Angle / ° 2θ** | **d- spacing / Angstrom** | **Angle / ° 2θ** | **d- spacing / Angstrom** |
|---|---|---|---|
| 6.1 | 14.40 | 21.2 | 4.19 |
| 8.0 | 11.06 | 21.7 | 4.09 |
| 10.2 | 8.66 | 22.5 | 3.95 |
| 10.8 | 8.19 | 23.0 | 3.86 |
| 11.2 | 7.89 | 23.8 | 3.74 |
| 11.5 | 7.67 | 24.5 | 3.63 |
| 12.2 | 7.23 | 25.3 | 3.52 |
| 12.7 | 6.96 | 26.1 | 3.41 |
| 16.5 | 5.38 | 26.5 | 3.36 |
| 17.3 | 5.12 | 26.9 | 3.32 |
| 17.5 | 5.07 | 27.4 | 3.26 |
| 18.1 | 4.89 | 27.9 | 3.20 |
| 18.4 | 4.81 | 28.8 | 3.10 |
| 19.3 | 4.59 | 29.6 | 3.01 |
| 20.4 | 4.34 | 30.9 | 2.89 |
| 20.9 | 4.25 | 28.4 | 3.14 |

Compound **9** Pattern 1 may be characterized in that it has one or more peaks in its X-ray powder diffraction pattern selected from those at about 6.1, 10.8, 12.7, 20.4, 25.3, and 26.5. Compound **9** Pattern 1 may be characterized in that it has two or more peaks in its X-ray powder diffraction pattern selected from those at about 6.1, 10.8, 12.7, 20.4, 25.3, and 26.5. Compound **9** Pattern 1 may be characterized in that it has three or more peaks in its X-ray powder diffraction pattern selected from those at about 6.1, 10.8, 12.7, 20.4, 25.3, and 26.5. Compound **9** Pattern 1 may be characterized in that it has four or more peaks in its X-ray powder diffraction pattern selected from those at about 6.1, 10.8, 12.7, 20.4, 25.3, and 26.5. Compound **9** Pattern 1 may be characterized in that it has five or more peaks in its X-ray powder diffraction pattern selected from those at about 6.1, 10.8, 12.7, 20.4, 25.3, and 26.5. Compound **9** Pattern 1 may be characterized in that it has all six peaks in its X-ray powder diffraction pattern selected from those at about 6.1, 10.8, 12.7, 20.4, 25.3, and 26.5. Compound **6** Pattern 4 may be characterized in that it has all six peaks in its X-ray powder diffraction pattern selected from those at about 6.1, 10.8, 12.7, 20.4, 25.3, and 26.5, corresponding to d-spacing (angstroms ± 0.2) of 14.4, 8.19, 6.96, 4.34, 3.52, and 3.36 (respectively).

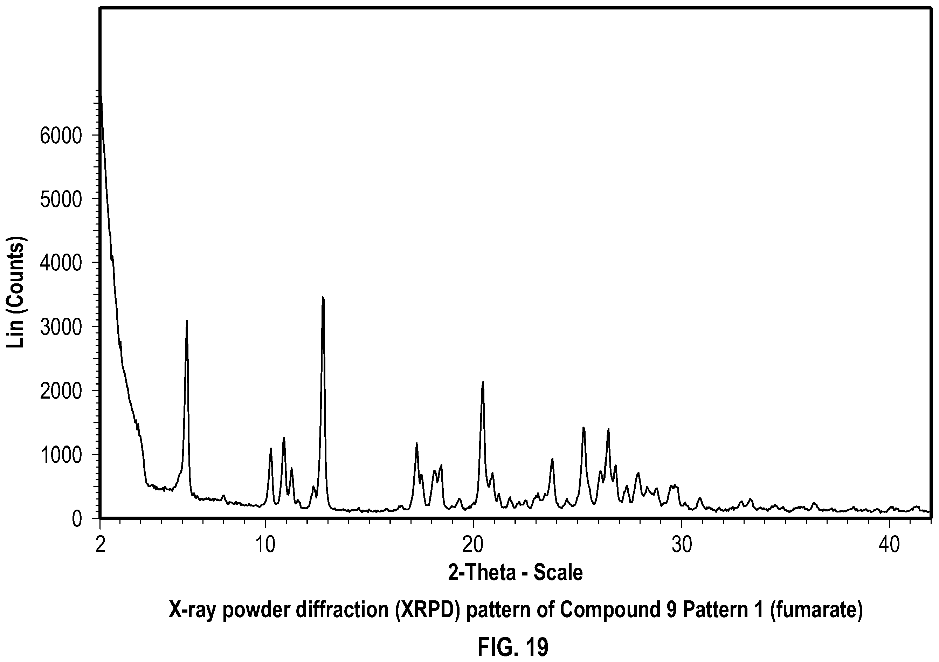

The X-ray powder diffraction pattern of Compound **9** Pattern 1 may be substantially similar to the XRPD provided in **Figure 19****.**

Methods for preparing Compound **9** Pattern 1 are described *infra.*

### III. Cocrystal forms

### 10. Compound 10 (Methyl gallate × Compound 1)

Described herein is a chemical species Compound **10** comprising Compound **1** and methyl gallate:

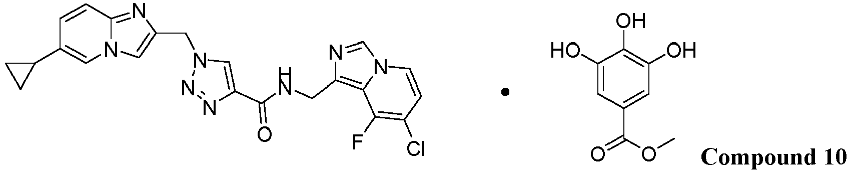

The solid form of Compound **10** may have a stoichiometry of (Compound **1**):(methyl gallate) that is about 1:1.

Compound **10** may be substantially free of impurities. As used herein, the term "substantially free of impurities" means that the compound contains no significant amount of extraneous matter. Such extraneous matter may include excess methyl gallate, excess Compound **1,** residual solvents, or any other impurities that may result from the preparation of, and/or isolation of, Compound **10.** At least about 95% by weight of Compound **10** may be present. At least about 99% by weight of Compound **10** may be present.

Compound **10** may be present in an amount of at least about 97.0, 97.5, 98.0, 98.5, 99.0, 99.5, or 99.8 weight percent where the percentages are based on the total weight of the composition. Compound **10** may contain no more than about 3.0 area percent HPLC of total organic impurities and, in certain embodiments, no more than about 1.5 area percent HPLC total organic impurities relative to the total area of the HPLC chromatogram. Compound **10** may contain no more than about 1.0 area percent HPLC of any single impurity; no more than about 0.6 area percent HPLC of any single impurity, and, in certain aspects, no more than about 0.5 area percent HPLC of any single impurity, relative to the total area of the HPLC chromatogram.

The structure depicted for Compound **10** is also meant to include all tautomeric forms of Compound **10.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are included.

It has been found that Compound **10** can exist in a variety of solid forms. Exemplary such forms include polymorphs such as those described herein.

Compound **10** may be amorphous. Compound **10** may be amorphous, and is substantially free of crystalline Compound **10.**

Compound **10** may be a crystalline solid. Compound **10** may be a crystalline solid substantially free of amorphous Compound **10.** As used herein, the term "substantially free of amorphous Compound **10**" means that the compound contains no significant amount of amorphous Compound **10.** At least about 95% by weight of crystalline Compound **10** may be present. At least about 99% by weight of crystalline Compound **10** may be present.

It has been found that Compound **10** can exist in at least one polymorphic form. Described herein is a polymorphic form of Compound **10** referred to herein as Pattern 1.

### 11. Compound 11 (Propyl gallate × Compound 1)

Described herein is a chemical species Compound **11** comprising Compound **1** and propyl gallate:

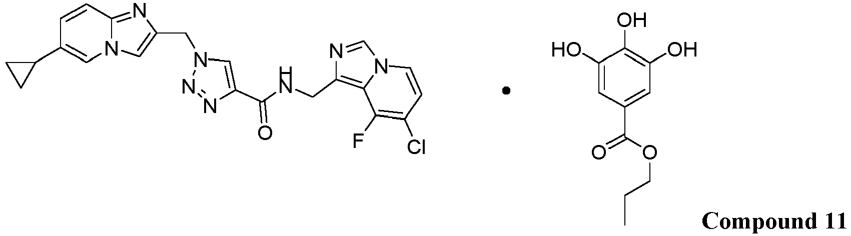

The solid form of Compound **11** may have a stoichiometry of (Compound **1**):(propyl gallate) that is about 1:1.

Compound **11** may be substantially free of impurities. As used herein, the term "substantially free of impurities" means that the compound contains no significant amount of extraneous matter. Such extraneous matter may include excess propyl gallate, excess Compound **1,** residual solvents, or any other impurities that may result from the preparation of, and/or isolation of, Compound **11.** At least about 95% by weight of Compound **11** may be present. At least about 99% by weight of Compound **11** may be present.

Compound **11** may be present in an amount of at least about 97, 97.5, 98.0, 98.5, 99, 99.5, 99.8 weight percent where the percentages are based on the total weight of the composition. Compound **11** may contain no more than about 3.0 area percent HPLC of total organic impurities and, in certain aspects, no more than about 1.5 area percent HPLC total organic impurities relative to the total area of the HPLC chromatogram. Compound 11 may contain no more than about 1.0 area percent HPLC of any single impurity; no more than about 0.6 area percent HPLC of any single impurity, and, in certain aspects, no more than about 0.5 area percent HPLC of any single impurity, relative to the total area of the HPLC chromatogram.

The structure depicted for Compound **11** is also meant to include all tautomeric forms of Compound **11.** Additionally, structures depicted here are also meant to include compounds that differ only in the presence of one or more isotopically enriched atoms. For example, compounds having the present structure except for the replacement of hydrogen by deuterium or tritium, or the replacement of a carbon by a ¹³C- or ¹⁴C-enriched carbon are included.

It has been found that Compound **11** can exist in a variety of solid forms. Exemplary such forms include polymorphs such as those described herein.

Compound **11** may be a crystalline solid. Compound **11** may be a crystalline solid substantially free of amorphous Compound **11.** As used herein, the term "substantially free of amorphous Compound **11**" means that the compound contains no significant amount of amorphous Compound **11.** At least about 95% by weight of crystalline Compound **11** may be present. At least about 99% by weight of crystalline Compound **11** may be present.

It has been found that Compound **11** can exist in at least one polymorphic form. Described herein is a polymorphic form of Compound **11** referred to herein as Pattern 1.

Compound **11** may be amorphous. Compound **11** may be amorphous, and substantially free of crystalline Compound **11.**

### General Methods of Providing the Compounds

Compound **1** is prepared according to the methods described in detail in the '129 publication.

The acid addition compounds of general formula A, which formula encompasses, *inter alia,* Compounds **2** through **9,** and/or particular forms thereof, are prepared from Compound **1,** according to the general Scheme below.

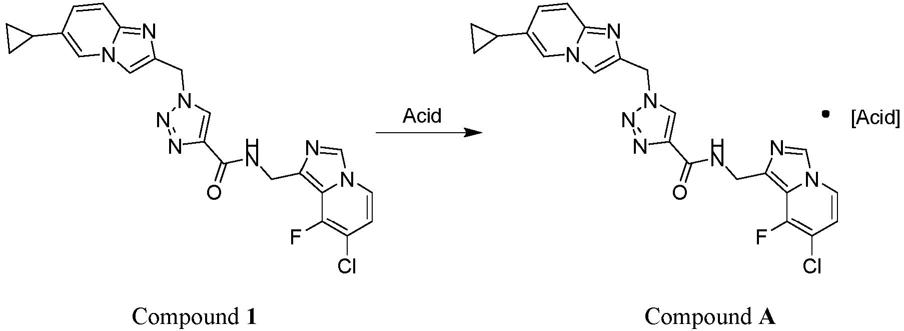

In this scheme, "Acid" represents, e.g., any of the co-formers described herein. For instance, each of Compounds **2** through **9,** and forms thereof, are prepared from Compound **1** by combining Compound **1** with an appropriate acid to form the product Compound. Thus, also described herein is a method for preparing Compounds **2** through **9,** and forms thereof, by combining Compound **1** with an appropriate acid to form the product Compound.

As described generally above, described herein is a method for preparing Compound **A:** 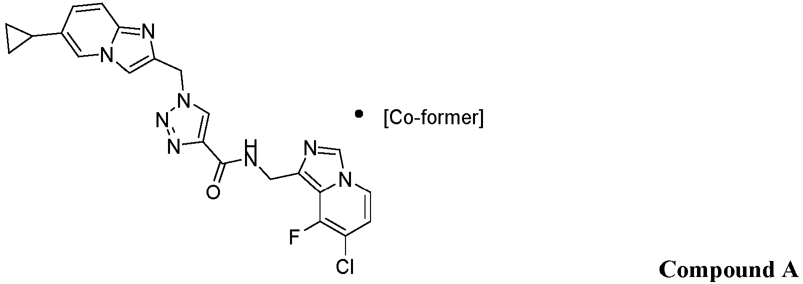 comprising steps of:
combining Compound **1:** 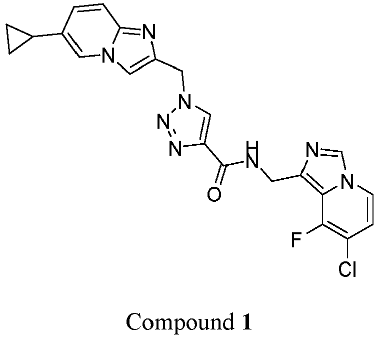 with a suitable co-former (e.g., a suitable acid) and optionally a suitable solvent under conditions suitable for forming Compound A.

Compound **1** may be treated with a co-former selected from: hydrochloric acid, sulfuric acid, p-toluenesulfonic acid, methanesulfonic acid, oxalic acid, L-malic acid, phosphoric acid, gentisic acid, salicylic acid, L-tartric acid, fumaric acid, citric acid, 4-amino salicylic acid, L-maleic acid, benzoic acid, succinic acid, nicotinic acid, sorbic acid, methyl gallate and propyl gallate.

A suitable co-former is hydrochloric acid.

A suitable co-former is sulfuric acid.

IA suitable co-former is p-toluenesulfonic acid.

A suitable co-former is methanesulfonic acid.

A suitable co-former is oxalic acid.

A suitable co-former is L-malic acid.

A suitable co-former is phosphoric acid.

A suitable co-former is gentisic acid.

A suitable co-former is salicylic acid.

A suitable co-former is L-tartaric acid.

A suitable co-former is fumaric acid.

A suitable co-former is citric acid.

A suitable co-former is 4-amino salicylic acid.

A suitable co-former is maleic acid.

A suitable co-former is benzoic acid.

A suitable co-former is succinic acid.

A suitable co-former is nicotinic acid.

A suitable co-former is sorbic acid.

A suitable co-former is methyl gallate.

A suitable co-former is propyl gallate.

A suitable solvent may be any solvent system (e.g., one solvent or a mixture of solvents) in which Compound 1 and/or an acid are soluble, or are at least partially soluble.

Examples of suitable solvents include, but are not limited to protic solvents, aprotic solvents, polar aprotic solvent, or mixtures thereof. Suitable solvents include an ether, an ester, an alcohol, a ketone, or a mixture thereof. A solvent may be one or more organic alcohols. A solvent may be chlorinated. A solvent A may bean aromatic solvent.

A suitable solvent may be methanol, ethanol, isopropanol, t-butanol, acetonitrile, tetrahydrofuran (THF), or acetone wherein said solvent is anhydrous or in combination with water or dichloromethane (DCM). Suitable solvents may include n-heptane, ethyl acetate, methyl ethyl ketone (MEK), tert-butyl methyl ether (TBME), isopropyl acetate (IPAC), methyl isobutyl ketone (MIBK), dimethylformamide (DMF), dimethylacetamide (DMAC), dimethylsulfoxide (DMSO), toluene, trifluorotoluene, anisole, chlorobenzene, cumene, or N-methylpyrrolidone (NMP). A suitable solvent may be acetone. A suitable solvent may be methanol. A suitable solvent may be ethyl acetate. A suitable solvent may ba combination of said solvents.

Described herein is a method for preparing a free base form of Compound **1** or Compound **A,** comprising one or more steps of removing a solvent and adding a solvent. An added solvent may bthe same as the solvent removed. An added solvent may bdifferent from a solvent removed. Means of solvent removal are known in the synthetic and chemical arts and include, but are not limited to, any of those described herein and in the Exemplification.

A method for preparing a free base form of Compound **1** or Compound **A** may comprise one or more steps of heating or cooling a preparation.

A method for preparing a free base form of Compound **1** or Compound **A** may comprise one or more steps of agitating or stirring a preparation.

A method for preparing a free base form of Compound **1** or Compound **A** may comprise a step of adding a suitable co-former to a solution or slurry of compound **1.**

A method for preparing a free base form of Compound **1** or Compound **A** may comprise a step of adding a suitable acid to a solution or slurry of compound **1.**

A method for preparing a free base form of Compound **1** or Compound **A** may comprise a step of heating.

A free base form of Compound **1** or Compound **A** may precipitate from the mixture. A free base form of Compound **1** or Compound **A** may crystallize from the mixture. A free base form of Compound **1** or Compound **A** may crystallize from solution following seeding of the solution (i.e., adding crystals of a free base form of Compound **1** or Compound A to the solution).

A free base form of Compound **1** or Compound **A** can precipitate out of the reaction mixture, or be generated by removal of part or all of the solvent through methods such as evaporation, distillation, filtration (e.g., nanofiltration, ultrafiltration), reverse osmosis, absorption and reaction, by adding a suitable anti-solvent, for example but not limited to, heptane, cumene, toluene, and TBME, by cooling or by different combinations of these methods.

As described generally above, a free base form of Compound **1** or Compound **A** is optionally isolated. It will be appreciated that a free base form of Compound **1** or Compound **A** may be isolated by any suitable physical means known to one of ordinary skill in the art. Precipitated solid free base form of Compound **1** or Compound **A** may be separated from the supernatant by filtration. Precipitated solid free base form of Compound **1** or Compound **A** may be separated from the supernatant by decanting the supernatant.

A free base form of Compound **1** or Compound **A** may be separated from the supernatant by filtration.

An isolated free base form of Compound **1** or Compound **A** may bdried in air. Isolated free base form of Compound **1** or Compound **A** may bdried under reduced pressure, optionally at elevated temperature.

### Methods of Use

Compounds described herein (e.g., any of Compounds **1-11**) are for use in medicine. Compounds described herein may be useful as serine protease zymogen inhibitor. Compounds described herein may be selective inhibitors of plasma kallikrein (pKal). Described herein are methods of decreasing pKal activity. Such methods include contacting pKal with an effective amount of a provided compound. Therefore, further described herein are methods of inhibiting pKal activity by contacting pKal with a compound described herein.

Provided compounds may be useful for the treatment of diseases and disorders that may be alleviated by inhibiting (i.e., decreasing) pKal activity. By "diseases" is meant diseases or disease symptoms. Thus, described herein are methods of treating pKal-mediated disorders in a subject in need thereof. Such methods include administering to the subject a therapeutically effective amount of a provided compound.

Exemplary pKal-mediated disorders include edema, which refers to swelling in the whole body of a subject or a part thereof due to inflammation or injury when small blood vessels become leaky and releases fluid into nearby tissues. In some examples, the edema is hereditary angioedema (HAE). In other examples, the edema occurs in eyes, *e.g.,* diabetic macular edema (DME). Described herein are methods of inhibiting the activity of pKal. Described herein is a method of inhibiting the activity of pKal *in vitro* via contacting any of the compounds described herein with pKal molecules in a sample, such as a biological sample. Described herein is a method of inhibiting the activity of pKal *in vivo* via delivering an effective amount of any of the compounds described herein to a subject in need of the treatment through a suitable route.

Provided methods may comprise administering to a subject in need thereof *(e.g.,* a subject such as a human patient with edema) any of the compounds described herein. The methods may comprise administering a compound of **1-11,** or a pharmaceutically acceptable composition thereof, to a subject in need thereof. The method may comprise administering a pharmaceutical composition comprising a compound of **1-11.**

The subject to be treated by any of the methods described herein may be a human patient having, suspected of having, or at risk for edema, for example, HAE or DME. A subject having an edema can be identified by routine medical examination, e.g., laboratory tests. A subject suspected of having an edema might show one or more symptoms of the disease/disorder. A subject at risk for edema can be a subject having one or more of the risk factors associated with the disease, for example, deficiency in C1 inhibitor (C1-INH) as for HAE.

Described herein are methods of alleviating one or more symptoms of HAE in a human patient who is suffering from an HAE attack. Such a patient can be identified by routine medical procedures. An effective amount of one or more of the provided compounds can be given to the human patient via a suitable route, for example, those described herein. The compounds described herein may be used alone, or may be used in combination with other anti-HAE agents, for example but not limited to, a C1 esterase inhibitor (e.g., Cinryze^{®} or Berinert^{®}), a pKal inhibitor (*e.g*., ecallantide or lanadelumab) or a bradykinin B2 receptor antagonist (*e.g.*, Firazyr^{®}).

Described herein are methods or reducing the risk of HAE attack in a human HAE patient who is in quiescent stage. Such a patient can be identified based on various factors, including history of HAE attack. An effective amount of one or more of the compounds can be given to the human patient *via* a suitable route, for example, those described herein. The compounds described herein may be used alone, or may be used in combination with other anti-HAE agents, for example but not limited to, a C1 esterase inhibitor (e.g., Cinryze^{®} or Berinert^{®}), a pKal inhibitor (*e.g*., ecallantide or lanadelumab) or a bradykinin B2 receptor antagonist (e.g., Firazyr^{®}).

Described herein is prophylactic treatment of HAE in human patients having risk to HAE attacks with one or more of the compounds described herein. Patients suitable for such prophylactic treatment may be human subjects having history of HAE attacks (*e.g*., human subjects experiencing more than 2 attacks per month). Alternatively, patients suitable for the prophylactic treatment may be human subjects having no HAE attack history but bearing one or more risk factors for HAE (*e.g*., family history, genetic defects in C1-INH gene, etc.) Such prophylactic treatment may involve the compounds described herein as the sole active agent, or involve additional anti-HAE agents, such as those described herein.

Described herein are methods for preventing or reducing edema in an eye of a subject (*e.g*., a human patient). In some examples, the human patient is a diabetic having, suspected of having, or at risk for diabetic macular edema (DME). DME is the proliferative form of diabetic retinopathy characterized by swelling of the retinal layers, neovascularization, vascular leak, and retinal thickening in diabetes mellitus due to leaking of fluid from blood vessels within the macula. An effective amount of one or more of the compounds described herein, or pharmaceutically acceptable salts thereof, may be delivered into the eye of the subject where treatment is needed. For example, the compound may be delivered by intraocular injection, or intravitreal injection. A subject may be treated with the compound as described herein, either as the sole active agent, or in combination with another treatment for DME. Nonlimiting examples of treatment for DME include laser photocoagulation, steroids, VEGF pathway targeting agents (*e.g*., Lucentis^{®} (ranibizumab) or Eylea^{®} (aflibercept)), and/or anti-PDGF agents.

The methods disclosed herein may comprise administering to the subject an effective amount of a compound of **1-11.** The effective amount may be a therapeutically effective amount. The effective amount may be a prophylactically effective amount.

The subject being treated may ban animal. The animal may be of either sex and may be at any stage of development. The subject may be a mammal. The subject being treated may be a human. The subject may be a domesticated animal, such as a dog, cat, cow, pig, horse, sheep, or goat. The subject may be a companion animal, such as a dog or cat. The subject may ba livestock animal, such as a cow, pig, horse, sheep, or goat. The subject may ba zoo animal. The subject may ba research animal such as a rodent (*e.g.*, mouse, rat), dog, pig, or non-human primate. The animal may ba genetically engineered animal. The animal may ba transgenic animal.

Certain methods described herein may comprise administering one or more additional pharmaceutical agent(s) in combination with the compounds described herein. The additional pharmaceutical agent(s) may be administered at the same time as the compound of **1-11,** or at different times than the compound of **1-11.** For example, the compound of Formulae **1-11** and any additional pharmaceutical agent(s) may be on the same dosing schedule or different dosing schedules. All or some doses of the compound of **1-11** may be administered before all or some doses of an additional pharmaceutical agent, after all or some does an additional pharmaceutical agent, within a dosing schedule of an additional pharmaceutical agent, or a combination thereof. The timing of administration of the compound of Formulae **1-11** and additional pharmaceutical agents may be different for different additional pharmaceutical agents.

The additional pharmaceutical agent may comprise an agent useful in the treatment of an edema, such as HAE or DME. Examples of such agents are provided herein.

### Assays

To develop useful pKal inhibitors, candidate inhibitors capable of decreasing pKal activity may be identified *in vitro.* The activity of the inhibitor compounds can be assayed utilizing methods known in the art and/or those methods presented herein.

### Pharmaceutical Compositions

Described herein are pharmaceutical compositions comprising any of the compounds described herein (e.g., any of Compounds **1-11**) or any of the compounds described herein (e.g., any of Compounds **1-11**) in combination with a pharmaceutically acceptable excipient (e.g., carrier).

The pharmaceutical compositions include optical isomers, diastereomers, or pharmaceutically acceptable salts of the inhibitors disclosed herein.

A "pharmaceutically acceptable carrier," as used herein refers to pharmaceutical excipients, for example, pharmaceutically, physiologically, acceptable organic or inorganic carrier substances suitable for enteral or parenteral application that do not deleteriously react with the active agent. Suitable pharmaceutically acceptable carriers include water, salt solutions (such as Ringer's solution), alcohols, oils, lipids, gelatins, and carbohydrates such as lactose, amylose or starch, fatty acid esters, hydroxymethycellulose, and polyvinyl pyrrolidine. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention.

The compounds described herein can be administered alone or can be coadministered to the subject. Coadministration is meant to include simultaneous or sequential administration of the compounds individually or in combination (more than one compound). The preparations can also be combined, when desired, with other active substances (e.g. to reduce metabolic degradation).

### Formulations

Compounds described herein can be prepared and administered in a wide variety of oral, parenteral, and topical dosage forms. Thus, the compounds described herein can be administered by injection (e.g. intravenously, intramuscularly, intracutaneously, subcutaneously, intraduodenally, or intraperitoneally). Also, the compounds described herein can be administered by inhalation, for example, intranasally. Additionally, the compounds described herein can be administered transdermally. It is also envisioned that multiple routes of administration (e.g., intramuscular, oral, transdermal) can be used to administer the compounds described herein. Accordingly, also described herein are pharmaceutical compositions comprising a pharmaceutically acceptable carrier or excipient and one or more compounds described herein.

For preparing pharmaceutical compositions from the compounds described herein, pharmaceutically acceptable carriers can be either solid or liquid. Solid form preparations include powders, tablets, pills, capsules, cachets, suppositories, and dispersible granules. A solid carrier can be one or more substance that may also act as diluents, flavoring agents, binders, preservatives, tablet disintegrating agents, or an encapsulating material.

In powders, the carrier is a finely divided solid in a mixture with the finely divided active component. In tablets, the active component is mixed with the carrier having the necessary binding properties in suitable proportions and compacted in the shape and size desired.

The powders and tablets preferably contain from 5% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

When parenteral application is needed or desired, particularly suitable admixtures for the compounds described herein are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampoules are convenient unit dosages. The compounds described herein can also be incorporated into liposomes or administered via transdermal pumps or patches. Suitable pharmaceutical admixtures include those described, for example, in Pharmaceutical Sciences (17th Ed., Mack Pub. Co., Easton, PA) and WO 96/05309.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted according to the particular application and the potency of the active component. The composition can, if desired, also contain other compatible therapeutic agents.

### Examples

The examples below are meant to illustrate certain embodiments of the invention, and not to limit the scope of the invention.

### General Experimental

### Abbreviations

| | |
|---|---|
| ¹H NMR | Proton Nuclear Magnetic Resonance |
| API | Active Pharmaceutical Ingredient |
| ASR | Analytical Service Report |
| ca. | Approximately |
| CCD | Charge Coupled Detector |
| DCM | Dichloromethane |
| DMSO | Dimethyl sulfoxide |
| DSC | Differential Scanning Calorimetry |
| DVS | Dynamic Vapour Sorption |
| eq | Equivalents |
| EtOH | Ethanol |
| GVS | Gravimetric Vapour Sorption |
| H₂O | Water |
| HPLC | High Performance Liquid Chromatography |
| IC | Ion Chromatography |
| ID | Identification |
| IDR | Intrinsic Dissolution Rate |
| IP | Intellectual Property |
| IPA | 2-Propanol |
| IPAC | Isopropyl Acetate |
| KF | Karl Fischer |
| MDSC | Modulated Differential Scanning Calorimetry |
| MeCN | Acetonitrile |
| MEK | Methyl ethyl ketone |
| MeOH | Methanol |
| MIBK | Methyl isobutyl ketone |
| MSZW | Metastable Zone Width |
| N/A | Not Applicable |
| NMP | N-Methylpyrrolidone |
| NMR | Nuclear Magnetic Resonance |
| PLM | Polarised Light Microscopy |
| RH | Relative Humidity |
| RM | Reaction Mixture |
| RT | Room Temperature |
| SCXRD | Single Crystal X-Ray Diffraction |
| TBME | *tert-Butyl* methyl ether |
| t-BuOH | tert-Butanol |
| TFA | Trifluoroacetic acid |
| TGA | Thermal Gravimetric Analysis |
| THF | Tetrahydrofuran |
| UV | Ultraviolet |
| VH-XRPD | Variable Humidity X-Ray Powder Diffraction |
| vol | Volumes |
| VT-XRPD | Variable Temperature X-Ray Powder Diffraction |
| XRPD | X-Ray Powder Diffraction |

### Instruments and Methods

### A. X-ray powder diffraction (XRPD)

For XRPD analysis, the following X-ray powder diffractometers were used. The parameters used are listed in **Table 19.** Unless otherwise stated, 2-theta (2θ) values disclosed herein were obtained using a Bruker AXS D8 Advance with parameters described in **Table 19.**

**Table 19. XRPD Parameters**

| **Instrument** | **Parameters** |
|---|---|
| Bruker AXS C2 GADDS | •Cu Kα radiation (40 kV, 40 mA) |
| | •Angular range: 1.5 to 32.5° 2θ |
| | •Total collection time: 2 min |
| Bruker AXS D8 Advance | •Cu Kα radiation (40 kV, 40 mA) |
| | •Angular range: 2 to 42° 2θ |
| | •Step size: 0.05° 2θ |
| | •Collection time: 0.5 s/step (total |
| | collection time: 6.40 min) |
| PANalytical Empyrean | •Cu Kα radiation (45 kV, 40 mA) |
| | •Angular range: 2.5 to 32.0° 20 |
| | •Step size: 0.0130° 2θ |
| | •Collection time: 12.75 s/step (total collection time of 2.07 min) |

### B. Thermogravimetric (TGA) and Differential Scanning Calorimetry (DSC)

TGA data were collected using a TA Q500/Q5000 TGA from TA Instruments. DSC was performed using a TA Q200/Q2000 DSC from TA Instruments. Detailed parameters used are listed in **Table 20.**

**Table 20. TGA and DSC Parameters**

| **Parameters** | **TGA** | **DSC** |
|---|---|---|
| Method | Ramp | Ramp |
| Sample pan | Platinum, open | Aluminum, crimped |
| Temperature | RT - 350 °C | 25 °C - 310 °C |
| Heating rate | 10 °C/min | 10 °C/min |
| Purge gas | N₂ | N₂ |

### C. HPLC

Agilent 1100 HPLC was utilized and detailed chromatographic conditions are listed in **Table 21.**

**Table 21. Chromatographic conditions and parameters**

| **Parameter** | **Value** | | |
|---|---|---|---|
| Type of method | Reverse phase with gradient elution | | |
| Sample Preparation | 0.25 mg/mL in DMSO | | |
| Column | Supelco Ascentis Express C18, 100 x 4.6 mm, 2.7 µm | | |
| Column Temperature (°C) | 25 | | |
| Injection (µl) | 5 | | |
| Wavelength, Bandwidth (nm) | 255, 90 | | |
| Flow Rate (mL/min) | 2 | | |
| Phase A | 0.1% TFA in water | | |
| Phase B | 0.085% TFA in acetonitrile | | |
| Timetable | **Time (min)** | **% Phase A** | **% Phase B** |
| | 0 | 95 | 5 |
| | 6 | 5 | 95 |
| | 6.2 | 95 | 5 |
| | 8 | 95 | 5 |

### D. Solution NMR

Solution NMR was collected on Bruker 400M NMR Spectrometer using DMSO-*d₆* unless otherwise stated.

### E. Intrinsic Dissolution Rate (IDR)

About 40 mg of the sample was compressed in a 6 mm disc recess, under 100 kg for 2 minutes, with greaseproof paper on the compression base, to form nondisintegrating discs. The discs were then plugged with a bung so that only one surface was exposed to the media during analysis and transferred to the Sirius inForm dissolution apparatus. Analysis was performed at 37 °C in 40 mL media (36 mL ISA water, 4 mL 0.1 M Acetate Phosphate Buffer). Dissolution data were collected over 4 pH sectors (pH 2.0, 5.5, 6.5, and 7.4) for a total of 2 hours - 30 minutes per sector, with UV spectra collected every 30 seconds. A stir speed of 225 rpm was used with a 10 mm path length probe. The IDR was calculated based on the surface area of the 3/6/8 mm disc recess used (28.3 mm2 surface area). XRPD analysis was performed on all samples, both after compression of the material into the disc recess, and post dissolution analysis to observe any change in form. XRPD diffractograms were collected on the Bruker C2.

### Example 1: N-((7-chloro-8-fluoroimidazo[1,5-a]pyridin-1-yl)methyl)-1-((6-cyclopropylimidazo[1,2-a]pyridin-2-yl)methyl)-1H-1,2,3-triazole-4-carboxamide (Compound 1)

The synthesis of Compound **1** is described in detail at Example 148 of the '897 application, which is reproduced herein for ease of reference.

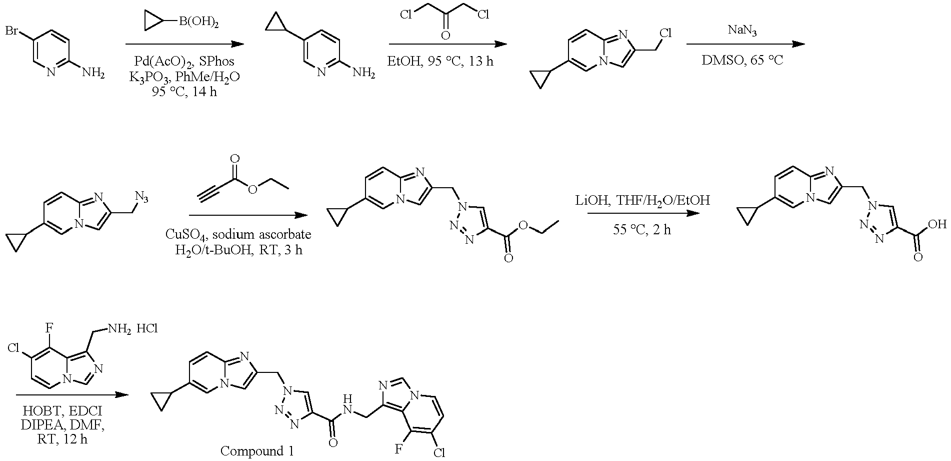

### Synthesis of 5-cyclopropylpyridin-2-amine.

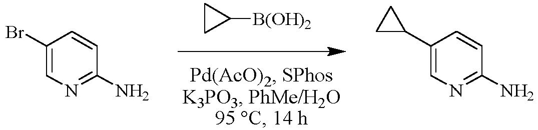

A mixture of 5-bromopyridin-2-amine (100 g, 585 mmol), cyclopropylboronic acid (60 g, 701 mmol), Pd(AcO)₂ (6.5 g, 29 mmol), SPhos (24 g, 58.5 mmol) and K₃PO₄ (372 g, 1.755 mol) in toluene/H₂O (1.2 L/0.12 L) was stirred at 90°C for 14 h under N₂. The reaction was concentrated in vacuo to give the crude, which was purified with silica gel chromatography (PE/EA = 1/2) to give the 5-cyclopropylpyridin-2-amine (61 g, yield: 78%) as a yellow solid. ESI-MS [M+H]⁺: 135.1.

### Synthesis of 2-(chloromethyl)-6-cyclopropylimidazo[1,2-a]pyridine

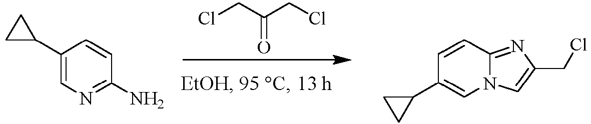

A mixture of 5-cyclopropylpyridin-2-amine (61 g, 455 mmol) and 1,3-dichloropropan-2-one (172 g, 1365 mmol) in EtOH (1 L) was stirred at 95 °C for 13 h. The reaction was concentrated to remove the EtOH. The pH of the residue was adjusted to 9 by addition of aqueous NaHCO₃ and extracted with EtOAc (1 L x 3). The combined organic layers were washed with brine, dried over Na₂SO₄ and concentrated in vacuo to give the crude, which was purified with silica gel chromatography (EA) to give the 2-(chloromethyl)-6-cyclopropylimidazo[1,2-a]pyridine (40 g, yield: 42%) as a yellow solid. ESI-MS [M+H]⁺: 207.1.

### Synthesis of 2-(azidomethyl)-6-cyclopropylimidazo[1,2-a]pyridine.

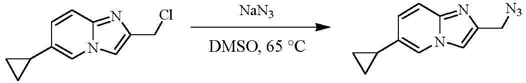

To a solution of 2-(chloromethyl)-6-cyclopropylimidazo[1,2-a]pyridine (40 g, 193 mmol) in DMF (600 mL) was added NaN₃ (18.8 g, 290 mmol). The resulting reaction was stirred at RT for 2 h. The reaction was diluted with H₂O (500 mL) and extracted with EtOAc (500 mL x 3). The combined organic layers were washed with brine, dried over Na₂SO₄ and concentrated in vacuo to give the crude, which was purified with silica gel chromatography (PE/EA = 2/1) to give the 2-(azidomethyl)-6-cyclopropylimidazo[1,2-a]pyridine (35 g, yield: 85%) as a yellow solid. ESI-MS [M+H]⁺: 214.1.

### Synthesis of ethyl 1-((6-cyclopropylimidazo[1,2-a]pyridin-2-yl)methyl)-1H-1,2,3-triazole-4-carboxylate.

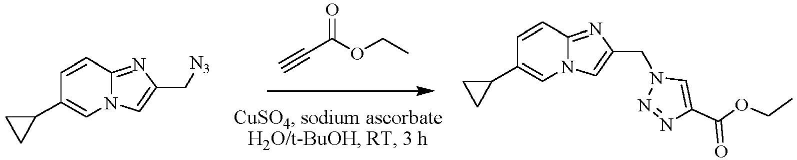

A mixture of 2-(azidomethyl)-6-cyclopropylimidazo[1,2-a]pyridine (35 g, 163.5 mmol), ethyl propiolate (17.6 g, 180 mmol), CuSO₄ (2.6 g, 16.35 mmol) and sodium ascorbate (3.3 g, 16.35 mmol) in H₂O/t-BuOH (150 mL/150 mL) was stirred at RT for 3 h. Yellow solid was precipitated after 3 h and the mixture was filtered. The cake was dried to give the ethyl 1-((6-cyclopropylimidazo[1,2-a]pyridin-2-yl)methyl)-1H-1,2,3-triazole-4-carboxylate (29 g, yield: 57%) as a yellow solid, which was used in the next step without further purification. ESI-MS [M+H]⁺: 312.1.

### Synthesis of 1-((6-cyclopropylimidazo[1,2-a]pyridin-2-yl)methyl)-1H-1,2,3-triazole-4-carboxylic acid.

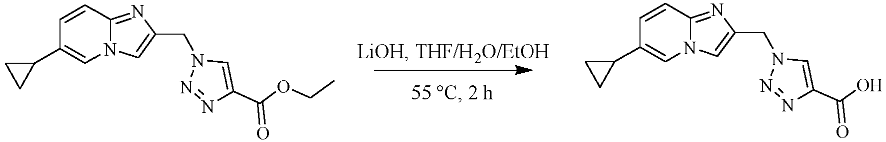

A mixture of ethyl 1-((6-cyclopropylimidazo[1,2-a]pyridin-2-yl)methyl)-1H-1,2,3-triazole-4-carboxylate (29 g, 93.2 mmol) and LiOH (6.7 g, 279.6 mmol, solution in 50 mL H₂O) in THF/EtOH (150 mL/150 mL) was stirred at 50 °C for 2 h. The reaction was concentrated to remove most of the solvent. The pH of the residue was adjusted to 4 by 2 N HCl and a pink solid was precipitated out. The mixture was filtered and the filter cake was dried to give 1-((6-cyclopropylimidazo[1,2-a]pyridin-2-yl)methyl)-1H-1,2,3-triazole-4-carboxylic acid (20 g, 77%) as a pink solid. ESI-MS [M+H]⁺: 284.1.

### Synthesis of N-((7-chloro-8-fluoroimidazo[1,5-a]pyridin-1-yl)methyl)-1-((6-cyclopropylimidazo[1,2-a]pyridin-2-yl)methyl)-1H-1,2,3-triazole-4-carboxamide.

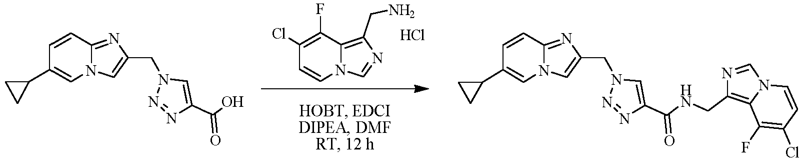

To a suspension of 1-((6-cyclopropylimidazo[1,2-a]pyridin-2-yl)methyl)-1H-1,2,3-triazole-4-carboxylic acid (37 mg, 0.13 mmol) and (7-chloro-8-fluoroimidazo[1,5-a]pyridin-1-yl)methanamine hydrochloride (35 mg, 0.15 mmol) in DMF (3 mL) was added HOBT (40 mg, 0.3 mmol) and EDCI (57 mg, 0.3 mmol), followed by DIPEA (65 mg, 0.5 mmol). The resulting mixture was stirred at RT for 12 h. The reaction mixture was poured into H₂O (15 mL) slowly. The suspension mixture was stirred for 1 h, and filtered. The filtered cake was washed with H₂O (20 mL) and MeOH (20 mL) then dried under vacuum pump to give the N-((7-chloro-8-fluoroimidazo[1,5-a]pyridin-1-yl)methyl)-1-((6-cyclopropylimidazo[1,2-a]pyridin-2-yl)methyl)-1H-1,2,3-triazole-4-carboxamide as pale solid (30 mg, yield: 50%). ESI-MS [M+H]⁺: 465.0. Purity: 98.4% (214 nm), 98.5% (254 nm). ¹H NMR (400 MHz, DMSO-d₆): 8.70 (t, J = 5.4 Hz, 1H), 8.55 (s, 1H), 8.44 (d, J = 2.4 Hz, 1H), 8.35 (s, 1H), 8.21 (d, J = 7.4 Hz, 1H), 7.83 (s, 1H), 7.41 (d, J = 9.3 Hz, 1H), 7.01 (dd, J = 9.4, 1.8 Hz, 1H), 6.76 (dd, J = 7.3, 6.6 Hz, 1H), 5.73 (s, 2H), 4.70 (d, J = 5.5 Hz, 2H), 1.94-1.90 (m, 1H), 0.94-0.89 (m, 2H), 0.69-0.65 (m, 2H).

### Reference Example 2: Solid Forms of Compound 1 (Free base)

Studies were undertaken to identify various new forms (e.g., solid forms) of Compound **1**.

### Solubility Study

Compound **1** obtained from the synthetic procedure described in Example 1 was found to show limited solubility in most of the organic solvents assessed (**Table 22**). In all solvents assessed, the material showed solubility of <10 mg/mL at 25 °C. Solubility of >10 mg/mL was only observed in DMSO, NMP and MeOH:DCM 1:3 at 50 °C.

**Table 22. Solubility of Compound 1 in Various Solvents**

| **Solvent** | **Solubility at 50 °C (mg/mL)** |
|---|---|
| *n*-heptane | <10 |
| Ethyl acetate | <10 |
| IPA | <10 |
| MEK | <10 |
| Acetone | <10 |
| Ethanol | <10 |
| TBME | <10 |
| IPAC | <10 |
| MIBK | <10 |
| DMSO | >10 |
| Toluene | <10 |
| THF | <10 |
| DCM | <10 |
| Methanol | <10 |
| Acetonitrile | <10 |
| t-BuOH | <10 |
| t-BuOH:H₂O 1:1 | <10 |
| Acetonitrile:H₂O 1:1 | <10 |
| DMF | <10 |
| DMAC | <10 |
| NMP | >10 |
| MeOH:DCM 1:3 | >10 |
| MeOH:H₂O 9:1 | <10 |
| THF:H₂O 9:1 | <10 |
| Acetone:H₂O 9:1 | <10 |
| MeOH: Toluene (1:1) | <10 |
| MeOH: Chlorobenzene (1:1) | <10 |
| MeOH:anisole (1:1) | <10 |

### Preparation of Amorphous Form of Compound 1

Compound **1** (30 mg) obtained from the synthetic procedure described in Example 1 was charged to a 2 mL Retsch milling jar equipped with one stainless steel milling ball. This was milled at 30 Hz for 30 minutes. Amorphous form of Compound **1** was successfully prepared by milling at 30 mg scale. The XRPD pattern of the as-prepared material was observed to be free from peaks concomitant with formation of amorphous material.

### Initial Experiments

Polymorph screening experiments were performed using different solution crystallization or solid transition methods. The methods utilized and crystal forms identified are summarized in **Table 23.** Two different solid forms were obtained from these initial screening experiments.

**Table 23. Summary of Polymorph Screening Experiments**

| **Method** | **Isolated Solid Forms** |
|---|---|
| Slurry of Amorphous Material at 50 °C | Form 1 |
| Slurry of Amorphous Material with Temperature Cycling | Form 1 |
| Slurry of Amorphous Material at 5 °C | Form 1 and 2 |
| Antisolvent Crystallization | Form 1 |
| Solvent Assisted Grinding of Free Form | Form 1 and 2 |
| Cooling Crystallization | Form 1 |

### Slurry of Amorphous Material at 50 °C

Slurry conversion experiments were conducted at 50 °C in different solvent systems. About 15 mg of amorphous Compound **1** were suspended in 100 vol (50 vol for DMSO) of solvent in an HPLC vial. After the suspension was stirred for 2 days at 50 °C, the remaining solids were isolated for XRPD analysis. Results summarized in **Table 24** indicated that Compound **1** Form 1 was generated.

**Table 24. Summary of Slurry Conversion Experiments at 50 °C**

| **Solvent** | **Solid Form** |
|---|---|
| *n*-heptane | Compound **1** Form 1 |
| Ethyl acetate | Compound **1** Form 1 |
| IPA | Compound **1** Form 1 |
| MEK | Compound **1** Form 1 |
| Acetone | Compound **1** Form 1 |
| Ethanol | Compound **1** Form 1 |
| TBME | Compound **1** Form 1 |
| IPAC | Compound **1** Form 1 |
| MIBK | Compound **1** Form 1 |
| Toluene | Compound **1** Form 1 |
| THF | Compound **1** Form 1 |
| DCM | Compound **1** Form 1 |
| Methanol | Compound **1** Form 1 |
| Acetonitrile | Compound **1** Form 1 |
| DMSO | Compound **1** Form 1 |

### Slurry of Amorphous Material with Temperature Cycling

15 mg amorphous Compound **1** was dispensed into HPLC vials, to these, aliquots of selected solvent were added and the suspensions heated to 50 °C with magnetic stirring (350 rpm) using an HEL block. Additions of solvent were made until a maximum of 100 vol had been added (50 vol for the DMSO sample). The material was slurried with temperature cycling between 25 °C and 50 °C for 2 days (4 hours at each temperature, with the exception of the first cycle in which a 16 hour isothermal hold at 50 °C was performed to assess dissolution). After this time, solids were isolated by filtration and dried under suction. Solids were analyzed by XRPD. Results summarized in **Table 25** indicated that Compound **1** Form 1 was generated.

**Table 25. Summary of Slurry Conversion Experiments with Temperature Cycling**

| **Solvent** | **Solid Form** |
|---|---|
| *n*-heptane | Compound **1** Form 1 |
| Ethyl acetate | Compound **1** Form 1 |
| IPA | Compound **1** Form 1 |
| MEK | Compound **1** Form 1 |
| Acetone | Compound **1** Form 1 |
| Ethanol | Compound **1** Form 1 |
| TBME | Compound **1** Form 1 |
| IPAC | Compound **1** Form 1 |
| MIBK | Compound **1** Form 1 |
| Toluene | Compound **1** Form 1 |
| THF | Compound **1** Form 1 |
| DCM | Compound **1** Form 1 |
| Methanol | Compound **1** Form 1 |
| Acetonitrile | Compound **1** Form 1 |
| DMSO | Compound **1** Form 1 |

### Slurry of Amorphous Material at 5 °C

Slurry conversion experiments were conducted at 5 °C in different solvent systems. About 15 mg of amorphous Compound **1** were suspended in 100 vol of solvent in an HPLC vial. After the suspension was stirred for 2 days at 5 °C, the remaining solids were isolated for XRPD analysis. Results summarized in **Table 26** indicated that Compound **1** Form 1 or Form 2 was generated.

**Table 26. Summary of Slurry Conversion Experiments at 5 °C**

| **Solvent** | **Solid Form** |
|---|---|
| *n*-heptane | Compound **1** Form 1 |
| Ethyl acetate | Compound **1** Form 1 |
| IPA | Compound **1** Form 1 |
| MEK | Compound **1** Form 1 |
| Acetone | Compound **1** Form 1 |
| Ethanol | Compound **1** Form 1 |
| TBME | Compound **1** Form 1 |
| IPAC | Compound **1** Form 1 |
| MIBK | Compound **1** Form 1 |
| Toluene | Compound **1** Form 1 |
| THF | Compound **1** Form 1 |
| DCM | Compound **1** Form 1 |
| Methanol | Compound **1** Form 2 |
| Acetonitrile | Compound **1** Form 1 |
| DMSO | Compound **1** Form 1 |

### Antisolvent Crystallization

25 mg amorphous form of Compound **1** was charged to 4 mL vials, to these aliquots of selected solvent were added and suspensions heated to 50 °C. Where an observation was made, further additions of solvent were made until dissolution was observed. Antisolvent was then added in 2×1 mL aliquots and an observation made. Solutions were then cooled to 25 °C at 1 °C/min and a further observation made. Further antisolvent (2 mL) was added to DMSO solutions and an observation made. Solids were isolated by filtration, dried under suction and analysed by XRPD. Results summarized in **Table 27** showed that Compound **1** Form 1 was generated.

**Table 27. Summary of Anti-Solvent Addition Experiments**

| **Solvent** | **Antisolvent** | **Solid Form** |
|---|---|---|
| DMSO | Cumene | Compound **1** Form 1 |
| DMSO | Toluene | N/A |
| DMSO | TBME | Compound **1** Form 1 |
| NMP | Cumene | Compound **1** Form 1 |
| NMP | Toluene | Compound **1** Form 1 |
| NMP | TBME | Compound **1** Form 1 |

### Solvent Assisted Grinding of Free Form

20 mg amorphous form of Compound **1** was charged to HPLC vials, to these 2 stainless steel milling balls were added and 20 µL of each selected solvent. Samples were milled on a Fritsch planetary mill equipped with an Automaxion adaptor for 2 hours at 500 rpm, using 2 3 mm ball bearings. Solids were subsequently isolated and analyzed by XRPD. Results summarized in **Table 28** showed that Compound **1** Form 1 and Form 2 were generated.

**Table 28. Summary of Solvent Assisted Grinding of Free Form experiments**

| **Solvent** | **Solid Form** |
|---|---|
| *n*-heptane | Compound **1** Form 1 |
| Ethyl acetate | Compound **1** Form 1 |
| IPA | Compound **1** Form 1 |
| MEK | Compound **1** Form 1 |
| Acetone | Compound **1** Form 1 |
| Ethanol | Compound **1** Form 1 |
| TBME | Compound **1** Form 1 |
| IPAC | Compound **1** Form 1 |
| MIBK | Poorly crystalline |
| Toluene | Compound **1** Form 1 |
| THF | Compound **1** Form 1 |
| DCM | Compound **1** Form 1 |
| Methanol | Mixture of Form 1 and 2 |
| Acetonitrile | Compound **1** Form 1 |
| DMSO | Compound **1** Form 1 |
| DMF | Compound **1** Form 1 |
| DMAC | Compound **1** Form 1 |
| NMP | Compound **1** Form 1 |
| MeOH:DCM 1:3 | Compound **1** Form 1 |
| MeOH:H₂O 9:1 | Compound **1** Form 1 |
| THF:H₂O 9:1 | Compound **1** Form 1 |
| Acetone:H₂O 9:1 | Compound **1** Form 1 |

### Cooling Crystallization

30 mg amorphous form of Compound **1** was charged to HPLC vials, to these aliquots of selected solvent were added and suspensions heated to 100 °C, where an observation was made. Further additions of solvent were made until dissolution was observed. Solutions were subsequently cooled to 25 °C at 0.25 °C/min. Solids were isolated by filtration, dried under suction and analyzed by XRPD. Results summarized in **Table 29** showed that Compound **1** Form 1 was generated.

**Table 29. Summary of Solvent Assisted Grinding of Free Form experiments**

| **Solvent** | **Solid Form** |
|---|---|
| NMP | Compound **1** Form 1 |
| DMSO | Compound **1** Form 1 |

### 2. Characterization Data for Polymorphs of Compound 1

### Compound 1 Form 1

Compound **1** Form 1 can be prepared from an amorphous form of Compound 1. Acetone (100 vol, 42 mL) was charged to the vessel and the amorphous form was slurried at 5 °C with 500 rpm suspended magnetic stirring for 3 days. The solid was subsequently isolated by filtration and dried under suction for 30 minutes. Recovery: 339.33 mg, Yield: 80.2%.

**Table 30. Characterisation of Compound 1 Form 1**

| **Description** | | Compound 1 Form 1 |
|---|---|---|
| **XRPD** | | Free base - Pattern 2 Crystalline consistent with previously isolated material. |
| **DSC** | | Onset of sharp endotherm at 239.6 °C (136.1 J/g). |
| **TGA** | | Start of thermal degradation at ca. 275 °C |
| **HPLC (a.u.c)** | | 98.2 % |
| **GVS** | | 0.5% increase in mass, slightly hygroscopic. Material unchanged by XRPD post GVS |
| **¹H-NMR** | | Consistent with structure. 0.02 molar equivalents of acetone. |

| **PLM** | | Small particles |
|---|---|---|
| **Storage for 7 days** | **40 °C / 75%RH** | Compound **1** Form 1 |
| | **25°C / 97%RH** | Compound **1** Form 1 |
| **IDR Sector 1 average (pH 2.0) / (µg/min/cm²)** | | 104.8 |

**Figure 1** provides an XRPD Compound **1** Form 1.

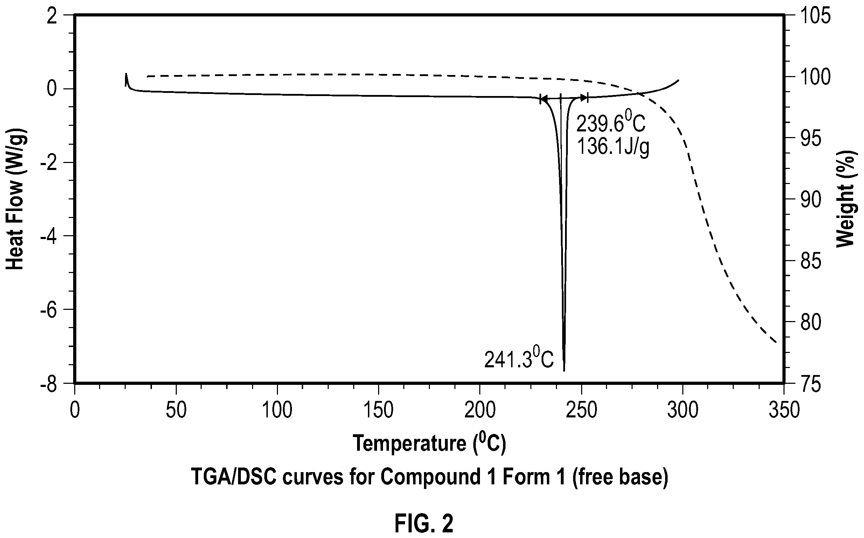

**Figure 2** provides TGA/DSC curves for Compound **1** Form 1.

### Compound 1 Form 2

Compound **1** Form 2 can be prepared from amorphous form of Compound **1.** 393 mg amorphous form of Compound **1** was charged to a 100 mL HEL polyblock vial. Methanol (100 vol, 40 mL), which had been dried with molecular sieves and cooled to 5 °C, was charged to the vessel and the material was slurried at 5 °C with 500 rpm suspended magnetic stirring for 1 hour. After 1 hour, an aliquot of the material was isolated by filtration and partially dried under positive pressure, this was analyzed by XRPD, then allowed to dry and reanalyzed. The remaining solid was subsequently isolated by filtration and dried under suction for 30 minutes. Recovery: 288.74 mg, Yield 73.4 %.

**Table 31. Characterization of Compound 1 Form 2**

| **Description** | | Compound **1** Form 2 |
|---|---|---|
| **XRPD** | | Poorly crystalline, consistent with previously isolated material. |
| **DSC** | | Onset of small exotherm at 131.8 °C (10 J/g), onset of sharp endotherm at 238.2 °C (137 J/g). |
| **TGA** | | Start of thermal degradation at ca. 275 °C |
| **HPLC (a.u.c)** | | 98.3 % |
| **GVS** | | Hygroscopic. 4% increase in mass between 80 and 90% RH. |
| | | Hysteresis observed on humidity decrease. Mixture of Freebase Patterns 2 & 3 by XRPD post GVS. |
| **¹H-NMR** | | Consistent with structure. |
| **PLM** | | Small particles |
| **Storage for seven days** | **40 °C** / **75%RH** | Free base, Compound **1 -** Form 1 |
| | **25°C** / **97%RH** | Mixture of Compound **1 -** Form 1, Compound **1 -** Form 2 and additional peaks. |
| **IDR Sector 1 average (pH 2.0) / (µg/min/cm²)** | | 290.8 (Conversion to Compound **1** - Form 1 during analysis) |

**Figure 3** provides an XRPD pattern of Compound **1** Form 2.

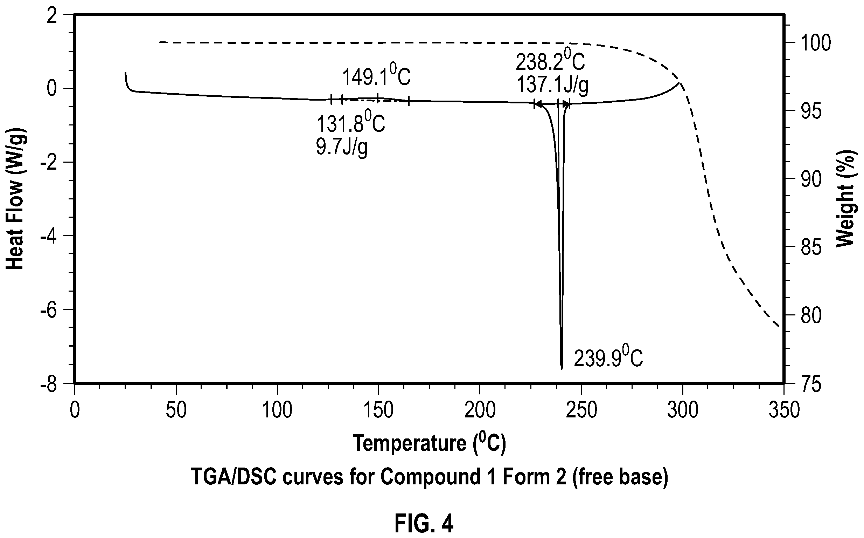

**Figure 4** provides TGA/DSC curves of Compound **1** Form 2.

### Compound 1 Form 3

Compound **1** Form 3 can be prepared from amorphous form of Compound **1.** 502 mg amorphous form of Compound **1** was charged to a 20 mL scintillation vial. To this, acetic acid (5 vol) was added and the sample heated to 50 °C on a Polar Bear with 500 rpm magnetic stirring to give a clear brown solution. This solution was cooled to 20 °C at 1 °C/min, whereupon acetonitrile (15 vol) was added dropwise to give a white precipitate. The sample was then stirred for a further 10 minutes prior to isolation by filtration. The sample was analysed by XRPD and shown to be an intermediate Acetic Acid solvate - Pattern 1, which was heated to 165 °C using a Karl Fischer oven, and held isothermally for 15 minutes to give rise to Compound **1** Form 3. Recovery 343.63 mg

**Table 32. Characterization of Compound 1 Form 3**

| **Description** | | Compound **1** Form 3 |
|---|---|---|
| **XRPD** | | Crystalline, consistent with smaller scale. Compound **1** Form 3 |
| 1 **H-NMR** | | Consistent with structure. No trace acetic acid observed. |
| **TGA** | | 0.3 % mass loss between 100 and 190 °C. Start of thermal degradation at ca. 300 °C |
| **DSC** | | Exotherm with onset 181.6 °C (17 J/g), sharp endotherm with onset at 240.5 °C (145 J/g) |
| **GVS** | | Slightly hygroscopic, 0.5 % increase in mass between 0 and 90 % RH. Unchanged by XRPD post analysis |
| **HPLC** | | 98.7 % |
| **PLM** | | Needles |
| **Storage for One week** | **40 °C / 75%RH** | Unchanged by XRPD |
| | **25 °C / 97%RH** | Unchanged by XRPD |

**Figure 5** provides an XRPD pattern of Compound **1** Form 3.

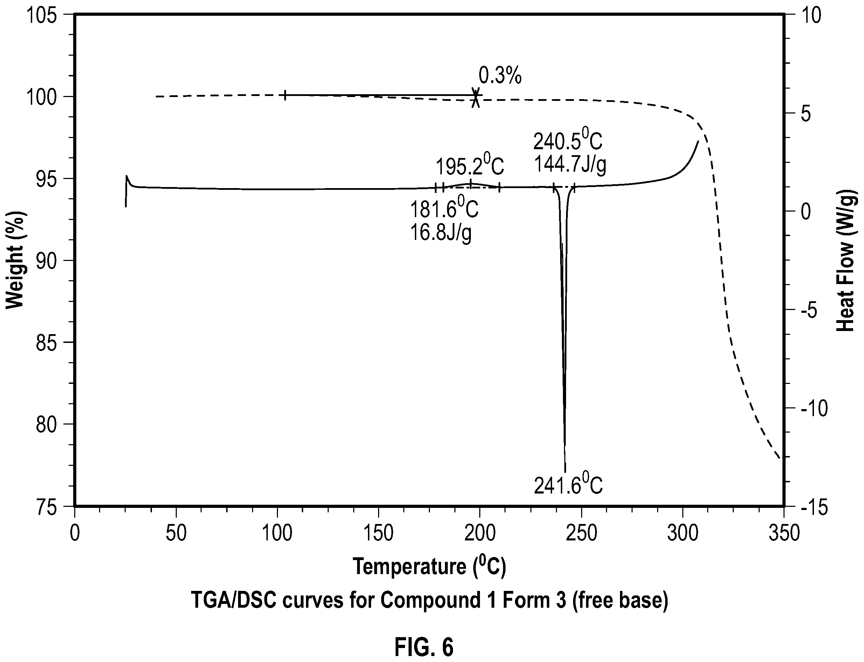

**Figure 6** provides TGA/DSC curves of Compound **1** Form 3.

### 3. Investigation of Thermodynamic Stability Relationships of Compound 1 Free Base Polymorphs

### Competitive Slurry between Compound 1 Form 1 and Form 2

25 mg of a mixture of Compound **1** Form 1 and Form 2 was charged to HPLC vials. To these, 40 vol (1 mL) selected solvents was added and the material slurried at 5 °C or 50 °C for 72 hours on a HEL polyblock (500 rpm stirring). Solids were subsequently isolated by filtration and dried under suction prior to analysis by XRPD. The results of the competitive slurries are shown Table 33. In all but one experiment, Form 1 was the sole detectable phase by XRPD obtained after 72 hours. From this it is therefore evident that in all but one of the solvent mixtures investigated, Form 1 is more thermodynamically stable than Form 2. In the remaining experiment in which the mixture of Form 1 and Form 2 was slurried at 5 °C in methanol, the isolated material was shown to be a mixture of the two forms.

**Table 33. Results of Competitive Slurries of Compound 1 Form 1 and Form 2**

| **Experiment #** | **Solvent** | **Temperature** | **XRPD** |
|---|---|---|---|
| 1 | Methanol | 5 °C | Mixture of Form 1 and Form 2 |
| 2 | Ethanol:H₂O 1:1 | 5 °C | Compound **1** Form 1 |
| 3 | NMP:TBME 1:1 | 5 °C | Compound **1** Form 1 |
| 4 | Acetone | 5 °C | Compound **1** Form 1 |
| 5 | THF | 5 °C | Compound **1** Form 1 |
| 6 | Methanol | 50 °C | Compound **1** Form 1 |
| 7 | Ethanol:H₂O 1:1 | 50 °C | Compound **1** Form 1 |
| 8 | NMP:TBME 1:1 | 50 °C | Compound **1** Form 1 |
| 9 | Acetone | 50 °C | Compound **1** Form 1 |
| 10 | THF | 50 °C | Compound **1** Form 1 |

Amorphous form, Form 1, and Form 2 of Compound **1** were successfully scaled-up and characterized. Form 2 was shown to form *via* a tentatively assigned solvated phase (methanol) which transformed to Form 2 on drying. Form 2 was shown to convert to Form 1 on GVS analysis, and during storage for one week at conditions of 40 °C/75%RH. Peaks corresponding to a suspected hydrated phase were also observed in the XRPD pattern of Form 2 material post storage at conditions of 25 °/97%RH. Amorphous form of Compound **1** was shown to convert to Form 1 upon heating to 150 °C, post GVS analysis and after storage at conditions of 40 °C/75%RH and 25 °C/97%RH for one week. Competitive slurries performed on a mixture of Form 1 and Form 2 (**Table 33**) showed that in the majority of experimental conditions investigated, Form 1 is the more thermodynamically stable polymorph.

### Competitive Slurry between Compound 1 Form 1 and Form 3

120 mg of both Compound **1** Form 3 and Compound **1** Form 1 were dispensed into a 20 mL vial. These were mixed, and then aliquots (20 mg) of this mixture were dispensed into HPLC vials and 90 vol (1.8 mL) selected solvents added. Samples were slurried using a Polar Bear with 500 rpm magnetic stirring at 5 and 50 °C. After 24 hours, aliquots were removed and analysed by XRPD. Experiment numbers: 01-10.

Results from the competitive slurries of Compound **1** Form 1 and Form 3 are shown in **Table 34.** After 24 hours, all samples were shown by XRPD to have converted to Compound **1** Form 1 demonstrating Compound **1** Form 1 to be more thermodynamically stable than Compound **1** Form 3, and the most stable identified freebase form of Compound **1.**

**Table 34. Results from the competitive slurries of Compound 1 Form 1 and Form 3**

| **Experiment #** | **Solvent** | **Temperature / °C** | **XRPD** |
|---|---|---|---|
| 01 | Ethanol | 5 | Compound **1** - Form 1 |
| 02 | Ethyl Acetate | 5 | Compound **1** - Form 1 |
| 03 | Acetone | 5 | Compound **1** - Form 1 |
| 04 | THF | 5 | Compound **1** - Form 1 |
| 05 | Acetonitrile | 5 | Compound **1** - Form 1 |
| 06 | Ethanol | 50 | Compound **1** - Form 1 |
| 07 | Ethyl Acetate | 50 | Compound **1** - Form 1 |
| 08 | Acetone | 50 | Compound **1** - Form 1 |
| 09 | THF | 50 | Compound **1** - Form 1 |
| 10 | Acetonitrile | 50 | Compound **1** - Form 1 |

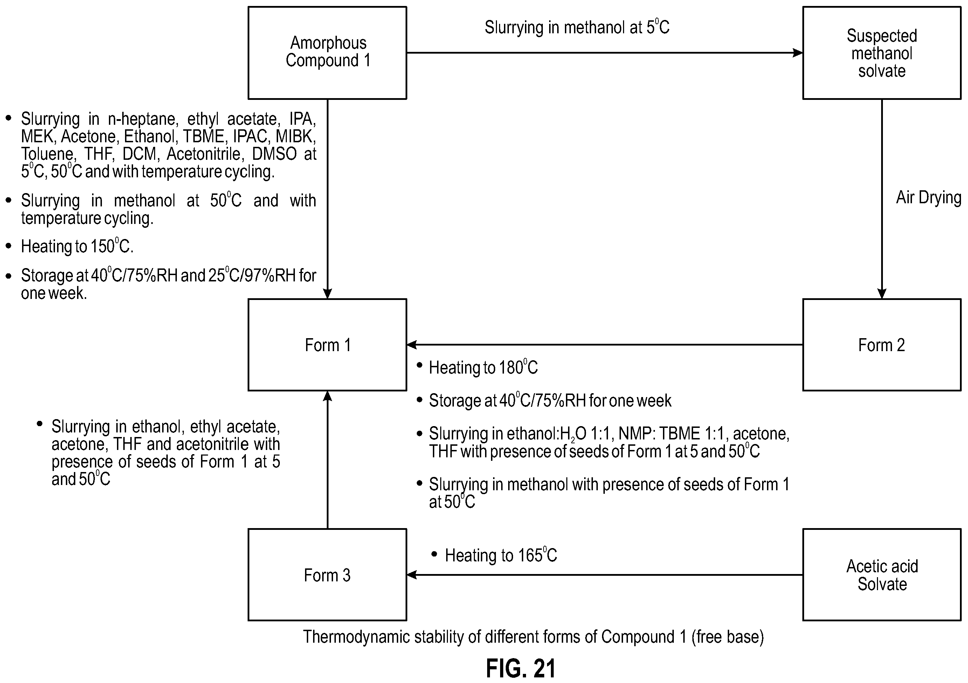

The form diagram of the polymorphs of Compound **1** is shown in **Figure 21****,** and which depicts the relationships between the three crystalline forms, the suspected methanol solvate, the unstable acetic acid solvate, and the amorphous material. As is evident, no conditions were identified for the conversion of Form 1 to any other Form. Taken together, this data demonstrates Form 1 to be the polymorph most stable to typical storage and process conditions.

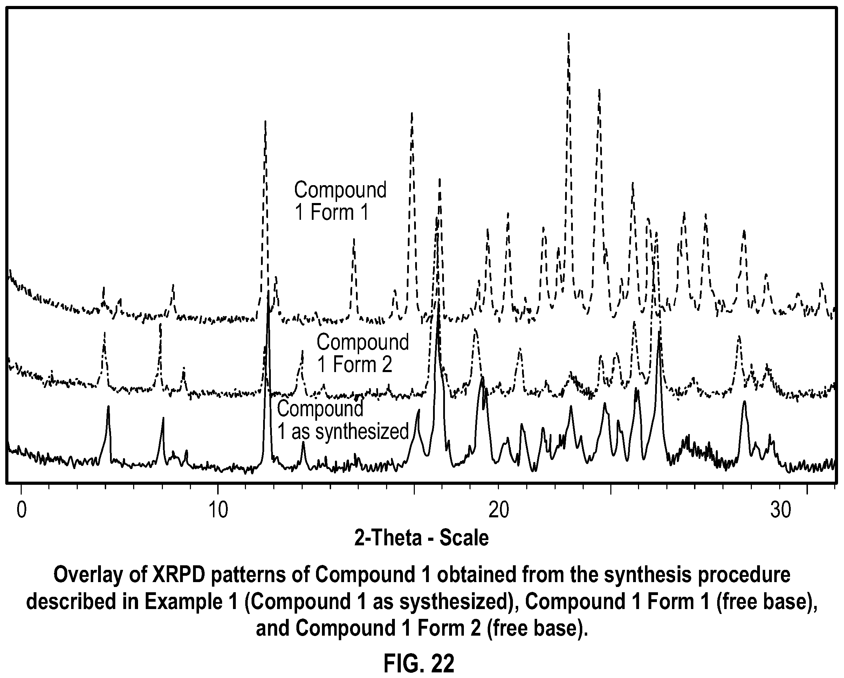

The overlay of the XRPD patterns of Compound **1** obtained from the synthetic procedure described in Example 1, and Compound **1** Form 1 and Form 2 demonstrates that Compound **1** obtained from the synthetic procedure described in Example 1 is composed of a mixture of Compound **1** Form 1 and Form 2 (**Figure 22** - varying peak magnitudes are thought to result from compositional variation and preferred orientation, data obtained by a PANalytical Empyrean with parameters described in **Table 19**).

### Example 3: Initial Salts and Cocrystals

Compound **1** obtained from the synthetic procedure described in Example 1 was used as the starting material for salt and cocrystal experiments.

### 1. Initial HCl Experiments (Reference Example)

Initial salt formation experiments were performed using HCl as the counter-ion. From these experiments thirteen samples with novel XRPD patterns were obtained (**Table 35** and **Table 36**).

### A. Salt Formation with One Equivalent of HCl

15 mg Compound 1 in 1.5 mL HPLC vials was treated with 100 vol of selected solvents at 50 °C with magnetic stirring (500 rpm). 1 equiv. of HCl was added at 50 °C, except for the sample with DCM as solvent which was cooled to RT prior to the addition of the counter-ion. Samples were then cooled to 5 °C at 0.1 °C/min, except for the NMP/DMSO samples which were cooled to 25 °C. All solids were then isolated by filtration and dried under suction. All solutions were allowed to evaporate at ambient conditions. All isolated solids were analyzed by XRPD. The results of the XRPD study are summarized in **Table 35.**

### B. Salt Formation with Two Equivalents of HCl

15 mg Compound 1 in 1.5 mL HPLC vials was treated with 100 vol of selected solvents at 50 °C with magnetic stirring (500 rpm). To these, 2 equivalents of HCl (1 M in THF) was added, an observation was made, and the samples were stirred and held isothermally for 30 minutes. Except for the DCM sample which was cooled to room temperature prior to the addition of HCl. The samples were then slowly cooled to 5 °C at 0.5 °C/min, then held isothermally for 16 hours, except for the DMSO and NMP samples which were cooled to 25 °C. All solids were then isolated by filtration and dried under suction. All solutions were allowed to evaporate at ambient conditions. All isolated solids were analyzed by XRPD. The results of the XRPD study are summarized in **Table 36.**

**Table 35. Results from Salt Screening with One Equivalent of HCl in Various Solvents**

| **Solvent** | **Observation after HCl addition** | **Observation after cooling** | **XRPD analysis damp** | **XRPD analysis dry** |
|---|---|---|---|---|
| *n*-heptane | Cloudy suspension | Cloudy suspension | HCl Salt - Pattern 1 + 2 peaks | Unchanged |
| Ethyl acetate | Cloudy suspension | Cloudy suspension | HCl Salt - Pattern 1 | Insufficient material |
| IPA | Cloudy suspension | Cloudy suspension | HCl Salt - Pattern 1 | Unchanged |
| MEK | Cloudy suspension | Cloudy suspension | HCl Salt - Pattern 1 | Unchanged |
| Acetone | Clear Solution | Cloudy suspension | HCl Salt - Pattern 2 | Unchanged |
| Ethanol | Cloudy suspension | Cloudy suspension | HCl Salt - Pattern 1 | Unchanged |
| TBME | Cloudy suspension | Cloudy suspension | HCl Salt - Pattern 1 | Unchanged |
| IPAC | Cloudy suspension | Cloudy suspension | HCl Salt - Pattern 1 | Unchanged |
| MIBK | Cloudy suspension | Cloudy suspension | HCl Salt - Pattern 1 | Unchanged |
| DMSO | Clear solution | Clear solution | N/A | Form 1 |
| Toluene | Cloudy suspension | Cloudy suspension | HCl Salt - Pattern 1 | Unchanged |
| THF | Cloudy suspension | Cloudy suspension | HCl Salt - Pattern 2 | Insufficient sample |
| DCM | Cloudy suspension | Cloudy suspension | HCl Salt - Pattern 1 | Unchanged |
| Methanol | Clear solution | Clear solution | N/A | HCl Salt - Pattern 10 |
| Acetonitrile | Cloudy suspension | Cloudy suspension | HCl Salt - Pattern 1 + 1 peak | Unchanged |
| T-BuOH | Cloudy suspension | Cloudy suspension | HCl Salt - Pattern 2 | Insufficient sample |
| T-BuOH:H₂O 1:1 | Thin suspension | Thin suspension | Insufficient material | Insufficient sample |
| Acetonitrile:H₂O 1:1 | Clear solution | Clear solution | N/A | HCl Salt - Pattern 11 |
| DMF | Clear solution | Clear solution | N/A | Compound 1 Form 1 |
| DMAC | Clear solution | Clear solution | N/A | Compound 1 Form 1 |
| NMP | Clear solution | Clear solution | N/A | Still a solution at end of study |
| MeOH:DCM 1:3 | Cloudy suspension | Cloudy suspension | N/A | HCl Salt - Pattern 13 |
| MeOH:H₂O 9:1 | Clear solution | Cloudy suspension | N/A | Freebase - Pattern 4 |
| THF:H₂O 9:1 | Clear solution | Cloudy suspension | N/A | HCl Salt - Pattern 1 |
| Acetone:H₂O 9:1 | Cloudy suspension | Cloudy suspension | N/A | HCl Salt - Pattern 1 |

**Table 36. Results from Salt Screening with Two Equivalents of HCl in Various Solvents**

| **Solvent** | **Observation after HCl addition** | **Observation after cooling** | **XRPD analysis damp** | **XRPD analysis dry** |
|---|---|---|---|---|
| *n*-heptane | Cloudy suspension | Cloudy suspension | HCl salt - Pattern 3 | Insufficient sample |
| Ethyl acetate | Cloudy suspension | Cloudy suspension | HCl salt - Pattern 4 | Unchanged |
| IPA | Cloudy suspension | Cloudy suspension | HCl salt - Pattern 1 + 1 peak | Unchanged |
| MEK | Cloudy suspension | Cloudy suspension | HCl salt - Pattern 5 | Unchanged |
| Acetone | Cloudy suspension | Cloudy suspension | HCl salt - Pattern 6 | Unchanged |
| Ethanol | Cloudy suspension | Cloudy suspension | HCl salt - Pattern 7 | Unchanged |
| TBME | Cloudy suspension | Cloudy suspension | HCl salt - Pattern 4 | HCl salt - Pattern 9 |
| IPAC | Cloudy suspension | Cloudy suspension | HCl salt - Pattern 4 | Insufficient sample |
| MIBK | Cloudy suspension | Cloudy suspension | HCl salt - Pattern 4 | HCl salt - Pattern 9 |
| DMSO | Clear solution | Clear solution | N/A | Compound 1 Form 1 |
| Toluene | Cloudy suspension | Cloudy suspension | HCl salt - Pattern 8 | Unchanged |
| THF | Cloudy suspension | Cloudy suspension | HCl salt - Pattern 6 | Unchanged |
| DCM | Cloudy suspension | Cloudy suspension | HCl salt - Pattern 4 | HCl salt - Pattern 9 |
| Methanol | Clear solution | Cloudy suspension | HCl salt - Pattern 9 | Unchanged |
| Acetonitrile | Cloudy suspension | Cloudy suspension | HCl salt - Pattern 9 | Unchanged |
| T-BuOH | Cloudy suspension | Cloudy suspension | HCl salt - Pattern 6 | Unchanged |
| T-BuOH:H₂O 1:1 | Clear solution | Thin suspension | Insufficient material | Insufficient material |
| Acetonitrile:H₂O 1:1 | Clear solution | Clear solution | N/A | HCl salt - Pattern 12 |
| DMF | Cloudy suspension | Cloudy suspension | N/A | HCl salt - Pattern 1 |
| DMAC | Clear solution | Cloudy suspension | N/A | HCl salt - Pattern 1 |
| NMP | Clear solution | Clear solution | N/A | Still a solution at end of study |
| MeOH:DCM 1:3 | Cloudy suspension | Cloudy suspension | N/A | HCl salt - Pattern 13 |
| MeOH:H₂O 9:1 | Clear solution | Clear solution | N/A | HCl salt - Pattern 1 |
| THF:H₂O 9:1 | Cloudy suspension | Cloudy suspension | N/A | HCl salt - Pattern 1 + 1 peak |
| Acetone:H₂O 9:1 | Cloudy suspension | Cloudy suspension | N/A | HCl salt - Pattern 1 |

It was found that all provided HCl patterns exhibited complex thermal behavior with the onset of endotherms at low temperatures. In three instances on drying, the isolated Compound **2** Pattern 4 material was observed to change to Compound **2** Pattern 9.

¹H NMR analysis of solids obtained from the HCl experiments showed shifts consistent with salt formation in all but one sample. This sample was thought to be a novel freebase pattern. Of the remaining twelve novel patterns, Cl⁻ was shown to be present in all samples by ion chromatography. Thermal analysis of the samples showed all novel HCl patterns to exhibit complex thermal behavior with the onset of endotherms at low temperatures. In three instances on drying, the isolated HCl Salt - Pattern 4 material was observed to change to HCl salt - Pattern 9. The HCl salts isolated in the preliminary salt formation experiments were deemed unsuitable for further investigation due to the extensive polymorphism exhibited by the HCl salts, and the complex thermal behaviour of all isolated HCl salts. Based on the salt formation results and the observations made on introduction of the counter-ion, acetone, methanol and ethyl acetate were selected as the solvents for further salt experiments.

### 2. Salt Forms Experiment with Other Acids in Acetone, Methanol, and Ethyl Acetate

25 mg Compound 1 in 4 mL vials was suspended in 100 vol acetone, methanol, or ethyl acetate at 50 °C with 500 rpm magnetic stirring. 1 or 2 equivalents of each counter-ion was added (1 M stock solutions in THF, except nicotinic acid 0.25 M in H₂O and succinic acid 0.5 M in MeOH) and the samples were slowly cooled to 5 °C at 0.1 °C/min, and then held isothermally for 12 hours. All solids were then isolated by filtration and dried under suction. All solutions were uncapped and allowed to evaporate at ambient conditions. All isolated solids were analyzed by XRPD. In total, the salt experiments resulted in the isolation of 13 materials with a novel XRPD pattern (**Table 37**).

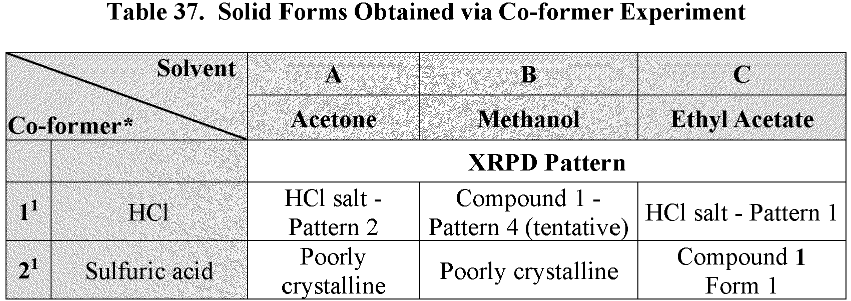

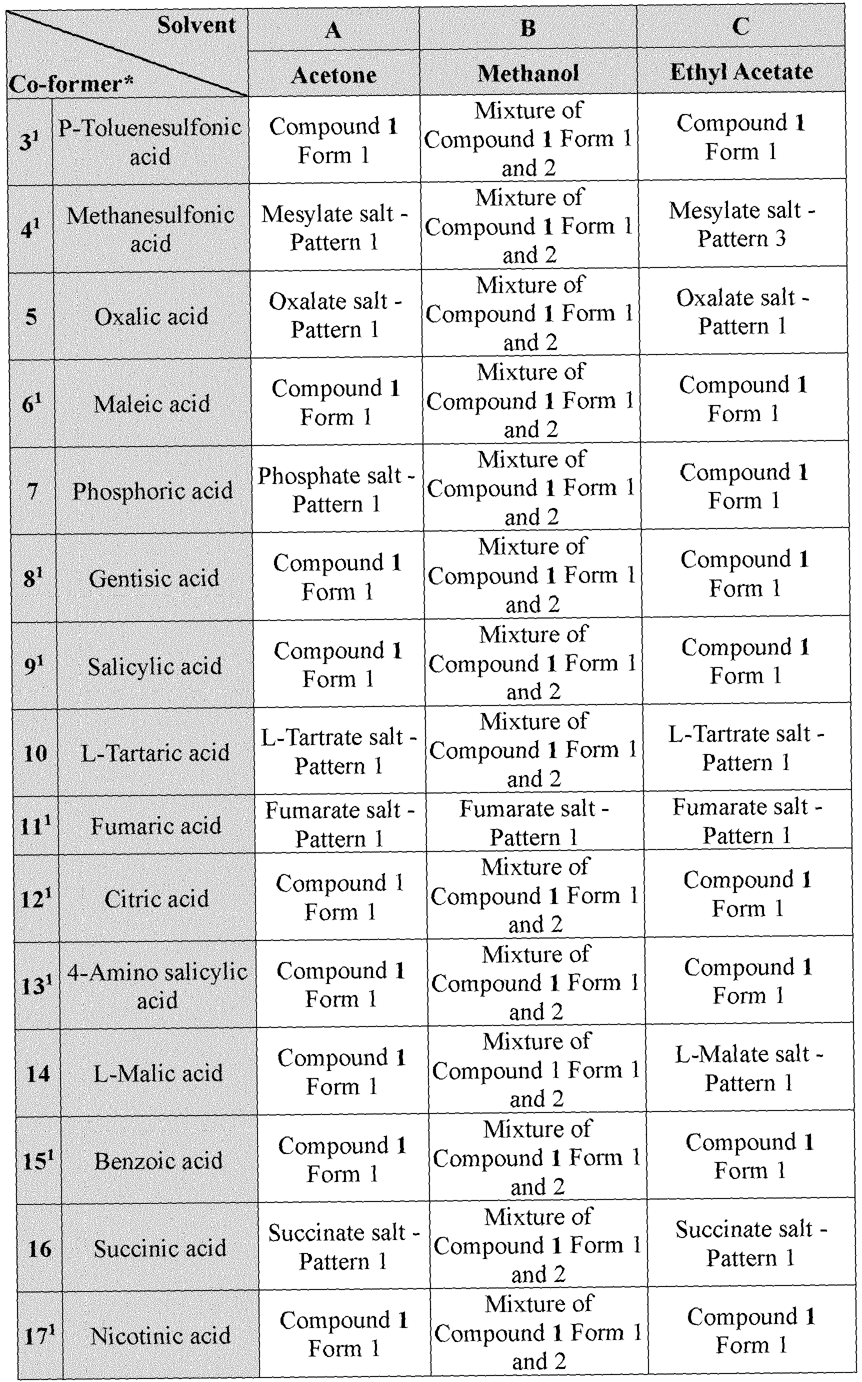

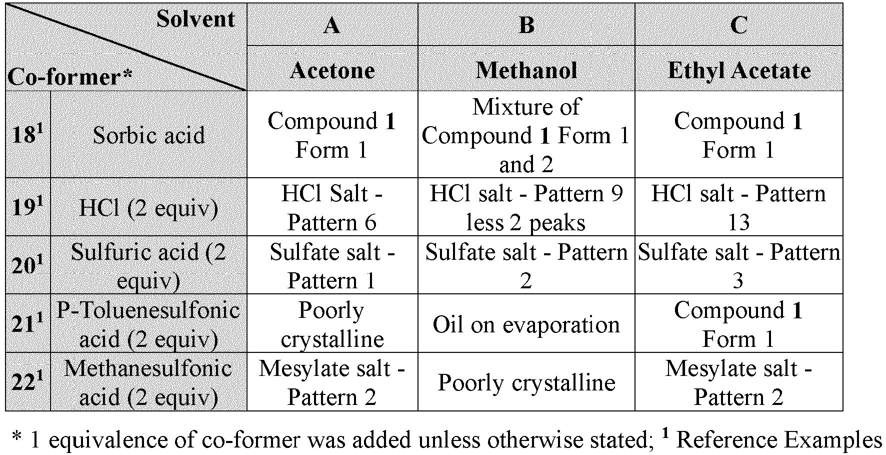

The salt experiment in which acetone was used as a solvent resulted in the isolation of ten salts, eight of which were new in this experiment (two HCl salts previously isolated in preliminary experiments). In ten of the twelve experiments in which a salt was not formed, Compound **1** Form 1 was isolated after cooling. In the remaining two instances, material with an XRPD pattern corresponding to poorly crystalline material was isolated.

The salt experiment in methanol resulted in the isolation of three salt patterns, one of which was new in this experiment (Sulfate salt - Pattern 2), as both the fumarate salt - Pattern 1 and the HCl salt - Pattern 9 were isolated from previous experiments. In the experiments in which salts were not formed, the isolation of poorly crystalline material, or a mixture of Compound **1** Form 1 and Compound **1** Form 2 resulted after cooling to 5 °C.

From the salt experiment performed in which ethyl acetate was used as the solvent, ten salts were isolated of which four were previously unobserved. The remaining six salts were identified in the previously performed experiments. In the remaining experiments from which salts were not isolated in the ethyl acetate experiment, Compound **1** Form 1 was shown by XRPD to be the sole material isolated.

In total, thirteen salt forms were identified from a total of sixty-six experiments. Of these thirteen salt forms, seven had properties deemed suitable for further characterization. Specifically, these seven salt forms are identified from the experiment with L-malic acid, succinic acid, phosphoric acid, oxalic acid, L-tartaric acid, methanesulfonic acid, and fumaric acid. The rest of the salt forms, e.g., sulfate salts, HCl salts, etc., exhibited complex thermal behavior typified by multiple endotherms at low temperature, or changed XRPD pattern after storage at conditions of 40 °C/75%RH for one week, and were deemed unsuitable for further scale-up and characterization.

### Example 4: Compound 3 (L-malate salt)

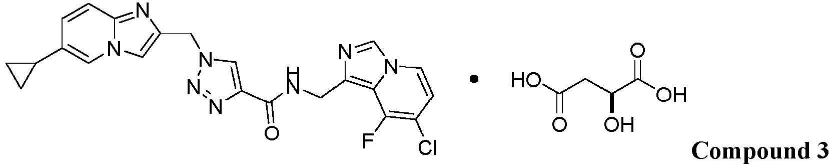

### Compound 3 Pattern 1

Compound **3** Pattern 1 was isolated from the experiment performed using ethyl acetate as the solvent. Compound **3** Form 1 was shown to contain 0.7 molar equivalents of L-malic acid by ¹H-NMR spectroscopy. Slight changes were observed in the XRPD pattern of the material after storage at conditions of 40 °C/75%RH for one week.

*Scale-up:* Compound **1** obtained from the synthetic procedure described in Example 1 was charged to an HEL polyblock 100 mL vial. Ethyl acetate (100 vol, 50 mL) was subsequently charged to the vial and the resulting suspension was heated to 50 °C with 500 rpm suspended magnetic stirring. To this, one equivalent L-malic was added (1 M in THF). The suspension was held isothermally for 16 hours with stirring, during which time, aliquots were removed and analyzed by XRPD. After 16 hours, conversion was not observed to have occurred. Suspension then cooled to 5 °C at 0.5 °C/min and held isothermally for 72 hours then a further aliquot was isolated and analyzed by XRPD. Conversion was observed to have occurred. The resulting solid was isolated by filtration and dried under suction. The material was isolated in 78.4 % yield with a purity of 98.2 %.

**Table 38. Characterization of the Scaled-up Compound 3 Pattern 1**

| | |
|---|---|
| **Description** | L-Malate, Compound **3** Pattern 1 |
| **Recovery** / **Yield** | 507.24 mg / 78.4 % |
| **XRPD** | Crystalline, consistent with material isolated from screen albeit absence of peaks corresponding to freebase. |
| **HPLC** | 98.2 % |
| **NMR** | Consistent with structure, 0.85 equivalents of L-malic acid. 0.02 equivalents of ethyl acetate. |
| **PLM** | Needles |
| **IDR Sector 1 average (pH 2.0) / (µg/min/cm²)** | 1791 *(additional peaks observed in XRPD pattern post analysis)* |
| **(*XRPD post analysis*)** | |

**Figure 7** provides the XRPD pattern of the Compound **3** Pattern 1.

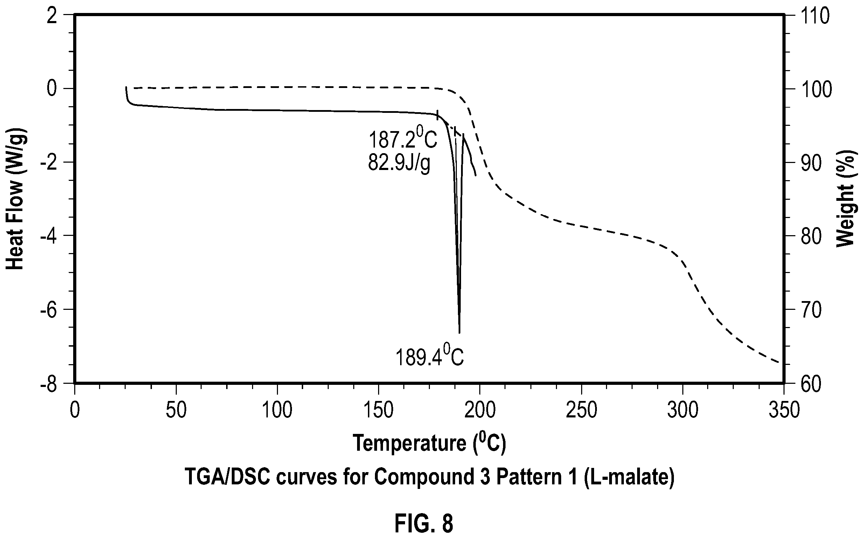

**Figure 8** provides the TGA/DSC curves of Compound **3** Pattern 1. The figure shows an endotherm (possibly melting/decomposition) at 187.2 °C (onset temperature) and a weight loss of 1.9% up to 150 °C.

TGA analysis of Compound **3** Pattern 1 shows the start of thermal decomposition to occur at approximately 190 °C. The DSC trace of the scaled-up material contained a single endotherm with an onset at 187.2 °C (82.9 J/g). GVS analysis of the material showed it to be slightly hygroscopic with a 0.22 % mass increase observed between 0 and 90 %RH, no observed change to the material was evidenced by XRPD post GVS analysis. The material was shown to be unchanged by XRPD after storage for one week at conditions of 40 °C/75%RH and 25 °C/97%RH.

### Example 5: Compound 4 (succinate salt)

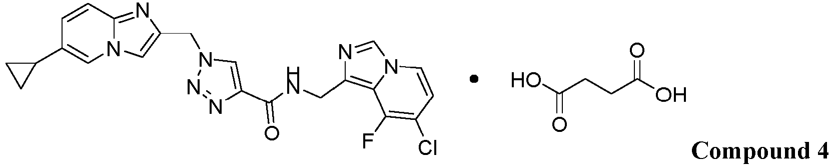

### Compound 4 Pattern 1

Compound **4** Pattern 1 was isolated from the experiments performed using acetone and ethyl acetate as solvents. Compound **4** Pattern 1 was shown to contain 0.75 molar equivalents of succinic acid by ¹H-NMR. Compound **4** Pattern 1 was unchanged as evidenced by XRPD after storage for one week at 40 °C/75%RH.

*Scale-up*: Compound **1** obtained from the synthetic procedure described in Example 1 was charged to an HEL polyblock 100 mL vial. Acetone (100 vol, 50 mL) was subsequently charged to the vial and the resulting suspension was heated to 50 °C with 500 rpm suspended magnetic stirring. To this, one equivalent succinic acid (1 M in MeOH) was added to give a suspension. The suspension was held isothermally for 16 hours with stirring, and aliquots were removed and analyzed by XRPD. After 16 hours, conversion was observed to have occurred. Suspensions were then cooled to 5 °C at 0.5 °C/min, held isothermally for 1 hour then isolated by filtration. The material was isolated in 80.5 % yield with a purity of 98.2 %.

**Table 39. Characterization of the Scaled-up Compound 4 Pattern 1**

| | |
|---|---|
| **Description** | Succinate, Compound **4** Pattern 1 |
| **Recovery / Yield** | 80.5 %, 502.9 mg |
| **XRPD** | Crystalline, consistent with material isolated from screen albeit absence of peaks corresponding to freebase. |
| **HPLC** | 98.2 % |
| **NMR** | Consistent with structure, 1.06 equivalents of succinic acid. 0.02 equivalents of acetone. |
| **PLM** | Fine needles |
| **IDR Sector 1 average (pH 2.0) / (µg/min/cm²)** | 255.5 *(additional peaks observed in XRPD pattern post analysis)* |
| ***(XRPD analysis post analysis)*** | |

**Figure 9** provides the XRPD pattern of the Compound **4** Pattern 1.

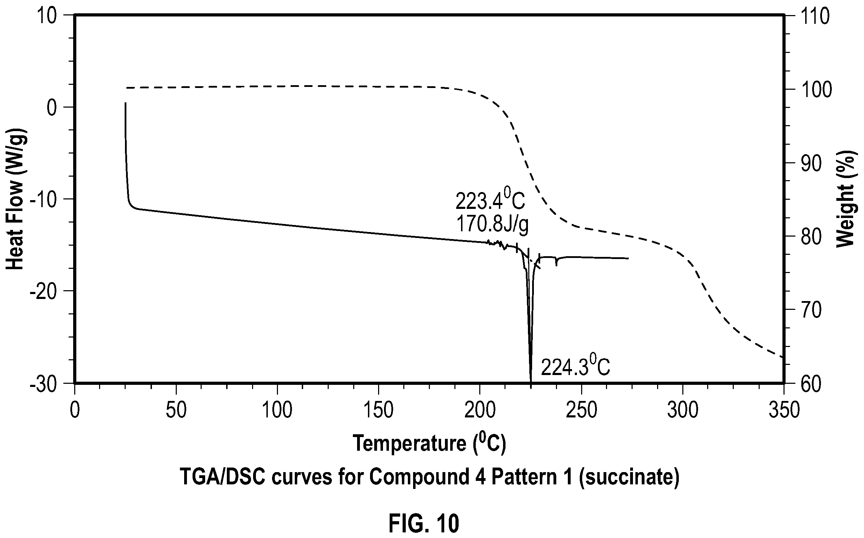

**Figure 10** provides the TGA/DSC curves of Compound **4** Pattern 1.

TGA analysis of the material shows the start of thermal decomposition to occur at approximately 200 °C. The DSC trace of the scaled-up material contained a single endotherm with an onset at 223.4 °C (171 J/g), whereas the DSC trace of material obtained from the screening experiments showed two thermal events. GVS analysis of the material showed it to be non-hygroscopic with a 0.16 % mass increase observed between 0 and 90 %RH, and no observed change to the material was evidenced by XRPD post GVS analysis. The material was shown to be unchanged by XRPD after storage for one week at conditions of 40 °C/75%RH and 25 °C/97%RH.

### Example 6: Compound 5 (phosphate salt)

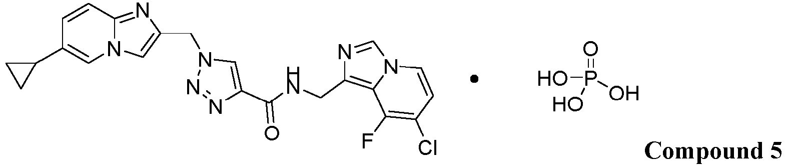

### Compound 5 Pattern 1

Compound **5** Pattern 1 was isolated from the screening experiment performed in acetone. This material was shown to contain one equivalent of phosphate by ion chromatography and was unchanged as evidenced by XRPD after storage for one week at conditions of 40 °C/75%RH. Compound **5** Pattern 1 was shown to have a small exotherm with onset at 90.3 °C, prior to a sharp endotherm with onset at 227.6 °C in its DSC thermogram.

**Table 40. Characterization of the Scaled-up Compound 5 Pattern 1**

| | |
|---|---|
| **Description** | Phosphate, Compound **5** Pattern 1 |
| **Recovery / Yield** | 571.09 mg / 94.3 % |
| **XRPD** | Crystalline, consistent with screen. |
| **IC** | 0.9 equivalents |
| **HPLC** | 98.4% |
| **NMR** | Consistent with structure. 0.03 equivalents of acetone. |
| **PLM** | Needles and fine particles |
| **IDR Sector 1 average (pH 2.0) / (µg/min/cm²)** | *404.1 (partial conversion of material to Compound **1** - Form 1 plus additional peaks as evidenced by XRPD post analysis)* |
| **(*XRPD analysis of the disc post analysis*)** | |

*Scale-up*: Compound **1** obtained from the synthetic procedure described in Example 1 was charged to an HEL polyblock 100 mL vessel. Acetone (100 vol, 50 mL) was charged to the vessel and the resulting suspension was heated to 50 °C with 500 rpm suspended magnetic stirring. One equivalent of phosphoric acid (1 M in THF) was added to the suspension. The suspension was held isothermally for 30 minutes with stirring then cooled to 5 °C at 0.1 °C/min. The suspension was then held isothermally for 14 hours and the resulting material isolated by filtration and dried under suction. The material was isolated in high yield (94.3 %) and purity (98.4 %).

**Figure 11** provides the XRPD pattern of Compound **5** Pattern 1.

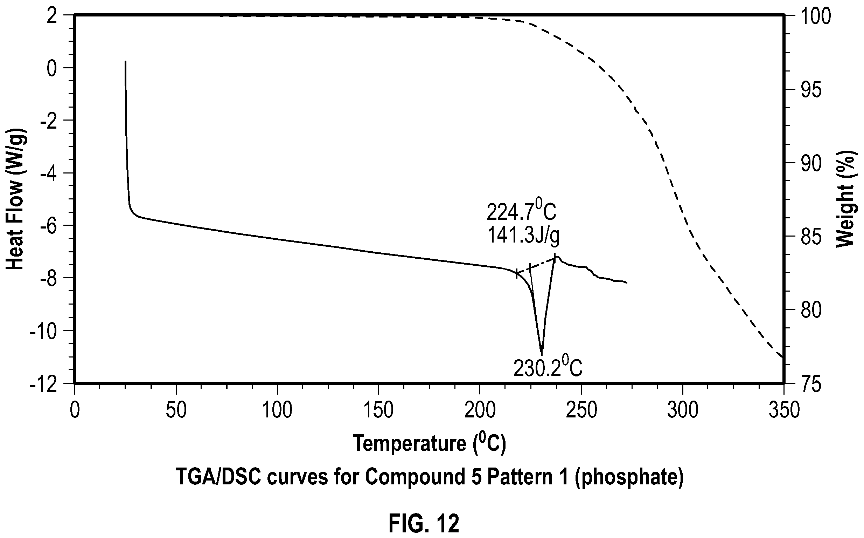

**Figure 12** provides the TGA/DSC curves of Compound **5** Pattern 1.

Thermal analysis of the material showed thermal decomposition of the material to occur at approximately 225 °C, and the DSC trace contained a single endotherm with an onset at 224.7 °C (141 J/g). GVS analysis of the material showed it to be slightly hygroscopic with a 1.2 % mass increase observed between 0 and 90 %RH, with no observed change to the material as evidenced by XRPD post analysis. The material was shown to be unchanged by XRPD after storage for one week at conditions of 40 °C/75%RH and 25 °C/97%RH.

### Example 7: Compound 6 (oxalate salt)

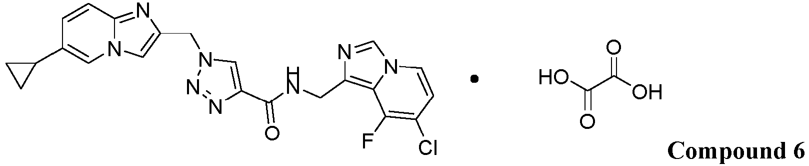

### Compound 6 Pattern 1

Compound **6** *Pattern* 1 was isolated from experiments in both acetone and ethyl acetate. This material was shown to contain one equivalent of oxalic acid by ion chromatography, and had a single melt in its DSC thermogram with onset at 212.8 °C. The XRPD pattern of the material after storage at conditions of 40 °C/75%RH for one week was slightly changed to that of the material isolated from screening.

*Scale-up*: Compound **1** obtained from the synthetic procedure described in Example 1 was charged to an HEL polyblock 100 mL vessel. Acetone (100 vol, 50 mL) was charged to the vessel and the resulting suspension was heated to 50 °C with 500 rpm suspended magnetic stirring. One equivalent of oxalic acid (1 M in THF) was added to the suspension. The suspension was held isothermally for 30 minutes with stirring then cooled to 5 °C at 0.1 °C/min. The suspension was then held isothermally for 14 hours and the resulting material isolated by filtration and dried under suction. The material was isolated in high yield (95 %) and purity (98.4 %).

**Table 41. Characterization of the Scaled-up Compound 6 Pattern 1**

| | |
|---|---|
| **Description** | Oxalate, Compound **6** Form 1 |
| **Recovery / Yield** | 567.42 mg / 95 % |
| **XRPD** | Crystalline, consistent with screen. |
| **IC** | 0.9 equivalents of oxalate |
| **HPLC** | 98.4 % |
| **NMR** | Consistent with structure. 0.02 equivalents of acetone. |
| **PLM** | Needles |
| **IDR Sector 1 average (pH 2.0) / (µg/min/cm²)** | 866.8 (*partial conversion of material to Compound **1** - Form 1 plus additional peaks as evidenced by XRPD post analysis)* |
| **(*XRPD analysis of disc post analysis*)** | |

**Figure 13** provides the XRPD pattern of the Compound **6** Pattern 1.

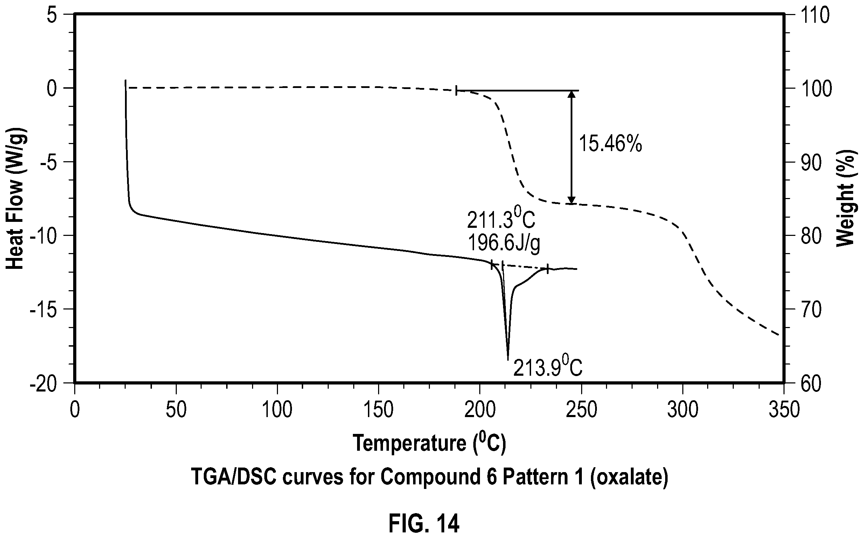

**Figure 14** provides the TGA/DSC curves of Compound **6** Pattern 1.

Thermal analysis of the material showed thermal decomposition of the material to occur at approximately 200°C, and the DSC trace contained a single endotherm with a shoulder (onset 211.3 °C, 197 J/g). GVS analysis of the material showed it to be slightly hygroscopic with a 0.6 % mass increase observed between 0 and 90 %RH, with no observed change to the material as evidenced by XRPD post analysis. The material was shown to be unchanged by XRPD after storage for one week at conditions of 40 °C/75%RH and 25 °C/97%RH.

### Further Experiments of New Forms of Compound 6

30 mg Compound **1** was charged to 7 mL vials. To these, 100 vol of selected solvents (55 vol used for NMP) was added, and the samples heated to 50 °C (60 °C for DMSO samples as complete dissolution not observed at 50 °C). All samples were stirred with 500 rpm magnetic stirring. Samples were stirred for 30 minutes, then 1 equivalent of the oxalic acid was added (1 M in THF). Samples were then stirred for three hours isothermally and then cooled to 5 °C at 0.1 °C/min (25 °C for DMSO samples). Samples were held isothermally for 16 hours then isolated by filtration. Samples with NMP as solvent were observed to be clear solutions. To these, 55 vol TBME was added, and the samples stirred for a further 30 minutes prior to being isolated by filtration. Solids were analyzed by XRPD, then dried in a vacuum oven overnight at 40 °C before a second analysis by XRPD. Experiment number = 11-20.

**Table 42. Results of the Polymorphism Assessment of the Oxalate Salt**

| Experiment # | Solvent | XRPD |
|---|---|---|
| 11 | NMP | Compound **1 -** Form 1 |
| 12 | DMSO | Poorly crystalline |
| 13 | MEK | Oxalate Salt - Pattern 1 |
| 14 | MIBK | Oxalate Salt - Pattern 1 |
| 15 | IPAC | Oxalate Salt - Pattern 1 + one additional peak at 5.5 °2θ consistent with highest intensity peak in Novel Pattern |
| 16 | TBME | Compound **1 -** Form 1 |
| 17 | THF | Compound **1 -** Form 1 |
| 18 | EtOH | Compound **1** - Form 1 |
| 19 | IPA | Novel Pattern - denoted |
| | | Oxalate Salt - Pattern 2 |
| 20 | Acetonitrile | Oxalate Salt - Pattern 1 |

### Compound 6 Pattern 4

As noted above, Compound **6** Pattern 2 was isolated from the screening experiments in IPA. About 10 mg of Compound **6** Pattern 2 was then heated to 175 °C at a rate of 10 °C in a TGA pan, held isothermally for 5 minutes, then cooled to ambient temperature to obtain Compound **6** Pattern 4. The TGA thermogram of the material, which showed no mass loss corresponding to desolvation demonstrated the material to be a solvent-free form. The TGA thermogram was seen to contain a mass loss between 200 and 260 °C corresponding to 0.84 molar equivalents of oxalic acid. The temperature of this mass loss coincides with an endotherm in the DSC trace of the material (onset 223.8 °C, 215 J/g).

*Scale-up*: Compound **1** obtained from the synthetic procedure described in Example 1 was charged to an HEL polyblock 140 mL vessel. To this, 100 vol (50 mL) IPA was added and the sample heated to 50 °C and held isothermally for 30 minutes with 500 rpm stirring using an HEL polyblock. One equivalent oxalic acid was added (1 M in THF), the sample was stirred for 3 hours, and then cooled to 5 °C at 0.1 °C/min. The sample was then stirred for 3 days, with aliquots removed intermittently and analysed by XRPD. After 3 days, the sample was adjudged to have fully converted and was therefore isolated by filtration and dried under vacuum. This sample was heated to 175 °C for 5 minutes using a Karl Fischer oven, then analysed by XRPD.

**Table 43. Characterization of the Scaled-up Compound 6 Pattern 4**

| | |
|---|---|
| **XRPD** | Crystalline. Consistent with Oxalate salt - Pattern 4 obtained at lower scale. |
| **NMR** | Consistent with structure. No trace IPA. |
| **IC** | 0.8 molar equivalents of oxalic acid. |
| **HPLC** | 98.2% |
| **PLM** | Needles |

**Figure 15** provides the XRPD pattern of the Compound **6** Pattern 4.

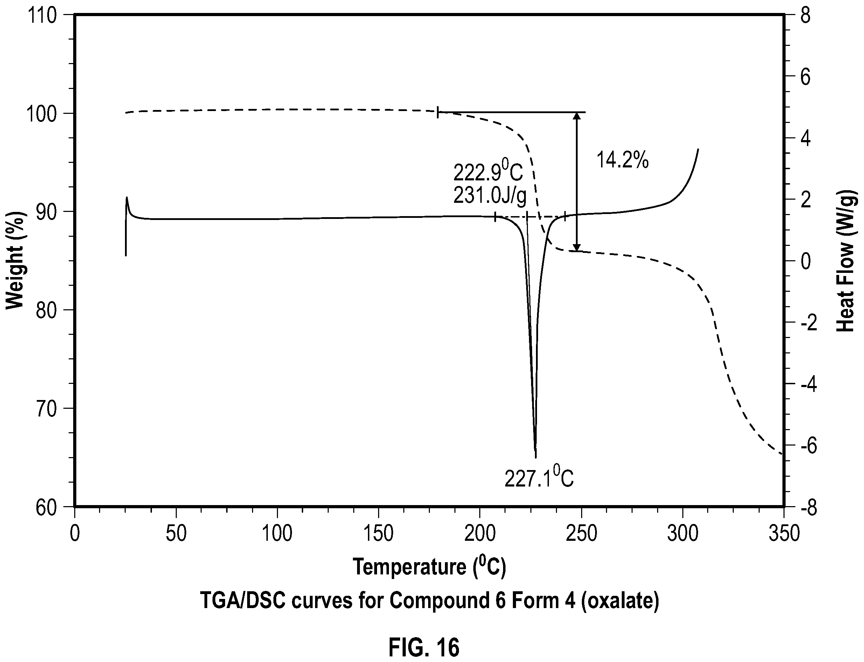

**Figure 16** provides the TGA/DSC curves of Compound **6** Pattern 4.

Compound **6** Pattern 4 was shown to be slightly hygroscopic by GVS with a 0.66 % mass loss observed between 0 and 90 %RH. Analysis of the material post GVS analysis showed it to have been unchanged as demonstrated by XRPD. The material was shown to be unchanged by XRPD after storage for one week at conditions of 40 °C/75 %RH and 25 °C/97%RH.

Alternatively, Compound **6** Pattern 4 can be prepared according to the following procedure: Compound **1** obtained from the synthetic procedure described in Example 1 (500 mg) was charged to a 100 mL round bottom flask. To this, 50 vol (25 mL) MeCN:H₂O 1:1 was added and the suspension heated to 50 °C. To this, two equivalents of oxalic acid (1 M in THF) were added and clarification of the suspension was observed. The solution was stirred isothermally for 30 minutes, then cooled to 5 °C at 0.1 °C/min. The resulting suspension was held isothermally for 12 hours, then isolated by filtration and dried under suction. The 523.19 mg material was recovered with a yield of 87.6%. The material was shown to have an XRPD pattern consistent with Oxalate Salt - Pattern 4. Ion chromatography showed the material to contain 1.0 equivalent of oxalic acid, demonstrating that although two equivalents of oxalic acid were used, the material is a monosalt.

### Competitive Slurry between Compound 6 Pattern 1 and Compound 6 Pattern 1

120 mg of both Compound **6** Pattern 1 and Compound **6** Pattern 4 were dispensed into a 20 mL vial. These were mixed, and then aliquots (20 mg) of this mixture was dispensed into HPLC vials and 90 vol selected solvents were added. Samples were slurried using a Polar Bear with 500 rpm magnetic stirring. Aliquots were removed after four days and analyzed by XRPD.

The results of the competitive slurries of Compound **6** Pattern 1 and Compound **6** Pattern 4 are shown in **Table 44.** In all but one experiment (Experiment 1), after four days of slurrying, the sole detectable phase by XRPD was Compound **6** Pattern 4. In the remaining experiment in which ethanol was used as a solvent and the material was slurried at 5 °C the XRPD pattern of the isolated material was shown to be a mixture of Compound **6** Pattern 4 and a novel pattern, hypothesized to be an ethanol solvate. Taken together, the competitive slurry data demonstrates that Compound **6** Pattern 4 is the more thermodynamically stable of the two solvent-free oxalate salts.

**Table 44. Results of Competitive Slurry of Compound 6 Pattern 4 and Compound 6 Pattern 1**

| Experiment # | Solvent | Temperature /°C | XRPD |
|---|---|---|---|
| 1 | Ethanol | 5 | Novel pattern, thought to be a mixture of Compound **6** Pattern 4 and ethanol solvate |
| 2 | Ethyl Acetate | 5 | Compound **6** Pattern 4 |
| 3 | Acetone | 5 | Compound **6** Pattern 4 |
| 4 | THF | 5 | Compound **6** Pattern 4 |
| 5 | Acetonitrile | 5 | Compound **6** Pattern 4 |
| 6 | Ethanol | 50 | Compound **6** Pattern 4 |
| 7 | Ethyl Acetate | 50 | Compound **6** Pattern 4 |
| 8 | Acetone | 50 | Compound **6** Pattern 4 |
| 9 | THF | 50 | Compound **6** Pattern 4 |
| 10 | Acetonitrile | 50 | Compound **6** Pattern 4 |

### Example 8: Compound 7 (L-tartrate salt)

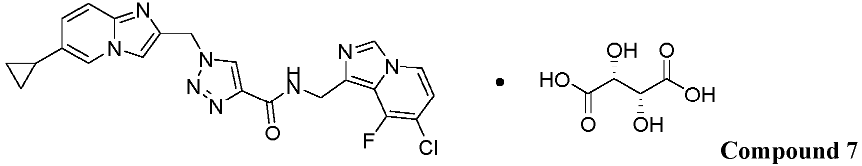

### Compound 7 Pattern 1

Compound 7 Form 1 was isolated from the experiments performed using acetone or ethyl acetate as solvent. The material was shown to contain 0.87 molar equivalents of L-tartaric acid by ¹H-NMR spectroscopy. The material was unchanged as evidenced by XRPD after storage for one week at 40 °C/75%RH.

*Scale-up*: Compound 1 obtained from the synthetic procedure described in Example 1 was charged to an HEL polyblock 100 mL vessel. Acetone (100 vol, 50 mL) was charged to the vessel and the resulting suspension was heated to 50 °C with 500 rpm suspended magnetic stirring. One equivalent of L-tartaric acid (1 M in THF) was added to the suspension. The suspension was held isothermally for 30 minutes with stirring then cooled to 5 °C at 0.1 °C/min. The suspension was then held isothermally for 14 hours and the resulting material isolated by filtration and dried under suction. The material was isolated in high yield (93.7 %) and purity (98.3 %).

**Table 45. Characterization of the Scaled-up Compound 7 Pattern 1**

| | |
|---|---|
| **Description** | L-Tartrate, Compound **7** Pattern 1 |
| **Recovery / Yield** | 619.89 mg / 93.7 % |
| **XRPD** | Consistent with screen. |
| **HPLC** | 98.3 % |
| **NMR** | Consistent with structure. 0.88 equivalents of L-tartaric acid. 0.04 equivalents of acetone. |
| **PLM** | Broken needles |
| **IDR Sector 1 average (pH 2.0) / (µg/min/cm²)** | 656.4 (*poorly crystalline post analysis*) |
| **(*XRPD analysis of the disc post analysis*)** | |

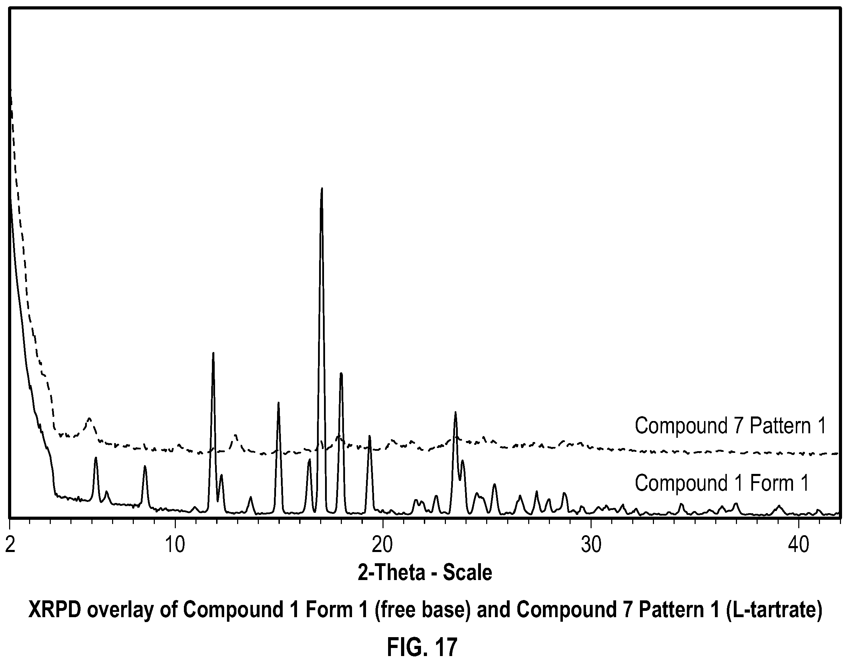

**Figure 17** provides XRPD overlay of Compound **1** Form 1 and Compound 7 Pattern 1.

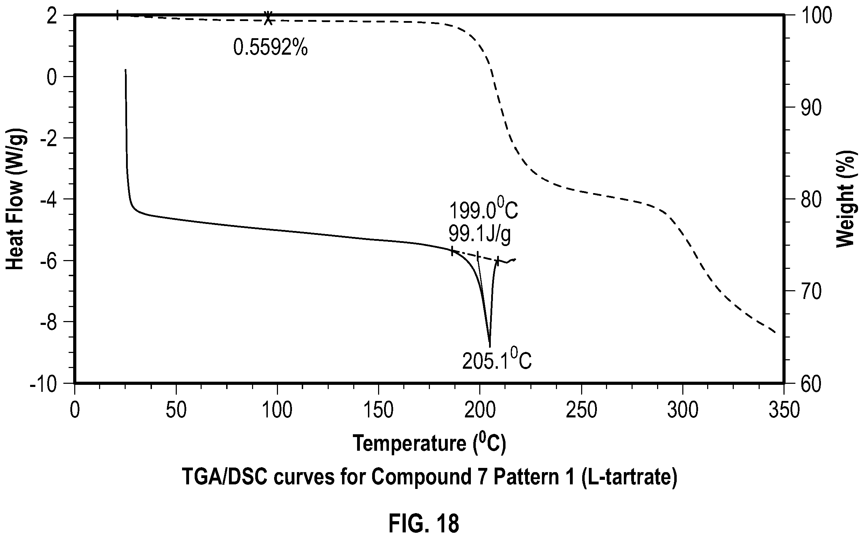

**Figure 18** provides the TGA/DSC curves of Compound 7 Pattern 1.

This XRPD pattern demonstrated Compound 7 Pattern 1 to be poorly crystalline. Analysis of the material showed a mass loss of 0.6 % between 25 °C thought to correspond to water, and the start of thermal decomposition at approximately 190 °C. The DSC trace contained a single endotherm with an onset at 199.0 °C (99 J/g). GVS analysis of the material showed it to be hygroscopic with a 3.4 % mass increase observed between 0 and 90 %RH with no hysteresis observed. This mass increase is consistent with 1.0 molar equivalents of water, which is indicative of possible hydrate formation. No observed change to the material was evidenced by XRPD post GVS analysis. The material was shown to be unchanged by XRPD after storage for one week at conditions of 40 °C/75%RH and 25 °C/97%RH.

### Reference Example 9: Compound 8 (mesylate salt)

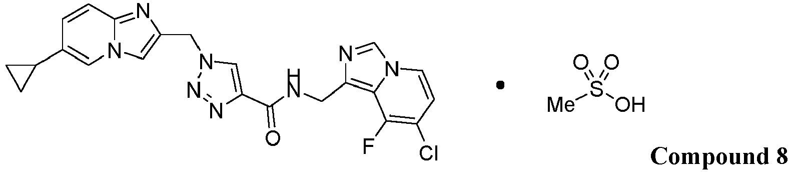

### Compound 8 Pattern 1, 2, and 3

In total three mesylate salts were isolated from the experiments performed. Mesylate salt - Pattern 1 was isolated from the acetone experiment after addition of one equivalent of methanesulfonic acid. This material was shown to contain 0.93 molar equivalents of methanesulfonic acid and contained two broad endotherms in its DSC trace. Storage of the material for one week at conditions of 40 °C/75%RH resulted in changes to its XRPD pattern with peaks corresponding to Compound **1** Form 1 being present. The material was therefore deemed unstable, and unsuitable for scale-up and further characterization.

Mesylate salt - Pattern 2 was isolated from the experiments in which two equivalents of methanesulfonic acid were added as the counter-ion and either acetone or ethyl acetate were used as the solvent. The material was shown to contain 1.76 molar equivalents of methanesulfonic acid by ¹H-NMR spectroscopy and was unchanged as evidenced by XRPD after one week of storage at conditions of 40 °C/75%RH. The thermal behavior of the material was shown to be complex by DSC, with multiple thermal events observed in the DSC trace. The material was therefore deemed unsuitable for scale-up and further characterization.

Mesylate salt - Pattern 3 was isolated from the screening experiment using one equivalent of methanesulfonic acid as the counter-ion and ethyl acetate as the solvent. The material was shown to contain 0.93 molar equivalents of methanesulfonic acid by ¹H-NMR spectroscopy, had a single endotherm in its DSC trace (onset 175.6 °C) and was unchanged as evidenced by XRPD after storage for one week at conditions of 40 °C/75%RH. Therefore, the material was deemed suitable for further scale-up and characterization.

### Reference Example 10: Compound 9 (Fumarate salt)

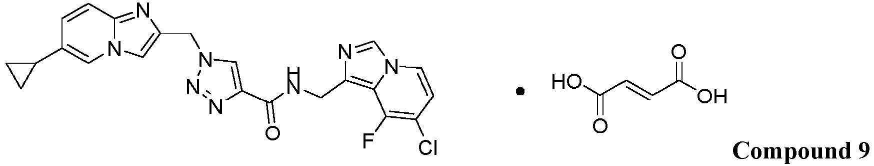

### Compound 9 Pattern 1

Compound **9** Pattern 1 was isolated from all three solvent-screens performed. This material was shown to contain 0.4 molar equivalents of fumaric acid by ¹H-NMR spectroscopy and was unchanged as evidenced by XRPD after storage for one week at 40 °C/75%RH.

*Scale-up*: Compound **1** obtained from the synthetic procedure described in Example 1 was charged to an HEL polyblock 100 mL vessel. Acetone (100 vol, 50 mL) was charged to the vessel and the resulting suspension was heated to 50 °C with 500 rpm suspended magnetic stirring. One equivalent of fumaric acid (0.5 M in THF:methanol 1:1) was added to the suspension. The suspension was held isothermally for 30 minutes with stirring then cooled to 5 °C at 0.1 °C/min. The suspension was then held isothermally for 14 hours and the resulting material isolated by filtration and dried under suction. The material was isolated in high yield (96.7 %) and purity (98.4 %).

**Table 46. Characterization of the Scaled-up Compound 9 Form 1**

| | |
|---|---|
| **Description** | Fumarate, Compound **9** Pattern 1 |
| **Recovery / Yield** | 604.04 mg / 96.7 % |
| **XRPD** | Crystalline, not consistent with screen. Peaks corresponding to freebase absent. |
| **HPLC** | 98.4 % |
| **NMR** | Consistent with structure 0.92 equivalents of fumaric acid. 0.08 equivalents of acetone. |
| **PLM** | Fine needles |
| **IDR Sector 1 average (pH 2.0) / (µg/min/cm²)** | 78.3 (*Additional peaks observed in XRPD pattern post analysis*) |
| **(*XRPD analysis of the disc post analysis*)** | |

**Figure 19** provides the XRPD pattern of the Compound **9** Pattern 1.

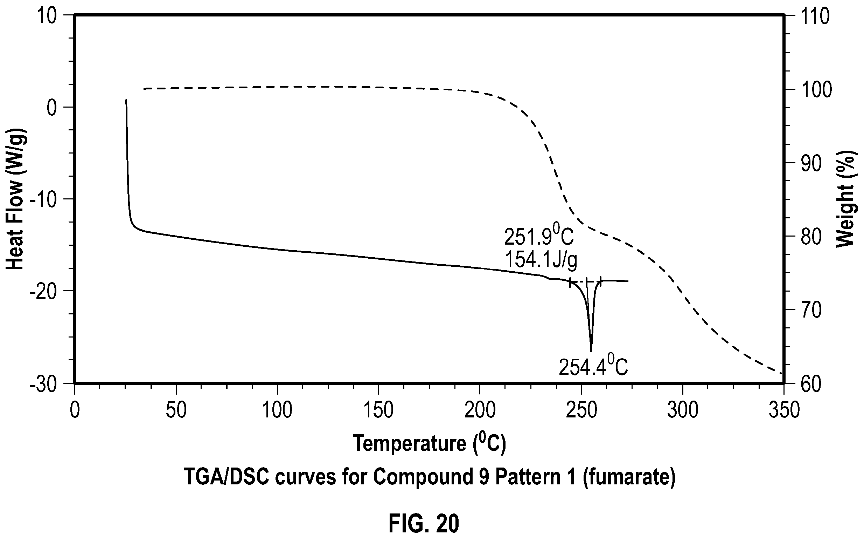

**Figure 20** provides the TGA/DSC curves of Compound **9** Pattern 1.

TGA analysis of the material showed the start of thermal decomposition to occur at approximately 210 °C. The DSC trace contained a single endotherm with an onset at 251.9 °C (154 J/g). The material isolated from the screening experiments was shown to contain 0.4 molar equivalents of fumaric acid by ¹H-NMR, and the sole endotherm in the DSC thermogram had a shoulder. The increase in fumaric acid equivalents as evidenced by ¹H-NMR, and the single peak in the DSC thermogram indicates the isolated material to be more phase pure than that obtained from screening. GVS analysis of the material showed it to be slightly hygroscopic with a 0.6 % mass increase observed between 0 and 90 %RH, and no observed change to the material was evidenced by XRPD post GVS analysis. The material was shown to be unchanged by XRPD after storage for one week at conditions of 40 °C/75%RH and 25 °C/97%RH.

### Example 11: Solid Form Solubility and Scalability

Seven of the eight solid forms selected for scale-up were successfully isolated from the experiments performed, with only the mesylate - Pattern 3 not obtained at 500 mg scale. Of the seven solid forms successfully scaled-up, only the L-tartrate was shown to be poorly crystalline and hygroscopic. The remaining solid forms, L-malate, succinate, phosphate, oxalate, and fumarate, were observed to be stable under storage, had single endotherms in their respective DSC traces and exhibited reduced hygroscopicity in GVS analysis. The tabulated IDR sector 1 average values of the obtained solid forms and the stable free base Compound **1** Form 1 are shown in **Table 47.** It is evident from the IDR data, that four of the stable solid forms isolated (oxalate, Compound **6** Pattern 1 and Pattern 4; phosphate, Compound **5** Pattern 1; succinate, Compound **4** Pattern 1; and L-malate, Compound **3** Pattern 1) exhibit increased sector 1 IDR compared to free base form Compound **1** Form 1. From these data it can be seen that the L-malate solid form (Compound **3** Form 1) has the highest IDR. However, no solid materials of L-malate, Pattern 1 could be isolated during solution-based scale-up experiments. In addition, further solution-based scale up experiments for succinate showed that external HCl is required for solution clarification (dissolution of all materials), making it a less attractive candidate for scale up production.

Compound **6** Pattern 4 is more thermodynamically stable than Compound **6** Pattern 1 in competitive slurry experiments. A scalable solution-based and operationally simple crystallization procedure for the isolation of phase pure Compound **6** Pattern 4 in high yield, purity and low residual solvent content was identified.

**Table 47. Sector 1 IDR Results of Scaled-up Solid Forms and Free Base Material of Compound 1**

| **Form** | **Average pH Sector 1** | **IDR (µg/min/cm²)** | | **XRPD Analysis Post Dissolution** | **Comments** |
|---|---|---|---|---|---|
| | | **Sector 1 pH 2.0** | **Sector 1 Average** | | |
| Compound 1 Form 1 | 2.00 | 104.3 | 104.8 | No change | |
| | | 105.2 | | | |
| Oxalate Pattern 1 | 1.99 | 837.7 | 866.8 | Partial conversion to Compound 1 Form 1 + additional peaks | Less thermodynamically stable than Oxalate Pattern 4 |
| | | 895.9 | | | |
| Oxalate Pattern 4 | 1.99 | 692.8 | 674.7 | Partial conversion to Compound 1 Form 1+ additional peaks | More thermodynamically stable than Oxalate Pattern 1. Scalable with high yield, purity, and low residual solvent |
| | | 656.6 | | | |
| Phosphate Pattern 1 | 1.99 | 364.7 | 404.1 | Partial conversion to Compound 1 Form 1 + additional peaks | Poorly crystalline |
| | | 443.4 | | | |
| L-Tartrate Pattern 1 | 1.99 | 647.0 | 656.4 | N/A | Poorly crystalline, hygroscopic |
| | | 665.7 | | | |
| Fumarate Pattern 1 | 1.99 | 76.8 | 78.3 | Additional peaks observed | |
| | | 79.8 | | | |
| Succinate Pattern 1 | 2.00 | 270.8 | 255.5 | Additional peaks observed | |
| | | 240.1 | | | |
| L-malate Pattern 1 | 2.01 | 1569 | 1791 | Additional peaks observed | |
| | | 2013 | | | |

### Reference Example 12: Compound 10 (Methyl Gallate Cocrystal)

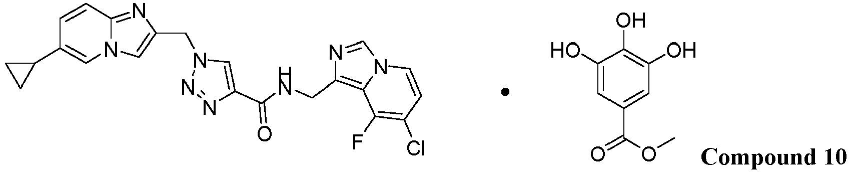

### Compound 10 Form 1

50 mg Compound **1** was dispensed into a 2 mL stainless-steel milling jar containing one stainless steel milling ball, and to this 1 molar equivalent of methyl gallate was charged. THF (35 µL) was charged to the vial, and was milled on a Retsch mill for 30 minutes at 30 Hz. An aliquot was isolated and analyzed by XRPD.

The DSC trace of the material showed a single endotherm at 190.7 °C, and the material remained unchanged by XRPD after storage of the material at conditions of 40 °C/75%RH for one week.

### Reference Example 13: Compound 11 (Propyl Gallate Cocrystal)

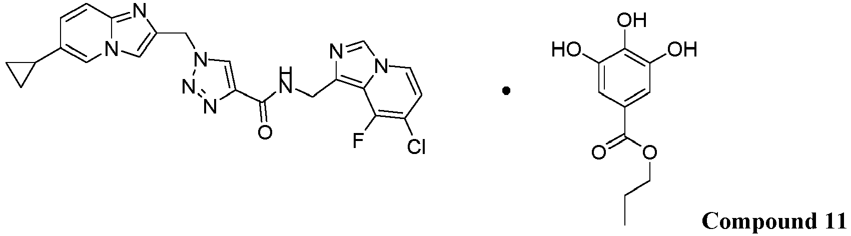

### Compound 11 Form 1

50 mg Compound **1** was dispensed into a 2 mL stainless-steel milling jar containing one stainless steel milling ball, to this 1 molar equivalent of propyl gallate was charged. THF (35 µl) was charged to the vial, and this was milled on a Retsch mill for 30 minutes at 30 Hz. An aliquot was isolated and analyzed by XRPD.

The DSC trace of the material contained a broad endotherm with onset at 148 °C with numerous shoulders typical of a material with complex thermal behavior, and a second endotherm with onset at 189 °C. The material remained unchanged by XRPD after storage of the material at conditions of 40 °C/75%RH for one week.

## Claims

1. A solid form of Compound **1:** 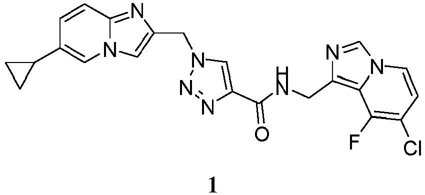 selected from the group consisting of a Compound 1 salt or co-crystal form of L-malic acid, oxalic acid, phosphoric acid, succinic acid, and L-tartaric acid.

2. The solid form of Compound **1** according to claim 1, selected from the group consisting of a Compound 1 salt form of L-malic acid, oxalic acid, phosphoric acid, succinic acid, and L-tartaric acid.

3. The solid form according to claim 1 wherein the solid form is a solid form of Compound **3** comprising Compound **1** and L-malic acid: 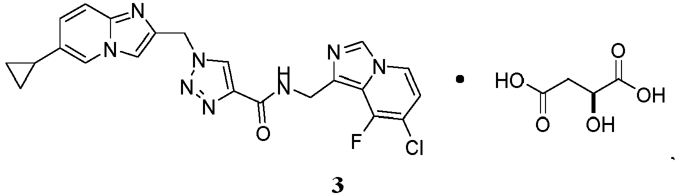 where
(i) the solid form is Compound **3** Pattern 1 and is **characterized by** any one of a) to g):
a) an X-ray powder diffraction (XRPD) pattern comprising three or more diffractions at angles (2 theta ± 0.2) of 6.2, 12.7, 17.3, 20.4, 25.3, and 26.4;
b) an X-ray powder diffraction (XRPD) pattern comprising four or more diffractions at angles (2 theta ± 0.2) of 6.2, 12.7, 17.3, 20.4, 25.3, and 26.4;
c) an X-ray powder diffraction (XRPD) pattern comprising five or more diffractions at angles (2 theta ± 0.2) of 6.2, 12.7, 17.3, 20.4, 25.3, and 26.4;
d) an X-ray powder diffraction (XRPD) pattern comprising diffractions at angles (2 theta ± 0.2) of 6.2, 12.7, 17.3, 20.4, 25.3, and 26.4;
e) an X-ray powder diffraction (XRPD) pattern comprising diffractions at angles (2 theta ± 0.2) of 6.2, 12.7, 17.3, 20.4, 25.3, and 26.4, corresponding to d-spacing (angstroms ± 0.2) of 14.32, 6.97, 5.13, 4.34, 3.52, and 3.37 (respectively);
f) an X-ray powder diffraction (XRPD) pattern comprising diffractions at angles (2 theta ± 0.2) of:
| **Angle / ° 2θ** | **Angle / ° 2θ** | **Angle /° 2θ** |
|---|---|---|
| 6.2 | 18.0 | 25.3 |
| 10.1 | 18.5 | 26.1 |
| 10.9 | 19.3 | 26.4 |
| 11.3 | 20.0 | 26.7 |
| 12.3 | 20.4 | 27.4 |
| 12.7 | 20.8 | 27.9 |
| 13.9 | 21.2 | 28.6 |
| 15.0 | 22.4 | 29.7 |
| 16.5 | 23.5 | 30.2 |
| 17.3 | 23.7 | 30.9 |
g) an X-ray powder diffraction (XRPD) pattern comprising diffractions at angles (2 theta ± 0.2) corresponding to d-spacing (angstroms ± 0.2) of:
| **Angle /° 2θ** | **d- spacing / Angstrom** | **Angle / ° 2θ** | **d- spacing / Angstrom** |
|---|---|---|---|
| 6.2 | 14.32 | 20.8 | 4.26 |
| 10.1 | 8.74 | 21.2 | 4.19 |
| 10.9 | 8.09 | 22.4 | 3.96 |
| 11.3 | 7.82 | 23.5 | 3.79 |
| 12.3 | 7.22 | 23.7 | 3.76 |
| 12.7 | 6.97 | 25.3 | 3.52 |
| 13.9 | 6.37 | 26.1 | 3.41 |
| 15.0 | 5.90 | 26.4 | 3.37 |
| 16.5 | 5.37 | 26.7 | 3.33 |
| 17.3 | 5.13 | 27.4 | 3.25 |
| 18.0 | 4.91 | 27.9 | 3.19 |
| 18.5 | 4.80 | 28.6 | 3.12 |
| 19.3 | 4.60 | 29.7 | 3.00 |
| 20.0 | 4.44 | 30.2 | 2.96 |
| 20.4 | 4.34 | 30.9 | 2.89 |
or
(ii) the solid form of Compound **3** or the solid form of Compound **3** Pattern 1 **characterized by** any one of (i) a) to g) is **characterized by** any one of h) or i):
h) a differential scanning calorimetry (DSC) endotherm having a minima at about 187.2 °C;
i) an about 0.22% mass increase between 0 and 90%RH by gravimetric vapor sorption (GVS) analysis.

4. The solid form according to claim 1 wherein the solid form is a solid form of Compound **4,** comprising Compound **1** and succinic acid: 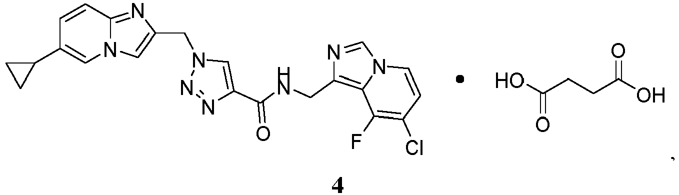 where
(i) the solid form is Compound **4** Pattern 1 and is **characterized by** any one of a) to g):
a) an X-ray powder diffraction (XRPD) pattern comprising three or more diffractions at angles (2 theta ± 0.2) of 6.2, 10.2, 12.7, 17.3, 20.4, and 23.8;
b) an X-ray powder diffraction (XRPD) pattern comprising four or more diffractions at angles (2 theta ± 0.2) of 6.2, 10.2, 12.7, 17.3, 20.4, and 23.8;
c) an X-ray powder diffraction (XRPD) pattern comprising five or more diffractions at angles (2 theta ± 0.2) of 6.2, 10.2, 12.7, 17.3, 20.4, and 23.8;
d) an X-ray powder diffraction (XRPD) pattern comprising diffractions at angles (2 theta ± 0.2) of 6.2, 10.2, 12.7, 17.3, 20.4, and 23.8;
e) an X-ray powder diffraction (XRPD) pattern comprising diffractions at angles (2 theta ± 0.2) of 6.2, 10.2, 12.7, 17.3, 20.4, and 23.8, corresponding to d-spacing (angstroms ± 0.2) of 14.26, 8.64, 6.95, 5.12, 4.35, and 3.73 (respectively);
f) an X-ray powder diffraction (XRPD) pattern comprising diffractions at angles (2 theta ± 0.2) of:
| **Angle / ° 2θ** | **Angle /° 2θ** | **Angle /° 2θ** |
|---|---|---|
| 6.2 | 18.5 | 26.5 |
| 7.9 | 19.3 | 26.8 |
| 10.2 | 20.4 | 27.4 |
| 10.9 | 20.9 | 27.9 |
| 11.2 | 21.8 | 28.4 |
| 11.5 | 23.0 | 28.7 |
| 12.7 | 23.8 | 29.8 |
| 16.5 | 24.6 | 31.0 |
| 17.3 | 25.4 | |
| 18.1 | 26.2 | |
g) an X-ray powder diffraction (XRPD) pattern comprising diffractions at angles (2 theta ± 0.2) corresponding to d-spacing (angstroms ± 0.2) of:
| **Angle / ° 20** | **d- spacing / Angstrom** | **Angle / ° 20** | **d- spacing / Angstrom** |
|---|---|---|---|
| 6.2 | 14.26 | 21.8 | 4.08 |
| 7.9 | 11.19 | 23.0 | 3.86 |
| 10.2 | 8.64 | 23.8 | 3.73 |
| 10.9 | 8.11 | 24.6 | 3.62 |
| 11.2 | 7.86 | 25.4 | 3.51 |
| 11.5 | 7.69 | 26.2 | 3.40 |
| 12.7 | 6.95 | 26.5 | 3.36 |
| 16.5 | 5.37 | 26.8 | 3.32 |
| 17.3 | 5.12 | 27.4 | 3.25 |
| 18.1 | 4.89 | 27.9 | 3.19 |
| 18.5 | 4.80 | 28.4 | 3.14 |
| 19.3 | 4.59 | 28.7 | 3.10 |
| 20.4 | 4.35 | 29.8 | 3.00 |
| 20.9 | 4.24 | 31.0 | 2.89 |
or
(ii) the solid form of Compound **4** or the solid form of Compound **4** Pattern 1 **characterized by** any one of (i) a) to g) is **characterized by** any one of h) or i):
h) a differential scanning calorimetry (DSC) endotherm having a minima at about 223.4 °C;
i) an about 0.16% mass increase between 0 and 90%RH by gravimetric vapor sorption (GVS) analysis.

5. The solid form according to claim 1 wherein in the solid form is a solid form of Compound **5,** comprising Compound **1** and phosphoric acid: 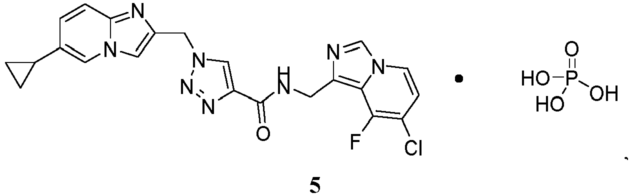 where
(i) the solid form is Compound **5** Pattern 1 and is **characterized by** any one of a) to g):
a) an X-ray powder diffraction (XRPD) pattern comprising three or more diffractions at angles (2 theta ± 0.2) of 4.7, 6.1, 17.0, 17.4, 18.1, and 24.6;
b) an X-ray powder diffraction (XRPD) pattern comprising four or more diffractions at angles (2 theta ± 0.2) of 4.7, 6.1, 17.0, 17.4, 18.1, and 24.6;
c) an X-ray powder diffraction (XRPD) pattern comprising five or more diffractions at angles (2 theta ± 0.2) of 4.7, 6.1, 17.0, 17.4, 18.1, and 24.6;
d) an X-ray powder diffraction (XRPD) pattern comprising diffractions at angles (2 theta ± 0.2) of 4.7, 6.1, 17.0, 17.4, 18.1, and 24.6;
e) an X-ray powder diffraction (XRPD) pattern comprising diffractions at angles (2 theta ± 0.2) of 4.7, 6.1, 17.0, 17.4, 18.1, and 24.6, corresponding to d-spacing (angstroms ± 0.2) of 18.68, 14.45, 5.20, 5.10, 4.91, and 3.62 (respectively);
f) an X-ray powder diffraction (XRPD) pattern comprising diffractions at angles (2 theta ± 0.2) of:
| **Angle / ° 2θ** | **Angle / ° 2θ** |
|---|---|
| 4.7 | 19.4 |
| 6.1 | 21.4 |
| 12.1 | 22.8 |
| 13.6 | 23.6 |
| 14.2 | 24.6 |
| 15.0 | 25.3 |
| 16.4 | 27.4 |
| 17.0 | 28.7 |
| 17.4 | 30.9 |
| 18.1 | 31.6 |
g) an X-ray powder diffraction (XRPD) pattern comprising diffractions at angles (2 theta ± 0.2) corresponding to d-spacing (angstroms ± 0.2) of:
| **Angle / ° 2θ** | **d- spacing / Angstrom** | **Angle / ° 2θ** | **d- spacing / Angstrom** |
|---|---|---|---|
| 4.7 | 18.68 | 19.4 | 4.58 |
| 6.1 | 14.45 | 21.4 | 4.15 |
| 12.1 | 7.33 | 22.8 | 3.89 |
| 13.6 | 6.51 | 23.6 | 3.77 |
| 14.2 | 6.24 | 24.6 | 3.62 |
| 15.0 | 5.91 | 25.3 | 3.51 |
| 16.4 | 5.40 | 27.4 | 3.25 |
| 17.0 | 5.20 | 28.7 | 3.11 |
| 17.4 | 5.10 | 30.9 | 2.89 |
| 18.1 | 4.91 | 31.6 | 2.83 |
or
(ii) the solid form of Compound **5** or the solid form of Compound **5** Pattern 1 **characterized by** any one of (i) a) to g) is **characterized by** any one of h) or i):
h) a differential scanning calorimetry (DSC) endotherm having a minima at about 224.7 °C; or
i) an about 1.2% mass increase between 0 and 90%RH by gravimetric vapor sorption (GVS) analysis.

6. The solid form according to claim 1 wherein the solid form is a solid form of Compound **6,** comprising Compound **1** and oxalic acid: 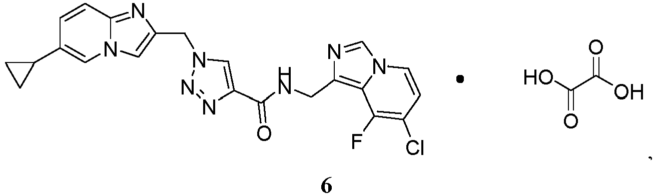 where:
(i) the solid form is Compound **6** Pattern 1 and is **characterized by** any one of a) to g):
a) an X-ray powder diffraction (XRPD) pattern comprising three or more diffractions at angles (2 theta ± 0.2) of 4.7, 6.1, 18.7, 24.0, 24.2, and 24.6;
b) an X-ray powder diffraction (XRPD) pattern comprising four or more diffractions at angles (2 theta ± 0.2) of 4.7, 6.1, 18.7, 24.0, 24.2, and 24.6;
c) an X-ray powder diffraction (XRPD) pattern comprising five or more diffractions at angles (2 theta ± 0.2) of 4.7, 6.1, 18.7, 24.0, 24.2, and 24.6;
d) an X-ray powder diffraction (XRPD) pattern comprising diffractions at angles (2 theta ± 0.2) of 4.7, 6.1, 18.7, 24.0, 24.2, and 24.6;
e) an X-ray powder diffraction (XRPD) pattern comprising diffractions at angles (2 theta ± 0.2) of 4.7, 6.1, 18.7, 24.0, 24.2, and 24.6, corresponding to d-spacing (angstroms ± 0.2) of 18.88, 14.36, 4.73, 3.71, 3.67, and 3.62 (respectively);
f) an X-ray powder diffraction (XRPD) pattern comprising diffractions at angles (2 theta ± 0.2) of:
| **Angle / ° 2θ** | **Angle / ° 2θ** | **Angle /° 2θ** |
|---|---|---|
| 4.7 | 15.9 | 24.0 |
| 6.1 | 16.4 | 24.2 |
| 7.1 | 16.7 | 24.6 |
| 8.4 | 17.0 | 25.3 |
| 9.3 | 17.3 | 26.1 |
| 11.9 | 18.7 | 27.3 |
| 13.6 | 19.7 | 27.9 |
| 14.0 | 20.3 | 28.7 |
| 14.9 | 21.7 | 29.8 |
| 15.4 | 22.0 | 30.9 |
g) an X-ray powder diffraction (XRPD) pattern comprising diffractions at angles (2 theta ± 0.2) corresponding to d-spacing (angstroms ± 0.2) of:
| **Angle / ° 2θ** | **d- spacing / Angstrom** | **Angle / ° 2θ** | **d- spacing / Angstrom** |
|---|---|---|---|
| 4.7 | 18.88 | 18.7 | 4.73 |
| 6.1 | 14.36 | 19.7 | 4.50 |
| 7.1 | 12.43 | 20.3 | 4.37 |
| 8.4 | 10.58 | 21.7 | 4.09 |
| 9.3 | 9.51 | 22.0 | 4.03 |
| 11.9 | 7.44 | 24.0 | 3.71 |
| 13.6 | 6.51 | 24.2 | 3.67 |
| 14.0 | 6.32 | 24.6 | 3.62 |
| 14.9 | 5.96 | 25.3 | 3.52 |
| 15.4 | 5.74 | 26.1 | 3.42 |
| 15.9 | 5.56 | 27.3 | 3.26 |
| 16.4 | 5.40 | 27.9 | 3.19 |
| 16.7 | 5.30 | 28.7 | 3.11 |
| 17.0 | 5.20 | 29.8 | 2.99 |
| 17.3 | 5.12 | 30.9 | 2.90 |
(ii) the solid form of Compound **6** or the solid form of Compound **6** Pattern 1 **characterized by** any one of (i) a) to g) is **characterized by** any one of h) or i):
h) a differential scanning calorimetry (DSC) endotherm having a minima at about 211.3 °C; or
i) an about 0.6% mass increase between 0 and 90%RH by gravimetric vapor sorption (GVS) analysis;
(iii) the solid form is Compound 6 Pattern 4 and is **characterized by** any one of a) to g):
a) an X-ray powder diffraction (XRPD) pattern comprising three or more diffractions at angles (2 theta ± 0.2) of 6.4, 11.9, 19.2, 26.1, 26.6, and 27.2;
b) an X-ray powder diffraction (XRPD) pattern comprising four or more diffractions at angles (2 theta ± 0.2) of 6.4, 11.9, 19.2, 26.1, 26.6, and 27.2;
c) an X-ray powder diffraction (XRPD) pattern comprising five or more diffractions at angles (2 theta ± 0.2) of 6.4, 11.9, 19.2, 26.1, 26.6, and 27.2;
d) an X-ray powder diffraction (XRPD) pattern comprising diffractions at angles (2 theta ± 0.2) of 6.4, 11.9, 19.2, 26.1, 26.6, and 27.2;
e) an X-ray powder diffraction (XRPD) pattern comprising diffractions at angles (2 theta ± 0.2) of 6.4, 11.9, 19.2, 26.1, 26.6, and 27.2, corresponding to d-spacing (angstroms ± 0.2) of 13.89, 7.43, 4.63, 3.42, 3.35, and 3.27 (respectively);
f) an X-ray powder diffraction (XRPD) pattern comprising diffractions at angles (2 theta ± 0.2) of:
| **Angle / ° 20** | **Angle / ° 2θ** | **Angle / ° 2θ** |
|---|---|---|
| 6.4 | 18.2 | 24.0 |
| 9.7 | 19.2 | 24.5 |
| 11.6 | 19.5 | 25.5 |
| 11.9 | 20.0 | 26.1 |
| 12.7 | 20.9 | 26.6 |
| 14.0 | 21.6 | 27.2 |
| 15.6 | 22.6 | 28.1 |
| 16.7 | 23.3 | 29.1 |
g) an X-ray powder diffraction (XRPD) pattern comprising diffractions at angles (2 theta ± 0.2) corresponding to d-spacing (angstroms ± 0.2) of:
| **Angle / ° 2θ** | **d- spacing / Angstrom** | **Angle / ° 2θ** | **d- spacing / Angstrom** |
|---|---|---|---|
| 6.4 | 13.89 | 20.9 | 4.25 |
| 9.7 | 9.08 | 21.6 | 4.10 |
| 11.6 | 7.65 | 22.6 | 3.93 |
| 11.9 | 7.43 | 23.3 | 3.81 |
| 12.7 | 6.97 | 24.0 | 3.71 |
| 14.0 | 6.31 | 24.5 | 3.62 |
| 15.6 | 5.67 | 25.5 | 3.49 |
| 16.7 | 5.31 | 26.1 | 3.42 |
| 18.1 | 4.87 | 26.6 | 3.35 |
| 19.2 | 4.63 | 27.2 | 3.27 |
| 19.5 | 4.55 | 28.1 | 3.18 |
| 20.0 | 4.45 | 29.2 | 3.06 |
or
(iv) the solid form of Compound **6** or the solid form of Compound **6** Pattern 4 **characterized by** any one of (iii) a) to g) is **characterized by** any one of h) or i):
h) a differential scanning calorimetry (DSC) endotherm having a minima at about 222.9 °C; or
i) an about 0.66% mass increase between 0 and 90%RH by gravimetric vapor sorption (GVS) analysis.

7. The solid form according to any one of claims 1-6, wherein the solid form of the compound has an acid:base ratio of about 1:1.

8. The solid form according to any one of claims 1-7, wherein said solid form is crystalline.

9. The solid form according to any one of claims 1-7, wherein said solid form is amorphous.

10. The solid form according to any one of claims 1-9, wherein said solid form is substantially free of impurities.

11. The solid form according to claim 1 wherein the solid form is a solid form of Compound **6:** 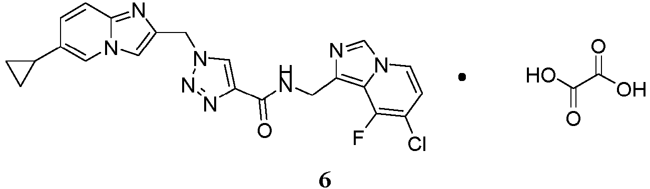 prepared by:
(i) a method comprising the steps of combining Compound **1** and oxalic acid in IPA, and then removing the solvent to provide the solid form of Compound **6,** wherein the solid form is prepared by:
a) a method comprising the steps of adding a solution of oxalic acid in THF to a solution of Compound **1** in IPA, and then removing the solvent to provide the solid form of Compound **6;** or
b) a method comprising the steps of:
i) adding a solution of oxalic acid in THF to a solution of Compound **1** in IPA at 50 °C,
ii) cooling the combined solution of step i) to 5 °C,
iii) stirring the cooled solution for a time sufficient to form the solid form of Compound **6**, and
iv) filtering the combined solution to remove the solvent;
or
(ii) a method comprising the steps of combining Compound **1** and oxalic acid in acetone, and then removing the solvent to provide the solid form of Compound **6,** wherein the solid form is prepared by:
a) a method comprising the steps of: adding a solution of oxalic acid in THF to a solution of Compound **1** in acetone, and then removing the solvent to provide the solid form of Compound **6;** or
b) a method comprising the steps of:
v) adding a solution of oxalic acid in THF to a solution of Compound 1 in acetone at 50 °C,
vi) cooling the combined solution of step i) to 5 °C,
vii) stirring the cooled solution for a time sufficient to form the solid form of Compound 6, and
viii) filtering the combined solution to remove the solvent.

12. A pharmaceutical composition comprising the solid form according to any one of claims 1-11 and a pharmaceutical acceptable carrier or excipient.

13. A method of preparing a solid form of Compound 6, 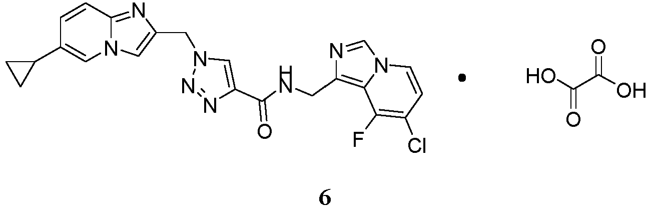 wherein the method comprises:
(i) the steps of combining Compound **1** and oxalic acid in IPA, and then removing the solvent to provide the solid form of Compound **6,** wherein the solid form is prepared by:
a) a method comprising the steps of adding a solution of oxalic acid in THF to a solution of Compound **1** in IPA, and then removing the solvent to provide the solid form of Compound **6;** or
b) a method comprising the steps of:
i) adding a solution of oxalic acid in THF to a solution of Compound 1 in IPA at 50 °C,
ii) cooling the combined solution of step i) to 5 °C,
iii) stirring the cooled solution for a time sufficient to form the solid form of Compound 6, and
iv) filtering the combined solution to remove the solvent;
or
(ii) the steps of combining Compound **1** and oxalic acid in acetone, and then removing the solvent to provide the solid form of Compound **6,** wherein the solid form is prepared by:
a) a method comprising the steps of: adding a solution of oxalic acid in THF to a solution of Compound **1** in acetone, and then removing the solvent to provide the solid form of Compound **6;** or
b) a method comprising the steps of:
v) adding a solution of oxalic acid in THF to a solution of Compound **1** in acetone at 50 °C
vi) cooling the combined solution of step i) to 5 °C,
vii) stirring the cooled solution for a time sufficient to form the solid form of Compound **6,** and
viii) filtering the combined solution to remove the solvent .

14. A solid form or composition of any one of claims 1-12 for use in a method of treating a plasma kallikrein-mediated disease or disorder.

15. A solid form or composition of any one of claims 1-12 for use in a method of treating hereditary angioedema or diabetic macular edema.

## Patentansprüche

1. Feste Form von Verbindung 1: 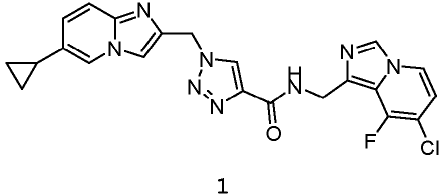 ausgewählt aus der Gruppe bestehend aus einem Verbindung-1-Salz oder der Co-Kristallform von L-Äpfelsäure, Oxalsäure, Phosphorsäure, Bernsteinsäure und L-Weinsäure.

2. Feste Form der Verbindung 1 nach Anspruch 1, ausgewählt aus der Gruppe bestehend aus einer Verbindung 1 Salzform von L-Äpfelsäure, Oxalsäure, Phosphorsäure, Bernsteinsäure und L-Weinsäure.

3. Feste Form nach Anspruch 1, wobei die feste Form eine feste Form von Verbindung 3 ist, die Verbindung 1 und L-Äpfelsäure umfasst: 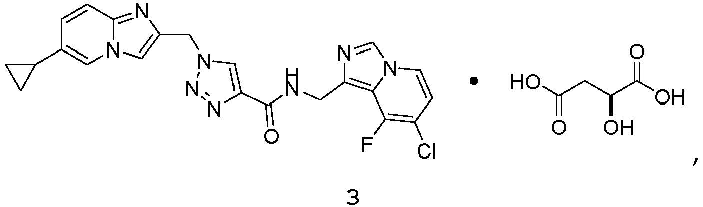 wobei
(i) die feste Form Verbindung 3 Muster 1 und **gekennzeichnet ist durch** eines von a) bis g):
a) ein Röntgen-Pulverdiffraktometriemuster (XRPD), umfassend drei oder mehr Diffraktionen in Winkeln (2 theta ± 0,2) von 6,2, 12,7, 17,3, 20,4, 25,3 und 26,4;
b) ein Röntgen-Pulverdiffraktometriemuster (XRPD), umfassend vier oder mehr Diffraktionen in Winkeln (2 theta ± 0,2) von 6,2, 12,7, 17,3, 20,4, 25,3 und 26,4;
c) ein Röntgen-Pulverdiffraktometriemuster (XRPD), umfassend fünf oder mehr Diffraktionen in Winkeln (2 theta ± 0,2) von 6,2, 12,7, 17,3, 20,4, 25,3 und 26,4;
d) ein Röntgen-Pulverdiffraktometriemuster (XRPD), umfassend Diffraktionen in Winkeln (2 theta ± 0,2) von 6,2, 12,7, 17,3, 20,4, 25,3 und 26,4;
e) ein Röntgen-Pulverdiffraktometriemuster (XRPD), umfassend Diffraktionen in Winkeln (2 theta ± 0,2) von 6,2, 12,7, 17,3, 20,4, 25,3 und 26,4, entsprechend einem (jeweiligen) d-Abstand (Angström ± 0,2) von 14,32, 6,97, 5,13, 4,34, 3,52 und 3,37;
f) ein Röntgen-Pulverdiffraktometriemuster (XRPD), umfassend Diffraktionen in Winkeln (2 theta ± 0,2) von:
| **Winkel / ° 2θ** | **Winkel / ° 2θ** | **Winkel / ° 2θ** |
|---|---|---|
| 6,2 | 18,0 | 25,3 |
| 10,1 | 18,5 | 26,1 |
| 10,9 | 19,3 | 26,4 |
| 11,3 | 20,0 | 26,7 |
| 12,3 | 20,4 | 27,4 |
| 12,7 | 20,8 | 27,9 |
| 13,9 | 21,2 | 28,6 |
| 15,0 | 22,4 | 29,7 |
| 16,5 | 23,5 | 30,2 |
| 17,3 | 23,7 | 30,9 |
g) ein Röntgen-Pulverdiffraktometriemuster (XRPD), umfassend Diffraktionen in Winkeln (2 theta ± 0,2) entsprechend einem d-Abstand (Angström ± 0,2) von:
| **Winkel / ° 2θ** | **d- Abstand / Angstrom** | **Winkel / ° 2θ** | **d- Abstand / Angstrom** |
|---|---|---|---|
| 6,2 | 14,32 | 20,8 | 4,26 |
| 10,1 | 8,74 | 21,2 | 4,19 |
| 10,9 | 8,09 | 22,4 | 3,96 |
| 11,3 | 7, 82 | 23,5 | 3,79 |
| 12,3 | 7,22 | 23,7 | 3,76 |
| **Winkel / ° 2θ** | **d- Abstand / Angstrom** | **Winkel / ° 2θ** | **d- Abstand / Angstrom** |
|---|---|---|---|
| 12,7 | 6, 97 | 25,3 | 3,52 |
| 13,9 | 6,37 | 26,1 | 3,41 |
| 15,0 | 5,90 | 26,4 | 3,37 |
| 16,5 | 5,37 | 26,7 | 3,33 |
| 17,3 | 5,13 | 27,4 | 3,25 |
| 18,0 | 4, 91 | 27,9 | 3,19 |
| 18,5 | 4,80 | 28,6 | 3,12 |
| 19,3 | 4,60 | 29,7 | 3,00 |
| 20,0 | 4,44 | 30,2 | 2,96 |
| 20,4 | 4,34 | 30,9 | 2,89 |
oder
(ii) die feste Form der Verbindung 3 oder die feste Form der Verbindung 3 Muster 1, **gekennzeichnet durch** eines von (i) a) bis g), durch eines von h) oder i) gekennzeichnet ist:
h) eine dynamische Differenzkalorimetrie (DSC) Endotherm mit einem Minimum bei etwa 187,2 °C;
i) eine etwa 0,22%ige Massenzunahme zwischen 0 und 90%RH durch gravimetrische Wasserdampfsorptionsanalyse (GVS).

4. Feste Form nach Anspruch 1, wobei die feste Form eine feste Form von Verbindung 4 ist, die Verbindung 1 und Bernsteinsäure umfasst: 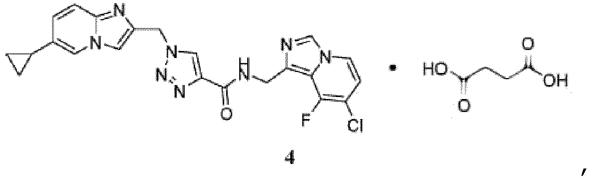 wobei
(i) die feste Form Verbindung 4 Muster 1 und **gekennzeichnet ist durch** eines von a) bis g):
a) ein Röntgen-Pulverdiffraktometriemuster (XRPD), umfassend drei oder mehr Diffraktionen in Winkeln (2 theta ± 0,2) von 6,2, 10,2, 12,7, 17,3, 20,4 und 23,8;
b) ein Röntgen-Pulverdiffraktometriemuster (XRPD), umfassend vier oder mehr Diffraktionen in Winkeln (2 theta ± 0,2) von 6,2, 10,2, 12,7, 17,3, 20,4 und 23,8;
c) ein Röntgen-Pulverdiffraktometriemuster (XRPD), umfassend fünf oder mehr Diffraktionen in Winkeln (2 theta ± 0,2) von 6,2, 10,2, 12,7, 17,3, 20,4 und 23,8;
d) ein Röntgen-Pulverdiffraktometriemuster (XRPD), umfassend Diffraktionen in Winkeln (2 theta ± 0,2) von 6,2, 10,2, 12,7, 17,3, 20,4 und 23,8;
e) ein Röntgen-Pulverdiffraktometriemuster (XRPD), umfassend Diffraktionen in Winkeln (2 theta ± 0,2) von 6,2, 10,2, 12,7, 17,3, 20,4 und 23,8, entsprechend einem (jeweiligen) d-Abstand (Angström ± 0,2) von 14,26, 8,64, 6,95, 5,12, 4,35 und 3,73;
f) ein Röntgen-Pulverdiffraktometriemuster (XRPD), umfassend Diffraktionen in Winkeln (2 theta ± 0,2) von:
| **Winkel / ° 2θ** | **Winkel / ° 2θ** | **Winkel / ° 2θ** |
|---|---|---|
| 6,2 | 18,5 | 26,5 |
| 7,9 | 19,3 | 26,8 |
| 10,2 | 20,4 | 27,4 |
| 10,9 | 20,9 | 27,9 |
| 11,2 | 21,8 | 28,4 |
| 11,5 | 23,0 | 28,7 |
| 12,7 | 23,8 | 29,8 |
| 16,5 | 24,6 | 31,0 |
| 17,3 | 25,4 | |
| 18,1 | 26,2 | |
g) ein Röntgen-Pulverdiffraktometriemuster (XRPD), umfassend Diffraktionen in Winkeln (2 theta ± 0,2) entsprechend einem d-Abstand (Angström ± 0,2) von:
| **Winkel / ° 2θ** | **d- Abstand / Angstrom** | **Winkel / ° 2θ** | **d- Abstand / Angstrom** |
|---|---|---|---|
| 6,2 | 14,26 | 21,8 | 4, 08 |
| 7,9 | 11,19 | 23,0 | 3,86 |
| 10,2 | 8,64 | 23,8 | 3,73 |
| 10,9 | 8,11 | 24,6 | 3,62 |
| 11,2 | 7,86 | 25,4 | 3,51 |
| 11,5 | 7,69 | 26,2 | 3,40 |
| 12,7 | 6,95 | 26,5 | 3,36 |
| 16,5 | 5,37 | 26,8 | 3,32 |
| 17,3 | 5,12 | 27,4 | 3,25 |
| 18,1 | 4, 89 | 27,9 | 3,19 |
| 18,5 | 4, 80 | 28,4 | 3,14 |
| 19,3 | 4,59 | 28,7 | 3,10 |
| 20,4 | 4,35 | 29,8 | 3,00 |
| 20,9 | 4,24 | 31,0 | 2,89 |
oder
(ii) die feste Form der Verbindung 4 oder die feste Form der Verbindung 4 Muster 1, **dadurch gekennzeichnet, dass** eines von (i) a) bis g) durch eines von h) oder i) gekennzeichnet ist:
h) eine dynamische Differenzkalorimetrie (DSC) Endotherme mit einem Minimum bei etwa 223,4 °C;
i) eine etwa 0,16%ige Massenzunahme zwischen 0 und 90%RH durch gravimetrische Wasserdampfsorptionsanalyse (GVS).

5. Feste Form nach Anspruch 1, wobei die feste Form eine feste Form von Verbindung 5 ist, die Verbindung 1 und Phosphorsäure umfasst: 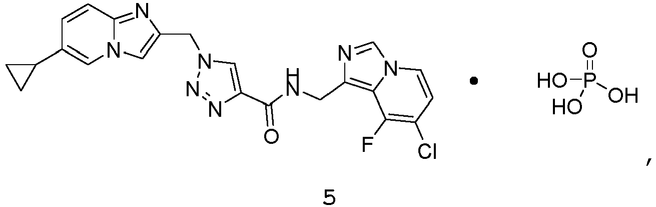 wobei
(i) die feste Form Verbindung 5 Muster 1 und **gekennzeichnet ist durch** eines von a) bis g):
a) ein Röntgen-Pulverdiffraktometriemuster (XRPD), umfassend drei oder mehr Diffraktionen in Winkeln (2 theta ± 0,2) von 4,7, 6,1, 17,0, 17,4, 18,1 und 24,6;
b) ein Röntgen-Pulverdiffraktometriemuster (XRPD), umfassend vier oder mehr Diffraktionen in Winkeln (2 theta ± 0,2) von 4,7, 6,1, 17,0, 17,4, 18,1 und 24,6;
c) ein Röntgen-Pulverdiffraktometriemuster (XRPD), umfassend fünf oder mehr Diffraktionen in Winkeln (2 theta ± 0,2) von 4,7, 6,1, 17,0, 17,4, 18,1 und 24,6;
d) ein Röntgen-Pulverdiffraktometriemuster (XRPD), umfassend Diffraktionen in Winkeln (2 theta ± 0,2) von 4,7, 6,1, 17,0, 17,4, 18,1 und 24,6;
e) ein Röntgen-Pulverdiffraktometriemuster (XRPD), umfassend Diffraktionen in Winkeln (2 theta ± 0,2) von 4,7, 6,1, 17,0, 17,4, 18,1 und 24,6, entsprechend einem (jeweiligen) d-Abstand (Angström ± 0,2) von 18,68, 14,45, 5,20, 5,10, 4,91 und 3,62;
f) ein Röntgen-Pulverdiffraktometriemuster (XRPD), umfassend Diffraktionen in Winkeln (2 theta ± 0,2) von:
| **Winkel / ° 2θ** | **Winkel / ° 2θ** |
|---|---|
| 4,7 | 19,4 |
| 6,1 | 21,4 |
| 12,1 | 22,8 |
| 13,6 | 23,6 |
| 14,2 | 24,6 |
| 15,0 | 25,3 |
| 16,4 | 27,4 |
| 17,0 | 28,7 |
| 17,4 | 30,9 |
| 18,1 | 31,6 |
g) ein Röntgen-Pulverdiffraktometriemuster (XRPD), umfassend Diffraktionen in Winkeln (2 theta ± 0,2) entsprechend einem d-Abstand (Angström ± 0,2) von:
| **Winkel / ° 2θ** | **d- Abstand / Angstrom** | **Winkel / ° 2θ** | **d- Abstand / Angstrom** |
|---|---|---|---|
| 4,7 | 18,68 | 19,4 | 4,58 |
| 6,1 | 14,45 | 21,4 | 4,15 |
| 12,1 | 7,33 | 22,8 | 3, 89 |
| 13,6 | 6,51 | 23,6 | 3,77 |
| 14,2 | 6,24 | 24,6 | 3, 62 |
| 15,0 | 5,91 | 25,3 | 3,51 |
| 16,4 | 5,40 | 27,4 | 3,25 |
| 17,0 | 5,20 | 28,7 | 3,11 |
| 17,4 | 5,10 | 30,9 | 2,89 |
| 18,1 | 4,91 | 31,6 | 2,83 |
oder
(ii) die feste Form der Verbindung 5 oder die feste Form der Verbindung 5 Muster 1, **dadurch gekennzeichnet, dass** eines von (i) a) bis g) durch eines von h) oder i) gekennzeichnet ist:
h) eine dynamische Differenzkalorimetrie (DSC) Endotherme mit einem Minimum bei etwa 224,7 °C; oder
i) eine etwa 1,2%ige Massenzunahme zwischen 0 und 90%RH durch gravimetrische Wasserdampfsorptionsanalyse (GVS).

6. Feste Form nach Anspruch 1, wobei die feste Form eine feste Form von Verbindung 6 ist, die Verbindung 1 und Oxalsäure umfasst: 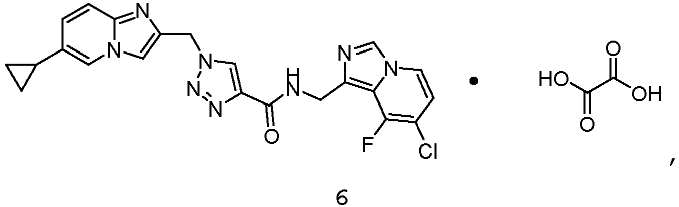 wobei
(i) die feste Form Verbindung 6 Muster 1 und **gekennzeichnet ist durch** eines von a) bis g):
a) ein Röntgen-Pulverdiffraktometriemuster (XRPD), umfassend drei oder mehr Diffraktionen in Winkeln (2 theta ± 0,2) von 4,7, 6,1, 18,7, 24,0 24,2 und 24,6;
b) ein Röntgen-Pulverdiffraktometriemuster (XRPD), umfassend vier oder mehr Diffraktionen in Winkeln (2 theta ± 0,2) von 4,7, 6,1, 18,7, 24,0 24,2 und 24,6;
c) ein Röntgen-Pulverdiffraktometriemuster (XRPD), umfassend fünf oder mehr Diffraktionen in Winkeln (2 theta ± 0,2) von 4,7, 6,1, 18,7, 24,0 24,2 und 24,6;
d) ein Röntgen-Pulverdiffraktometriemuster (XRPD), umfassend Diffraktionen in Winkeln (2 theta ± 0,2) von 4,7, 6,1, 18,7, 24,0 24,2 und 24,6;
e) ein Röntgen-Pulverdiffraktometriemuster (XRPD), umfassend Diffraktionen in Winkeln (2 theta ± 0,2) von 4,7, 6,1, 18,7, 24,0 24,2 und 24,6, entsprechend einem (jeweiligen) d-Abstand (Angström ± 0,2) von 18,88, 14,36, 4,73, 3,71, 3,67, und 3,62;
f) ein Röntgen-Pulverdiffraktometriemuster (XRPD), umfassend Diffraktionen in Winkeln (2 theta ± 0,2) von:
| **Winkel / ° 2θ** | **Winkel / ° 2θ** | **Winkel / ° 2θ** |
|---|---|---|
| 4,7 | 15,9 | 24,0 |
| 6,1 | 16,4 | 24,2 |
| 7,1 | 16,7 | 24,6 |
| 8,4 | 17,0 | 25,3 |
| 9,3 | 17,3 | 26,1 |
| 11,9 | 18,7 | 27,3 |
| 13,6 | 19,7 | 27,9 |
| 14,0 | 20,3 | 28,7 |
| 14,9 | 21,7 | 29,8 |
| 15,4 | 22,0 | 30,9 |
g) ein Röntgen-Pulverdiffraktometriemuster (XRPD), umfassend Diffraktionen in Winkeln (2 theta ± 0,2) entsprechend einem d-Abstand (Angström ± 0,2) von:
| **Winkel / ° 2θ** | **d- Abstand / Angstrom** | **Winkel / ° 2θ** | **d- Abstand / Angstrom** |
|---|---|---|---|
| 4,7 | 18,88 | 18,7 | 4,73 |
| 6,1 | 14,36 | 19,7 | 4,50 |
| 7,1 | 12,43 | 20,3 | 4,37 |
| 8,4 | 10,58 | 21,7 | 4,09 |
| 9,3 | 9,51 | 22,0 | 4,03 |
| 11,9 | 7,44 | 24,0 | 3,71 |
| 13,6 | 6,51 | 24,2 | 3,67 |
| 14,0 | 6,32 | 24,6 | 3,62 |
| 14,9 | 5,96 | 25,3 | 3,52 |
| 15,4 | 5,74 | 26,1 | 3,42 |
| 15,9 | 5,56 | 27,3 | 3,26 |
| 16,4 | 5,40 | 27,9 | 3,19 |
| 16,7 | 5,30 | 28,7 | 3,11 |
| 17,0 | 5,20 | 29,8 | 2,99 |
| 17,3 | 5,12 | 30,9 | 2,90 |
(ii) die feste Form der Verbindung 6 oder die feste Form der Verbindung 6 Muster 1, **dadurch gekennzeichnet, dass** eines von (i) a) bis g) durch eines von h) oder i) gekennzeichnet ist:
h) eine dynamische Differenzkalorimetrie (DSC) Endotherme mit einem Minimum bei etwa 211,3 °C; oder
i) eine etwa 0,6%ige Massenzunahme zwischen 0 und 90% RH durch gravimetrische Wasserdampfsorptionsanalyse (GVS);
(iii) die feste Form Verbindung 6 Muster 4 und **gekennzeichnet ist durch** eines von a) bis g):
a) ein Röntgen-Pulverdiffraktometriemuster (XRPD), umfassend drei oder mehr Diffraktionen in Winkeln (2 theta ± 0,2) von 6,4, 11,9, 19,2, 26,1, 26,6 und 27,2;
b) ein Röntgen-Pulverdiffraktometriemuster (XRPD), umfassend vier oder mehr Diffraktionen in Winkeln (2 theta ± 0,2) von 6,4, 11,9, 19,2, 26,1, 26,6 und 27,2;
c) ein Röntgen-Pulverdiffraktometriemuster (XRPD), umfassend fünf oder mehr Diffraktionen in Winkeln (2 theta ± 0,2) von 6,4, 11,9, 19,2, 26,1, 26,6 und 27,2;
d) ein Röntgen-Pulverdiffraktometriemuster (XRPD), umfassend Diffraktionen in Winkeln (2 theta ± 0,2) von 6,4, 11,9, 19,2, 26,1, 26,6 und 27,2;
e) ein Röntgen-Pulverdiffraktometriemuster (XRPD), umfassend Diffraktionen in Winkeln (2 theta ± 0,2) von 6,4, 11,9, 19,2, 26,1, 26,6 und 27,2, entsprechend einem (jeweiligen) d-Abstand (Angström ± 0,2) von 13,89, 7,43, 4,63, 3,42, 3,35, und 3,27;
f) ein Röntgen-Pulverdiffraktometriemuster (XRPD), umfassend Diffraktionen in Winkeln (2 theta ± 0,2) von:
| **Winkel / ° 2θ** | **Winkel / ° 2θ** | **Winkel / ° 2θ** |
|---|---|---|
| 6,4 | 18,2 | 24,0 |
| 9,7 | 19,2 | 24,5 |
| 11,6 | 19,5 | 25,5 |
| 11,9 | 20,0 | 26,1 |
| 12,7 | 20,9 | 26,6 |
| 14,0 | 21,6 | 27,2 |
| 15,6 | 22,6 | 28,1 |
| 16,7 | 23,3 | 29,1 |
g) ein Röntgen-Pulverdiffraktometriemuster (XRPD), umfassend Diffraktionen in Winkeln (2 theta ± 0,2) entsprechend einem d-Abstand (Angström ± 0,2) von:
| **Winkel / ° 2θ** | **d- Abstand / Angstrom** | **Winkel / ° 2θ** | **d- Abstand / Angstrom** |
|---|---|---|---|
| 6,4 | 13,89 | 20,9 | 4,25 |
| 9,7 | 9,08 | 21,6 | 4,10 |
| 11,6 | 7, 65 | 22,6 | 3,93 |
| 11,9 | 7,43 | 23,3 | 3,81 |
| 12,7 | 6, 97 | 24,0 | 3,71 |
| 14,0 | 6,31 | 24,5 | 3,62 |
| 15,6 | 5,67 | 25,5 | 3,49 |
| 16,7 | 5,31 | 26,1 | 3,42 |
| 18,1 | 4,87 | 26,6 | 3,35 |
| 19,2 | 4,63 | 27,2 | 3,27 |
| 19,5 | 4,55 | 28,1 | 3,18 |
| 20,0 | 4,45 | 29,2 | 3,06 |
oder
(iv) die feste Form der Verbindung 6 oder die feste Form der Verbindung 6 Muster 4, **dadurch gekennzeichnet, dass** eines von (iii) a) bis g) durch eines von h) oder i) gekennzeichnet ist:
h) eine dynamische Differenzkalorimetrie (DSC) Endotherme mit einem Minimum bei etwa 222,9 °C; oder
i) eine etwa 0,66%ige Massenzunahme zwischen 0 und 90%RH durch gravimetrische Wasserdampfsorptionsanalyse (GVS).

7. Feste Form nach einem der Ansprüche 1 bis 6, wobei die feste Form der Verbindung ein Säure:Basen-Verhältnis von etwa 1:1 aufweist.

8. Feste Form nach einem der Ansprüche 1 bis 7, wobei die feste Form kristallin ist.

9. Feste Form nach einem der Ansprüche 1 bis 7, wobei die feste Form amorph ist.

10. Feste Form nach einem der Ansprüche 1 bis 9, wobei die feste Form im Wesentlichen frei von Verunreinigungen ist.

11. Feste Form nach Anspruch 1, wobei die feste Form eine feste Form der Verbindung 6 ist: 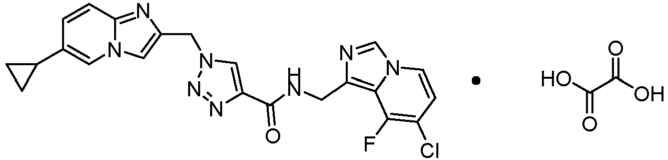 hergestellt durch:
(i) ein Verfahren, umfassend die Schritte des Kombinierens von Verbindung **1** und Oxalsäure in IPA und dann Entfernen des Lösungsmittels, um die feste Form der Verbindung **6** bereitzustellen, wobei die feste Form hergestellt wird durch:
a) ein Verfahren, umfassend die Schritte des Hinzufügens einer Lösung von Oxalsäure in THF zu einer Lösung von Verbindung 1 in IPA und des anschließenden Entfernens des Lösungsmittels, um die feste Form von Verbindung **6** bereitzustellen; oder
b) ein Verfahren, umfassend folgende Schritte:
i) Zugeben einer Lösung von Oxalsäure in THF zu einer Lösung von Verbindung **1** in IPA bei 50 °C,
ii) Abkühlen der kombinierten Lösung aus Schritt i) auf 5 °C,
iii) Rühren der gekühlten Lösung für eine Zeit, die ausreicht, um die feste Form der Verbindung **6** zu bilden, und
iv) Filtern der kombinierten Lösung, um das Lösungsmittel zu entfernen;
oder
(ii) ein Verfahren, umfassend die Schritte des Kombinierens von Verbindung **1** und Oxalsäure in Aceton und dann Entfernen des Lösungsmittels, um die feste Form der Verbindung **6** bereitzustellen, wobei die feste Form hergestellt wird durch:
a) ein Verfahren, umfassend die Schritte des Hinzufügens einer Lösung von Oxalsäure in THF zu einer Lösung von Verbindung 1 in Aceton, und anschließendes Entfernen des Lösungsmittels, um die feste Form von Verbindung **6** bereitzustellen; oder
b) ein Verfahren, umfassend folgende Schritte:
v) Zugeben einer Lösung von Oxalsäure in THF zu einer Lösung von Verbindung **1** in Aceton bei 50 °C,
vi) Abkühlen der kombinierten Lösung aus Schritt i) auf 5 °C,
vii) Rühren der gekühlten Lösung für eine Zeit, die ausreicht, um die feste Form der Verbindung **6**zu bilden, und
viii) Filtern der kombinierten Lösung, um das Lösungsmittel zu entfernen.

12. Pharmazeutische Zusammensetzung umfassend die feste Form nach einem der Ansprüche 1 bis 11 und einen pharmazeutisch verträglichen Träger oder Hilfsstoff.

13. Verfahren zur Herstellung einer festen Form der Verbindung 6, 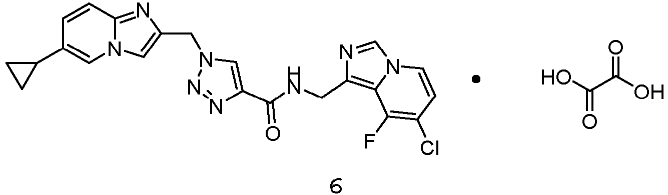 wobei das Verfahren umfasst:
(i) die Schritte des Kombinierens von Verbindung **1** und Oxalsäure in IPA und das anschließende Entfernen des Lösungsmittels, um die feste Form von Verbindung **6** zu bereitstellen, wobei die feste Form hergestellt wird durch:
a) ein Verfahren, umfassend die Schritte des Hinzufügens einer Lösung von Oxalsäure in THF zu einer Lösung von Verbindung **1** in IPA und des anschließenden Entfernens des Lösungsmittels, um die feste Form von Verbindung **6** bereitzustellen; oder
b) ein Verfahren, umfassend folgende Schritte:
i) Zugeben einer Lösung von Oxalsäure in THF zu einer Lösung von Verbindung **1** in IPA bei 50 °C,
ii) Abkühlen der kombinierten Lösung aus Schritt i) auf 5 °C,
iii) Rühren der gekühlten Lösung für eine Zeit, die ausreicht, um die feste Form der Verbindung **6**zu bilden, und
iv) Filtern der kombinierten Lösung, um das Lösungsmittel zu entfernen;
oder
(ii) die Schritte des Kombinierens der Verbindung **1** und Oxalsäure in Aceton und des anschließenden Entfernens des Lösungsmittels, um die feste Form der Verbindung **6**bereitzustellen, wobei die feste Form hergestellt wird durch:
a) ein Verfahren, umfassend die Schritte des Hinzufügens einer Lösung von Oxalsäure in THF zu einer Lösung von Verbindung **1** in Aceton, und anschließendes Entfernen des Lösungsmittels, um die feste Form von Verbindung **6**bereitzustellen; oder
b) ein Verfahren, umfassend folgende Schritte:
v) Zugeben einer Lösung von Oxalsäure in THF zu einer Lösung von Verbindung **1** in Aceton bei 50 °C
vi) Abkühlen der kombinierten Lösung aus Schritt i) auf 5 °C,
vii) Rühren der gekühlten Lösung für eine Zeit, die ausreicht, um die feste Form der Verbindung **6** zu bilden, und
viii) Filtern der kombinierten Lösung, um das Lösungsmittel zu entfernen.

14. Feste Form oder Zusammensetzung nach einem der Ansprüche 1 bis 12 zur Verwendung in einem Verfahren zur Behandlung einer durch Plasmakallikrein vermittelten Krankheit oder Störung.

15. Feste Form oder Zusammensetzung nach einem der Ansprüche 1 bis 12 zur Verwendung bei einem Verfahren zur Behandlung von vererblichen Angioödem oder diabetischem Makulaödem.

## Revendications

1. Forme solide du Composé 1 : 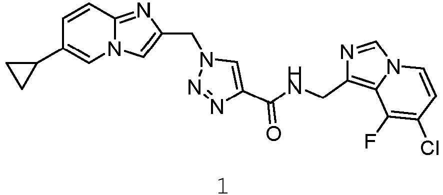 choisie dans le groupe constitué d'un sel du Composé 1 ou d'une forme co-cristalline de l'acide L-malique, l'acide oxalique, l'acide phosphorique, l'acide succinique et l'acide L-tartrique.

2. Forme solide de Composé 1 selon la revendication 1, choisie dans le groupe constitué d'une forme de sel du Composé 1 de l'acide L-malique, l'acide oxalique, l'acide phosphorique, l'acide succinique et l'acide L-tartrique.

3. Forme solide selon la revendication 1, dans laquelle la forme solide est une forme solide de Composé 3 comprenant le Composé 1 et l'acide L-malique : 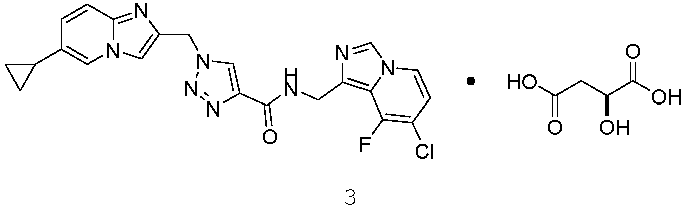 où
(i) la forme solide est le Composé 3 diagramme 1 et est **caractérisée par** l'un quelconque des points a) à g) :
a) un diagramme de diffraction des rayons X sur poudre (DRXP) comprenant trois diffractions ou plus à des angles (2 thêta ± 0,2) de 6,2, 12,7, 17,3, 20,4, 25,3 et 26,4 ;
b) un diagramme de diffraction des rayons X sur poudre (DRXP) comprenant quatre diffractions ou plus à des angles (2 thêta ± 0,2) de 6,2, 12,7, 17,3, 20,4, 25,3 et 26,4 **;**
c) un diagramme de diffraction des rayons X sur poudre (DRXP) comprenant cinq diffractions ou plus à des angles (2 thêta ± 0,2) de 6,2, 12,7, 17,3, 20,4, 25,3 et 26,4 ;
d) un diagramme de diffraction des rayons X sur poudre (DRXP) comprenant des diffractions à des angles (2 thêta ± 0,2) de 6,2, 12,7, 17,3, 20,4, 25,3 et 26,4 ;
e) un diagramme de diffraction des rayons X sur poudre (DRXP) comprenant des diffractions à des angles (2 thêta ± 0,2) de 6,2, 12,7, 17,3, 20,4, 25,3 et 26,4, correspondant à un espacement d (angströms ± 0,2) de 14,32, 6,97, 5,13, 4,34, 3,52 et 3,37 (respectivement) ;
f) un diagramme de diffraction des rayons X sur poudre (DRXP) comprenant des diffractions à des angles (2 thêta ± 0,2) de :
| Angle / ° 2θ | Angle / ° 2θ | Angle / ° 2θ |
|---|---|---|
| 6,2 | 18,0 | 25,3 |
| 10,1 | 18,5 | 26,1 |
| 10,9 | 19,3 | 26,4 |
| 11,3 | 20,0 | 26,7 |
| 12,3 | 20,4 | 27,4 |
| 12,7 | 20,8 | 27,9 |
| 13,9 | 21,2 | 28,6 |
| 15,0 | 22,4 | 29,7 |
| 16,5 | 23,5 | 30,2 |
| 17,3 | 23,7 | 30,9 |
g) un diagramme de diffraction des rayons X sur poudre (DRXP) comprenant des diffractions à des angles (2 thêta ± 0,2) correspondant à un espacement d (angströms ± 0,2) de :
| Angle / ° 2θ | espacement d / Angström | Angle / ° 2θ | espacement d / Angström |
|---|---|---|---|
| 6,2 | 14,32 | 20,8 | 4,26 |
| 10,1 | 8,74 | 21,2 | 4,19 |
| 10,9 | 8, 09 | 22,4 | 3, 96 |
| 11,3 | 7, 82 | 23,5 | 3,79 |
| 12,3 | 7,22 | 23,7 | 3,76 |
| 12,7 | 6, 97 | 25,3 | 3,52 |
| 13,9 | 6,37 | 26,1 | 3,41 |
| 15,0 | 5,90 | 26,4 | 3,37 |
| 16,5 | 5,37 | 26,7 | 3,33 |
| 17,3 | 5,13 | 27,4 | 3,25 |
| 18,0 | 4,91 | 27,9 | 3,19 |
| 18,5 | 4,80 | 28,6 | 3,12 |
| 19,3 | 4,60 | 29,7 | 3,00 |
| 20,0 | 4,44 | 30,2 | 2,96 |
| 20,4 | 4,34 | 30,9 | 2,89 |
ou
(ii) la forme solide du Composé 3 ou la forme solide du Composé 3 diagramme 1 **caractérisée par** l'un quelconque des points (i) a) à g) est **caractérisée par** l'un quelconque des points h) ou i) :
h) un endotherme de calorimétrie différentielle à balayage (DSC) ayant un minimum à environ 187,2 °C ;
i) une augmentation de masse d'environ 0,22 % entre 0 et 90 % d'humidité relative, HR, par analyse par sorption gravimétrique de vapeur (GVS).

4. Forme solide selon la revendication 1, dans laquelle la forme solide est une forme solide du Composé 4, comprenant le Composé 1 et l'acide succinique : 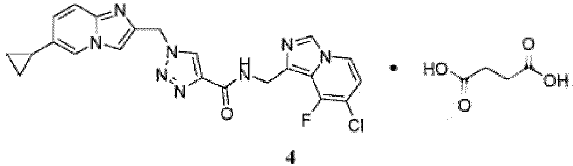 où
(i) la forme solide est le Composé 4 diagramme 1 et est **caractérisée par** l'un quelconque des points a) à g) :
a) un diagramme de diffraction des rayons X sur poudre (DRXP) comprenant trois diffractions ou plus à des angles (2 thêta ± 0,2) de 6,2, 10,2, 12,7, 17,3, 20,4 et 23,8 ;
b) un diagramme de diffraction des rayons X sur poudre (DRXP) comprenant quatre diffractions ou plus à des angles (2 thêta ± 0,2) de 6,2, 10,2, 12,7, 17,3, 20,4 et 23,8 ;
c) un diagramme de diffraction des rayons X sur poudre (DRXP) comprenant cinq diffractions ou plus à des angles (2 thêta ± 0,2) de 6,2, 10,2, 12,7, 17,3, 20,4 et 23,8 ;
d) un diagramme de diffraction des rayons X sur poudre (DRXP) comprenant des diffractions à des angles (2 thêta ± 0,2) de 6,2, 10,2, 12,7, 17,3, 20,4 et 23,8 ;
e) un diagramme de diffraction des rayons X sur poudre (DRXP) comprenant des diffractions à des angles (2 thêta ± 0,2) de 6,2, 10,2, 12,7, 17,3, 20,4 et 23,8, correspondant à un espacement d (angströms ± 0,2) de 14,26, 8,64, 6,95, 5,12, 4,35 et 3,73 (respectivement) ;
f) un diagramme de diffraction des rayons X sur poudre (DRXP) comprenant des diffractions à des angles (2 thêta ± 0,2) de :
| Angle / ° 2θ | Angle / ° 2θ | Angle / ° 2θ |
|---|---|---|
| 6,2 | 18,5 | 26,5 |
| 7,9 | 19,3 | 26,8 |
| 10,2 | 20,4 | 27,4 |
| 10,9 | 20,9 | 27,9 |
| 11,2 | 21,8 | 28,4 |
| 11,5 | 23,0 | 28,7 |
| 12,7 | 23,8 | 29,8 |
| 16,5 | 24,6 | 31,0 |
| 17,3 | 25,4 | |
| 18,1 | 26,2 | |
g) un diagramme de diffraction des rayons X sur poudre (DRXP) comprenant des diffractions à des angles (2 thêta ± 0,2) correspondant à un espacement d (angströms ± 0,2) de :
| Angle / ° 2θ | espacement d / Angström | Angle / ° 2θ | espacement d / Angström |
|---|---|---|---|
| 6,2 | 14,26 | 21,8 | 4,08 |
| 7,9 | 11,19 | 23,0 | 3,86 |
| 10,2 | 8,64 | 23,8 | 3,73 |
| 10,9 | 8,11 | 24,6 | 3,62 |
| 11,2 | 7,86 | 25,4 | 3,51 |
| 11,5 | 7,69 | 26,2 | 3,40 |
| 12,7 | 6,95 | 26,5 | 3,36 |
| 16,5 | 5,37 | 26,8 | 3,32 |
| 17,3 | 5,12 | 27,4 | 3,25 |
| 18,1 | 4,89 | 27,9 | 3,19 |
| 18,5 | 4,80 | 28,4 | 3,14 |
| 19,3 | 4,59 | 28,7 | 3,10 |
| 20,4 | 4,35 | 29,8 | 3,00 |
| 20,9 | 4,24 | 31,0 | 2,89 |
ou
(ii) la forme solide du Composé 4 ou la forme solide du Composé 4 diagramme 1 **caractérisée par** l'un quelconque des points (i) a) à g) est **caractérisée par** l'un quelconque des points h) ou i) :
h) un endotherme de calorimétrie différentielle à balayage (DSC) ayant un minimum à environ 223,4 °C ;
i) une augmentation de masse d'environ 0,16 % entre 0 et 90 % de HR par analyse par sorption gravimétrique de vapeur (GVS).

5. Forme solide selon la revendication 1, dans laquelle la forme solide est une forme solide du Composé 5, comprenant le Composé 1 et l'acide phosphorique : 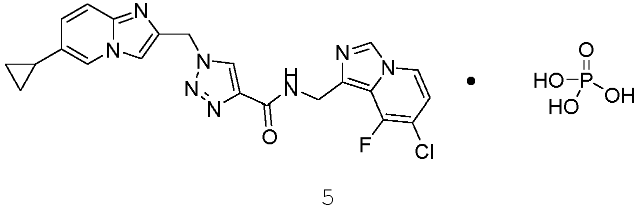 où
(i) la forme solide est le Composé 5 diagramme 1 et est **caractérisée par** l'un quelconque des points a) à g) :
a) un diagramme de diffraction des rayons X sur poudre (DRXP) comprenant trois diffractions ou plus à des angles (2 thêta ± 0,2) de 4,7, 6,1, 17,0, 17,4, 18,1 et 24,6 ;
b) un diagramme de diffraction des rayons X sur poudre (DRXP) comprenant quatre diffractions ou plus à des angles (2 thêta ± 0,2) de 4,7, 6,1, 17,0, 17,4, 18,1 et 24,6 ;
c) un diagramme de diffraction des rayons X sur poudre (DRXP) comprenant cinq diffractions ou plus à des angles (2 thêta ± 0,2) de 4,7, 6,1, 17,0, 17,4, 18,1 et 24,6 ;
d) un diagramme de diffraction des rayons X sur poudre (DRXP) comprenant des diffractions à des angles (2 thêta ± 0,2) de 4,7, 6,1, 17,0, 17,4, 18,1 et 24,6 ;
e) un diagramme de diffraction des rayons X sur poudre (DRXP) comprenant des diffractions à des angles (2 thêta ± 0,2) de 4,7, 6,1, 17,0, 17,4, 18,1 et 24,6, correspondant à un espacement d (angströms ± 0,2) de 18,68, 14,45, 5,20, 5,10, 4,91 et 3,62 (respectivement) ;
f) un diagramme de diffraction des rayons X sur poudre (DRXP) comprenant des diffractions à des angles (2 thêta ± 0,2) de :
| Angle / ° 2θ | Angle / ° 2θ |
|---|---|
| 4,7 | 19,4 |
| 6,1 | 21,4 |
| 12,1 | 22,8 |
| 13,6 | 23,6 |
| 14,2 | 24,6 |
| 15,0 | 25,3 |
| 16,4 | 27,4 |
| 17,0 | 28,7 |
| 17,4 | 30,9 |
| 18,1 | 31,6 |
g) un diagramme de diffraction des rayons X sur poudre (DRXP) comprenant des diffractions à des angles (2 thêta ± 0,2) correspondant à un espacement d (angströms ± 0,2) de :
| Angle / ° 2θ | espacement d / Angström | Angle / ° 2θ | espacement d / Angström |
|---|---|---|---|
| 4,7 | 18,68 | 19,4 | 4,58 |
| 6,1 | 14,45 | 21,4 | 4,15 |
| 12,1 | 7,33 | 22,8 | 3,89 |
| 13,6 | 6,51 | 23,6 | 3,77 |
| 14,2 | 6,24 | 24,6 | 3,62 |
| 15,0 | 5,91 | 25,3 | 3,51 |
| 16,4 | 5,40 | 27,4 | 3,25 |
| 17,0 | 5,20 | 28,7 | 3,11 |
| 17,4 | 5,10 | 30,9 | 2,89 |
| 18,1 | 4,91 | 31,6 | 2,83 |
ou
(ii) la forme solide du Composé 5 ou la forme solide du Composé 5 diagramme 1 **caractérisée par** l'un quelconque des points (i) a) à g) est **caractérisée par** l'un quelconque des points h) ou i) :
h) un endotherme de calorimétrie différentielle à balayage (DSC) ayant un minimum à environ 224,7 °C ; ou
i) une augmentation de masse d'environ 1,2 % entre 0 et 90 % de HR par analyse par sorption gravimétrique de vapeur (GVS).

6. Forme solide selon la revendication 1, dans laquelle la forme solide est une forme solide du Composé 6, comprenant le Composé 1 et l'acide oxalique : 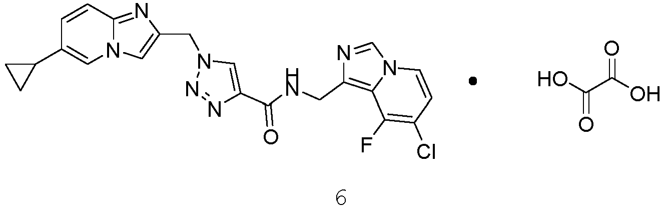 où
(i) la forme solide est le Composé 6 diagramme 1 et est **caractérisée par** l'un quelconque des points a) à g) :
a) un diagramme de diffraction des rayons X sur poudre (DRXP) comprenant trois diffractions ou plus à des angles (2 thêta ± 0,2) de 4,7, 6,1, 18,7, 24,0, 24,2 et 24,6 ;
b) un diagramme de diffraction des rayons X sur poudre (DRXP) comprenant quatre diffractions ou plus à des angles (2 thêta ± 0,2) de 4,7, 6,1, 18,7, 24,0, 24,2 et 24,6 ;
c) un diagramme de diffraction des rayons X sur poudre (DRXP) comprenant cinq diffractions ou plus à des angles (2 thêta ± 0,2) de 4,7, 6,1, 18,7, 24,0, 24,2 et 24,6 ;
d) un diagramme de diffraction des rayons X sur poudre (DRXP) comprenant des diffractions à des angles (2 thêta ± 0,2) de 4,7, 6,1, 18,7, 24,0, 24,2 et 24,6 ;
e) un diagramme de diffraction des rayons X sur poudre (DRXP) comprenant des diffractions à des angles (2 thêta ± 0,2) de 4,7, 6,1, 18,7, 24,0, 24,2 et 24,6, correspondant à un espacement d (angströms ± 0,2) de 18,88, 14,36, 4,73, 3,71, 3,67 et 3,62 (respectivement) ;
f) un diagramme de diffraction des rayons X sur poudre (DRXP) comprenant des diffractions à des angles (2 thêta ± 0,2) de :
| Angle / ° 2θ | Angle / ° 2θ | Angle / ° 2θ |
|---|---|---|
| 4,7 | 15,9 | 24,0 |
| 6,1 | 16,4 | 24,2 |
| 7,1 | 16,7 | 24,6 |
| 8,4 | 17,0 | 25,3 |
| 9,3 | 17,3 | 26,1 |
| 11,9 | 18,7 | 27,3 |
| 13,6 | 19,7 | 27,9 |
| 14,0 | 20,3 | 28,7 |
| 14,9 | 21,7 | 29,8 |
| 15,4 | 22,0 | 30,9 |
g) un diagramme de diffraction des rayons X sur poudre (DRXP) comprenant des diffractions à des angles (2 thêta ± 0,2) correspondant à un espacement d (angströms ± 0,2) de :
| Angle / ° 2θ | espacement d / Angström | Angle / ° 2θ | espacement d / Angström |
|---|---|---|---|
| 4,7 | 18,88 | 18,7 | 4,73 |
| 6,1 | 14,36 | 19,7 | 4,50 |
| 7,1 | 12,43 | 20,3 | 4,37 |
| 8,4 | 10,58 | 21,7 | 4,09 |
| 9,3 | 9,51 | 22,0 | 4,03 |
| 11,9 | 7,44 | 24,0 | 3,71 |
| 13,6 | 6,51 | 24,2 | 3,67 |
| 14,0 | 6,32 | 24,6 | 3, 62 |
| 14,9 | 5,96 | 25,3 | 3,52 |
| 15,4 | 5,74 | 26,1 | 3,42 |
| 15,9 | 5,56 | 27,3 | 3,26 |
| 16,4 | 5,40 | 27,9 | 3,19 |
| 16,7 | 5,30 | 28,7 | 3,11 |
| 17,0 | 5,20 | 29,8 | 2,99 |
| 17,3 | 5,12 | 30,9 | 2,90 |
(ii) la forme solide du Composé 6 ou la forme solide du Composé 6 diagramme 1 **caractérisée par** l'un quelconque des points (i) a) à g) est **caractérisée par** l'un quelconque des points h) ou i) :
h) un endotherme de calorimétrie différentielle à balayage (DSC) ayant un minimum à environ 211,3 °C ; ou
i) une augmentation de masse d'environ 0,6 % entre 0 et 90 % de HR par analyse par sorption gravimétrique de vapeur (GVS) ;
(iii) la forme solide est le Composé 6 diagramme 4 et est **caractérisée par** l'un quelconque des points a) à g) :
a) un diagramme de diffraction des rayons X sur poudre (DRXP) comprenant trois diffractions ou plus à des angles (2 thêta ± 0,2) de 6,4, 11,9, 19,2, 26,1, 26,6 et 27,2 ;
b) un diagramme de diffraction des rayons X sur poudre (DRXP) comprenant quatre diffractions ou plus à des angles (2 thêta ± 0,2) de 6,4, 11,9, 19,2, 26,1, 26,6 et 27,2 ;
c) un diagramme de diffraction des rayons X sur poudre (DRXP) comprenant cinq diffractions ou plus à des angles (2 thêta ± 0,2) de 6,4, 11,9, 19,2, 26,1, 26,6 et 27,2 ;
d) un diagramme de diffraction des rayons X sur poudre (DRXP) comprenant des diffractions à des angles (2 thêta ± 0,2) de 6,4, 11,9, 19,2, 26,1, 26,6 et 27,2 ;
e) un diagramme de diffraction des rayons X sur poudre (DRXP) comprenant des diffractions à des angles (2 thêta ± 0,2) de 6,4, 11,9, 19,2, 26,1, 26,6 et 27,2, correspondant à un espacement d (angströms ± 0,2) de 13,89, 7,43, 4,63, 3,42, 3,35 et 3,27 (respectivement) ;
f) un diagramme de diffraction des rayons X sur poudre (DRXP) comprenant des diffractions à des angles (2 thêta ± 0,2) de :
| Angle / ° 2θ | Angle / ° 2θ | Angle / ° 2θ |
|---|---|---|
| 6,4 | 18,2 | 24,0 |
| 9,7 | 19,2 | 24,5 |
| 11,6 | 19,5 | 25,5 |
| 11,9 | 20,0 | 26,1 |
| 12,7 | 20,9 | 26,6 |
| 14,0 | 21,6 | 27,2 |
| 15,6 | 22,6 | 28,1 |
| 16,7 | 23,3 | 29,1 |
g) un diagramme de diffraction des rayons X sur poudre (DRXP) comprenant des diffractions à des angles (2 thêta ± 0,2) correspondant à un espacement d (angströms ± 0,2) de :
| Angle / ° 2θ | espacement d / Angström | Angle / ° 2θ | espacement d / Angström |
|---|---|---|---|
| 6,4 | 13,89 | 20,9 | 4,25 |
| 9,7 | 9,08 | 21,6 | 4,10 |
| 11,6 | 7,65 | 22,6 | 3,93 |
| 11,9 | 7,43 | 23,3 | 3,81 |
| 12,7 | 6,97 | 24,0 | 3,71 |
| 14,0 | 6,31 | 24,5 | 3, 62 |
| 15,6 | 5,67 | 25,5 | 3,49 |
| 16,7 | 5,31 | 26,1 | 3,42 |
| 18,1 | 4,87 | 26,6 | 3,35 |
| 19,2 | 4,63 | 27,2 | 3,27 |
| 19,5 | 4,55 | 28,1 | 3,18 |
| 20,0 | 4,45 | 29,2 | 3,06 |
ou
(iv) la forme solide du Composé 6 ou la forme solide du Composé 6 diagramme 4 **caractérisée par** l'un quelconque des points (i) a) à g) est **caractérisée par** l'un quelconque des points h) ou i) :
h) un endotherme de calorimétrie différentielle à balayage (DSC) ayant un minimum à environ 222,9 °C ; ou
i) une augmentation de masse d'environ 0,66 % entre 0 et 90 % de HR par analyse par sorption gravimétrique de vapeur (GVS).

7. Forme solide selon l'une quelconque des revendications 1 à 6, dans laquelle la forme solide du composé a un rapport acide:base d'environ 1:1.

8. Forme solide selon l'une quelconque des revendications 1 à 7, dans laquelle ladite forme solide est cristalline.

9. Forme solide selon l'une quelconque des revendications 1 à 7, dans laquelle ladite forme solide est amorphe.

10. Forme solide selon l'une quelconque des revendications 1 à 9, dans laquelle ladite forme solide est sensiblement exempte d'impuretés.

11. Forme solide selon la revendication 1, dans laquelle la forme solide est une forme solide du Composé 6 : 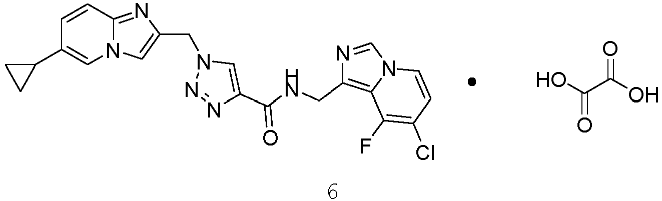 préparé par :
(i) un procédé comprenant les étapes de combinaison du Composé 1 et de l'acide oxalique dans de l'IPA, puis d'élimination du solvant pour obtenir la forme solide du Composé 6, dans lequel la forme solide est préparée par :
a) un procédé comprenant les étapes d'ajout d'une solution d'acide oxalique dans du THF à une solution du Composé 1 dans de l'IPA, puis d'élimination du solvant pour obtenir la forme solide du Composé 6 ; ou
b) un procédé comprenant les étapes de :
i) ajout d'une solution d'acide oxalique dans du THF à une solution du Composé 1 dans de l'IPA à 50 °C,
ii) refroidissement de la solution combinée de l'étape i) à 5 °C,
iii) agitation de la solution refroidie pendant un temps suffisant pour former la forme solide du Composé 6, et iv) filtration de la solution combinée pour éliminer le solvant ;
ou
(ii) un procédé comprenant les étapes de combinaison du Composé 1 et de l'acide oxalique dans de l'acétone, puis d'élimination du solvant pour obtenir la forme solide du Composé 6, dans lequel la forme solide est préparée par :
a) un procédé comprenant les étapes de : ajout d'une solution d'acide oxalique dans du THF à une solution du Composé 1 dans de l'acétone, puis élimination du solvant pour obtenir la forme solide du Composé 6 ; ou
b) un procédé comprenant les étapes de :
v) ajout d'une solution d'acide oxalique dans du THF à une solution du Composé 1 dans de l'acétone à 50 °C,
vi) refroidissement de la solution combinée de l'étape i) à 5 °C,
vii) agitation de la solution refroidie pendant un temps suffisant pour former la forme solide du Composé 6, et viii) filtration de la solution combinée pour éliminer le solvant.

12. Composition pharmaceutique comprenant la forme solide selon l'une quelconque des revendications 1 à 11 et un support ou excipient pharmaceutiquement acceptable.

13. Procédé de préparation d'une forme solide du Composé 6, 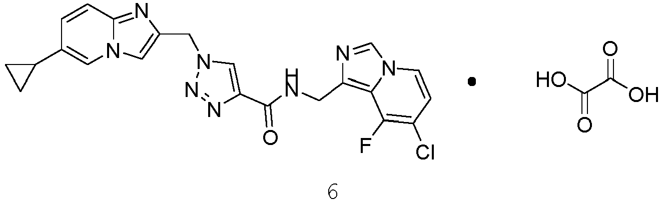 dans lequel le procédé comprend :
(i) les étapes de combinaison du Composé 1 et de l'acide oxalique dans de l'IPA, puis d'élimination du solvant pour obtenir la forme solide du Composé 6, dans lequel la forme solide est préparée par :
a) un procédé comprenant les étapes d'ajout d'une solution d'acide oxalique dans du THF à une solution du Composé 1 dans de l'IPA, puis d'élimination du solvant pour obtenir la forme solide du Composé 6; ou
b) un procédé comprenant les étapes de :
i) ajout d'une solution d'acide oxalique dans du THF à une solution du Composé 1 dans de l'IPA à 50 °C,
ii) refroidissement de la solution combinée de l'étape i) à 5 °C,
iii) agitation de la solution refroidie pendant un temps suffisant pour former la forme solide du Composé 6, et iv) filtration de la solution combinée pour éliminer le solvant ;
ou
(ii) les étapes de combinaison du Composé 1 et de l'acide oxalique dans de l'acétone, puis d'élimination du solvant pour obtenir la forme solide du Composé 6, dans lequel la forme solide est préparée par :
a) un procédé comprenant les étapes de : ajout d'une solution d'acide oxalique dans du THF à une solution du Composé 1 dans de l'acétone, puis élimination du solvant pour obtenir la forme solide du Composé 6 ; ou
b) un procédé comprenant les étapes de :
v) ajout d'une solution d'acide oxalique dans du THF à une solution du Composé 1 dans de l'acétone à 50 °C,
vi) refroidissement de la solution combinée de l'étape i) à 5 °C,
vii) agitation de la solution refroidie pendant un temps suffisant pour former la forme solide du Composé 6, et viii) filtration de la solution combinée pour éliminer le solvant.

14. Forme solide ou composition selon l'une quelconque des revendications 1 à 12 pour une utilisation dans un procédé de traitement d'une maladie ou d'un trouble médié(e) par la kallicréine plasmatique.

15. Forme solide ou composition selon l'une quelconque des revendications 1 à 12, pour une utilisation dans un procédé de traitement de l'angio-œdème héréditaire ou de l'œdème maculaire diabétique.
